# EUROPÄISCHE PATENTSCHRIFT

(11) **EP 0 752 989 B1**
(45) Veröffentlichungstag und Bekanntmachung des Hinweises auf die Patenterteilung: **19.07.2000**
(21) Anmeldenummer: 95930317.3
(22) Anmeldetag: 20.03.1995
(51) Int. Cl.: C07D 417/04, A01N 43/78

(54) **THIAZOLYLPYRAZOLINONE, IHRE VERWENDUNG ZUM SCHUTZE VON TECHNISCHEN MATERIALEN**
THIAZOLYLPYRAZOLINONES AND THEIR USE FOR PROTECTING TECHNICAL MATERIALS
THIAZOLYLPYRAZOLINONES ET LEUR APPLICATION POUR LA PROTECTION DE MATERIAUX A USAGE TECHNIQUE

(30) Priorität: 31.03.1994 DE 4411235
(43) Veröffentlichungstag der Anmeldung: 15.01.1997
(73) Patentinhaber: BAYER AG, 51368 Leverkusen (DE)
(72) Erfinder: HEUER, Lutz, D-47800 Krefeld (DE); WACHTLER, Peter, D-51061 Köln (DE); KUGLER, Martin, D-42799 Leichlingen (DE); SCHRAGE, Heinrich, D-47800 Krefeld (DE); SASSE, Klaus, D-51467 Bergisch Gladbach (DE)
(86) Internationale Anmeldenummer: EP9501032
(87) Internationale Veröffentlichungsnummer: WO9526962

(56) Entgegenhaltungen:
- CHEMICAL ABSTRACTS, vol. 120, no. 1, 3.Januar 1994 Columbus, Ohio, US; abstract no. 8516q, S.P.SINGH ET AL 'a facile route for the synthesis of 1-(5-hydroxy-3-methyl-1-(2-thiazolyl)-4-py razolyl)-1,3-butanediones from dehydroacetic acid' Seite 940; Spalte 1; in der Anmeldung erwähnt & SYNTH. COMMUN., Bd. 23,Nr. 13, 1993 Seiten 1855-1861,
- CHEMICAL ABSTRACTS, vol. 98, no. 15, 11.April 1983 Columbus, Ohio, US; abstract no. 125958y, S.N.SAWHNEY ET AL 'synthesis and antiinflammatory activity of some 2-(4'-carboxymethyl)-3'-methyl-5'-oxo-2'-p yrazolin-1'-yl)-4-arylthiazoles and 2-(4'-carboxymethyl-3'-methyl-5'-oxo-2'-py razolin-1'-yl)benzothiazoles' Seite 628; Spalte 2; in der Anmeldung erwähnt & INDIAN J. CHEM. SECT.B, Bd. 21b,Nr. 9, 1982 Seiten 869-871,
- CHEMICAL ABSTRACTS, vol. 96, no. 7, 15.Februar 1982 Columbus, Ohio, US; abstract no. 52303c, Seite 622; Spalte 1; & DD,A,150 203 (ANDREAS M.RICHTER ET AL) 19.August 1981 in der Anmeldung erwähnt

## Beschreibung

Die vorliegende Erfindung betrifft neue Thiazolylpyrazolinone, ein Verfahren zur ihrer Herstellung und ihre Verwendung zum Schutz von technischen Materialien.

Thiazolylpyrazolinone sind bekannt und werden z.B. in JP-2 149 617, Indian. J. Chem. 21 B, 869 (1982), Synth. Com. 23, 1855 (1993), J. Heterocyclic Chem. 27, 865 (1990) und DD-150 203 beschrieben. Deren Verwendung zum Schutz von technischen Materialien ist jedoch noch nicht bekannt.

Gegenstand der Anmeldung sind neue Thiazolylpyrazolinonderivate der Formel 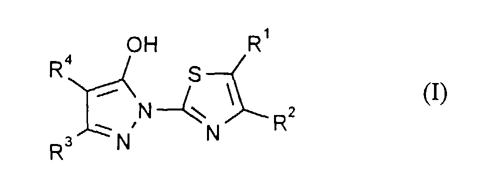 in der
- R¹, R², R³: Wasserstoff oder Methyl,
- R⁴: unsubstituiertes Alkyl, Cycloalkyl, Alkenyl, Aralkyl oder Aryl auch durch Halogen substitueirtes Alkyl; durch 1 bis 3 C₁-C₄-Alkyl oder 1 bis 3 Halogen substituiertes Cycloalkyl; durch jeweils 1 bis 3 Halogen, Nitro, Cyano, C₁-C₄-Alkyl, C₁-C₄-Alkoxy, halogenisertes C₁-C₄-Alkyl, halogeniertes C₁-C₄-Alkoxy, C₁-C₄-Alkylmercapto oder halogenisertes C₁-C₄-Alkylmercapto substituiertes Aralkyl; oder durch jeweils 1 bis 3 Halogen, C₁-C₆-Alkyl, C₁-C₆-Alkoxy, C₁-C₆-Thioalkyl, Halogen-C₁-C₂-alkyl, Cyano, Nitro, C₁-C₆-Alkoxycarbonyl oder Amino substituiertes Aryl bedeuten.

In der vorliegenden Anmeldung bedeutet:

Alkyl vorzugsweise geradkettiges oder verzweigtes, gegebenenfalls substituiertes Alkyl mit 1 bis 18 C-Atomen, wie Me, Et, n-, i-Propyl, n-, i-, s- und tert.-Butyl, n-, i und tert.-Pentyl, n-Hexyl, n-Octyl, n-Nonyl, n-Decyl, n-Undecyl, n-Dodecyl, n-Tridecyl, n-Tetradecyl, n-Pentadecyl, n-Hexadecyl, n-Heptodecyl oder n-Octadecyl oder ihre verzweigten Strukturisomeren.

Als Substituenten kommen vorzugsweise Halogen, wie Chlor und/oder Fluor in Frage.

Auch kann ein Alkylrest durch 1 bis 2 Heteroatomen wie Sauerstoff oder Schwefel, oder Atomgruppen wie N-Me, N-Et, -S(O), -SO₂ unterbrochen sein, ohne das sich seine Gesamtanzahl an Atomen ändert.

Alkenyl (+ Alkinyl) ist vorzugsweise wie Alkyl definiert, nur insofern geändert, daß mindestens eine und maximal drei C-C-Einfachbindung durch eine C-C-Doppel(Dreifach)bindung ersetzt wurde. Die Anzahl an C-Atomen beträgt mindestens drei und wird mit jeder weiteren Doppelbindung (Dreifachbindung), die hinzu kommt um mindestens zwei C-Atome verlängert.

Cycloalkyl- und Cycloalkenylgruppen umfassen Cycloalkyl mit vorzugsweise 3 (5) bis 7 C-Atomen, wie beispielsweise Cyclopropyl, Cyclobutyl, Cycloheptyl, Cyclopentyl, Cyclopentenyl, Cyclohexenyl, Cyclohexyl; bevorzugte substituierte Cycloalkylgruppen umfassen durch 1 bis 3 C₁-C₄-Alkylgruppen oder 1 bis 3 Halogenatomen, wie Chlor und/oder Fluor, substituiertes Cycloalkyl, wie beispielsweise Methylcyclohexyl, Dimethylcyclohexyl, 1,3,3-Trimethylcyclohexyl, 3-Chlorcyclohexyl. Alkyl(cycloalkyl)- (und Alkyl(cycloalkenyl)-Gruppen) enthalten vorzugsweise 1 bis 6 C-Atome im geradkettigen oder verzweigten Alkylteil und 3 (5) bis 7-Atome im Cycloalkyl/alkenyl-Teil; besonders (1-Cyclopentyl)methyl, (1-Cyclopentenyl)methyl, (1-Cyclohexenyl)methyl, (1-Cyclohexyl)methyl, (1-Cyclopropyl)methyl.

Alkoxycarbonyl steht für geradkettiges oder verzweigtes Alkoxycarbonyl mit vorzugsweise 1 bis 6 C-Atomen im Alkoxyrest, wie beispielsweise Methoxycarbonyl, Ethoxycarbonyl, n- und i-Propoxycarbonyl, n-, i-, sek.- und tert.-Butoxycarbonyl, Hexoxycarbonyl. Analoges gilt für die Alkoxycarbonylalkylgruppen.

Aralkyl enthält vorzugsweise 1 bis 6, insbesondere 1 bis 4 C-Atome im geradkettigen oder verzweigten Alkylteil und vorzugsweise Phenyl oder Naphthyl als Arylteil. Beispiele für solche Aralkylgruppen umfassen Benzyl, α-Methylbenzyl, α,α-Dimethylbenzyl, 2-Phenethyl, α- und β-Naphthylmethyl. Diese Aralkylreste können 1 bis 3 Substituenten aus der Reihe Halogen (insbesondere Chlor und/oder Fluor), Nitro, Cyano, gegebenenfalls halogeniertes C₁-C₄-Alkyl oder -Alkoxy, wie beispielsweise Methyl, Ethyl, Trifluormethyl, Difluorchlormethyl, Difluormethyl, Trichlormethyl, Methoxy, Ethoxy, Trifluormethoxy, Difluorchlormethoxy und Difluormethoxy, gegebenenfalls halogeniertes C₁-C₄-Alkylmercapto, wie beispielsweise Methylmercapto, Trifluormethylmercapto, Difluorchlormethylmercapto tragen.

Unter dem Begriff Aryl ist unsubstituiertes oder substituiertes Aryl mit vorzugsweise 6 bis 12 C-Atomen im Arylteil zu verstehen. Bevorzugte Beispiele umfassen Phenyl, Biphenyl und Naphthyl. Die Arylgruppen können 1 bis 3 Substituenten aus der Reihe Halogen (insbesondere Chlor und/oder Fluor), C₁-C₆-Alkyl, -Alkoxy oder Thioalkoxy, Halogen-C₁-C₂-alkyl (wie Trifluormethyl, Difluormethyl), Cyano, Nitro, C₁-C₆-Alkoxycarbonyl oder Amino tragen.

Unter dem Begriff Alkoxy ist geradkettiges und verzweigtes Alkoxy mit vorzugsweise 1 bis 12, insbesondere 1 bis 4 C-Atomen zu verstehen. Bevorzugte Beispiele umfassen Methoxy, Ethoxy, n- und i-Propoxy, n-, i-, sek.- und ter.-Butoxy, Pentoxy, Hexoxy, Heptoxy, Octoxy, Nonoxy und Decoxy. Die Alkoxygruppen können durch 1 bis 3 Halogenatome (Cl, F) substituiert sein, bevorzugt: O-CF₃, O-CHF₂, O-CF₂-O, O-CF₂-CF₂-O.

Alkylthio steht für geradkettiges oder verzweigtes Alkylthio mit vorzugsweise 1 bis 12 C-Atomen. Bevorzugte Beispiele umfassen Methylthio, Ethylthio, n- und i-Propylthio, n-, i-, sek.- und ter.-Butylthio, n-Pentylthio und seine Isomeren wie 1-, 2- und 3-Methyl-butylthio. Die Alkylthiogruppen können durch 1 bis 3 Halogenatome (vorzugsweise Chlor und/oder Fluor) substituiert sein; bevorzugte Beispiele hierfür sind Di- und Trifluormethylthio sowie Difluorchlormethylthio.

Aralkoxy enthält vorzugsweise 1 bis 6 C-Atome im geradkettigen oder verzweigten Alkylteil und vorzugsweise Phenyl als Arylteil. Bevorzugte Beispiele sind Benzyloxy und Phenethyloxy. Die Aralkoxygruppen können durch 1 bis 3 Halogenatome (vorzugsweise Chlor und/oder Fluor) oder durch eine C₁-C₄-Alkylgruppe substituiert sein.

Cyanoalkyl wie Alkyl (1 bis 6) nur mit Cyano-substituiert, bevorzugt endständig.

Hetaryl: Furanyl, Thienyl, Thiazolyl, Pyrazolyl, Pyrrolyl, Imidazyl, Triazolyl, gegebenenfalls mit 1 bis 2 Halogen oder Alkyl, Alkoxy oder Thioalkoxy-Substituenten.

### Halogen: F, Cl, Br, I.

Aralkylthio enthält vorzugsweise 1 bis 6 C-Atome im geradkettigen oder verzweigten Alkylteil und vorzugsweise Phenyl als Arylteil. Bevorzugtes Beispiel ist Benzylthio. Die Aralkylthiogruppen können durch 1 bis 3 Halogenatome (vorzugsweise Chlor und/oder Fluor) oder durch eine C₁-C₄-Alkylgruppe substituiert sein.

Aryloxy enthält vorzugsweise 1 bis 10 C-Atome im Arylteil. Bevorzugte Beispiele sind Phenoxy und Naphthoxy. Die Aryloxygruppen können durch 1 bis 3 Substituenten aus der Reihe Halogen (vorzugsweise Chlor und/oder Fluor), C₁-C₄-Alkyl, Halogen-C₁-C₂-alkyl (wie Di- und Trifluormethyl), Cyano, Nitro oder Amino tragen.

Arylthio enthält vorzugsweise 6 bis 10 C Atome im Arylteil. Bevorzugte Beispiele sind Phenylthio und Naphthylthio. Die Arylthiogruppen können die unter "Aryloxy" aufgezählten Substituenten tragen.

Bevorzugte Verbindungen für 1,ω-C₃-C₆-Alk(en)ylenreste umfassen 1,3-Propylen, 1,4-Butylen und 1,4-Butadien(1,3)ylen.

Verbindungen der Formel (I), worin 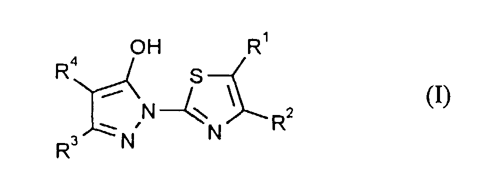
- R¹: Wasserstoff,
- R², R³: Wasserstoff oder Methyl, bedeuten
sind besonders bevorzugt.

Verbindungen der Formel (I), worin
- R¹, R² und R³: Wasserstoff,
- R⁴: unsubstituiertes Alkyl oder Cycloalkyl oder durch die oben angegebenen substituierten substituiertes Alkyl oder Cycloalkyl
bedeuten, sind ganz besonders bevorzugt.

Die erfindungsgemäßen Verbindungen der Formel (I) können als verschiedene (s.u.) Tautomere vorliegen, u.a. auch in ihrer tautomeren Pyrazol-5-on-Form.

Die neuen Thiazolylpyrazolinon-Derivate der Formel (I) werden erhalten, indem man Thiocarbamoylverbindungen der Formel (II) 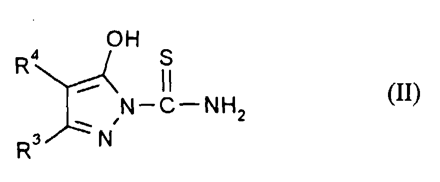 in denen R³ und R⁴ die oben angebenen Bedeutungen haben, mit Verbindungen der Formel (III) 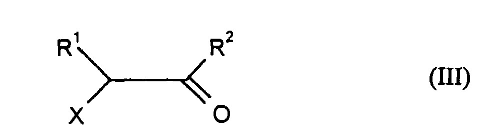 in denen R¹ und R² die oben angegebenen Bedeutungen haben und X für eine Abgangsgruppe steht, gegebenenfalls in Gegenwart eines Lösungs- bzw. Verdünnungsmittels und gegebenenfalls in Gegenwart einer Base umsetzt.

Die Thiocarbamoylpyrazolone der Formel (II) und das Verfahren zu ihrer Herstellung sind bekannt und werden z.B. in der JP-79/115 374, JP-79/119 031, J. Pesticide, Sci. 11, 205-212 (1986), Arch. Pharm. 316 (1983) 2-6, Sci. Pharm. 51 (2) (1982) 167-172 und der EP-1 515 934 beschrieben.

Die Verbindungen der Formel (II) werden üblicherweise erhalten, indem man entsprechende α-Formylessigsäureester oder α-Formylessigsäureamid oder β - Ketoessigsäureester oder ein β-Ketoessigsäureamid mit gegebenenfalls substituiertem Thiosemicarbazid umsetzt. Die zu verwendenden Formylsäureester werden nach verschiedenen, in der Literatur bekannten Wegen erhalten, so z.B. analog der in EP 417 597 oder DE 2 643 205 beschriebenen Methode der Hydroformylierung von α,β-ungesättigten Estern.

Die Verbindungen der Formel (III) sind ebenfalls bekannt oder nach allgemein bekannten Verfahren erhältich.

Zur Erleichterung der Ringschlußreaktionen setzt man vorteilhafterweise Basen wie Natriumhydroxid, Kaliumhydroxid oder Kalium-tert.-butylat zu. Vorzugsweise setzt man die Base in etwa äquivalenten Menge zu.

Die Verfahren werden gegebenenfalls in Gegenwart eines Lösungsmittels durchgeführt werden; als Lösungsmittel haben sich vor allem Alkohole wie Ethanol oder aromatische Kohlenwasserstoffe wie Toluol bewährt.

Die Verfahren sind ist innerhalb eines größeren Temperaturbereichs durchführbar. Für die zuerst ablaufende Thiosemicarbazonbildung wird bei Temperaturen von 20 bis 110°C, vorzugsweise zwischen 60 und 90°C gearbeitet. Die nach der Basenzugabe ablaufende Cyclokondensationsreaktion wird bei Temperaturen von 20 bis 100°C, vorzugsweise 20 bis 40°C, vorgenommen. Die Umsetzung der so erhaltenen Verbindungen der Formel (II) mit den Verbindungen der Formel (III) erfolgt dann bei Temperaturen von -20 bis 50°C, vorzugsweise 0 bis 30°C.

Die Verbindungen der Formel (I) und (II) können nach bekannten Methoden aus den Reaktionsgemischen isoliert werden. Man geht im allgemeinen so vor, daß man die Reaktionsgemische vom Lösungsmittel befreit und den Rückstand mit wäßriger Salzsäure behandelt. Die dabei ausfallenden Verbindungen werden durch Absaugen abgetrennt. Es ist jedoch auch möglich, das Reaktionsgemisch unmittelbar in einen großen Überschuß verdünnter Salzsäure einzugießen und die als Niederschlag sich abscheidenden Verbindungen abzufiltrieren.

Die Herstellung der erfindungsgemäßen Verrbindungen (I) ist auch in einem Eintopfverfahren ohne Isolierung der Vorstufe der Formel (II) möglich.

Die Wirkstoffe der Formel (I) und die erfindungsgemäßen Mittel weisen eine starke mikrobizide Wirkung auf und können zur Bekämpfung von unerwünschte Mikroorganismen praktisch eingesetzt werden. Die Wirkstoffe der Formel (I) und die erfindungsgemäßen Mittel sind zum Schutz von technischen Materialien gegen Befall und Zerstörung durch unerwünschte Mikroorganismen geeignet.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Anstrichmittel.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe der Formel (I) können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Bevorzugt werden die erfindungsgemäßen Wirkstoffe der Formel (I) zum Schutz von Anstrichen gegen Befall und Zerstörung durch unerwünschte Mikroorganismen eingesetzt.

Unter Anstrich ist im vorliegenden Zusammenhang eine aus Anstrichstoffen hergestellte Beschichtung auf einem Untergrund zu verstehen. Der Anstrich kann mehr oder weniger in den Untergrund eingedrungen sein. Er kann aus einer oder mehreren Schichten bestehen und durch Verfahren wie Streichen, Spritzen, Tauchen, Fluten oder ähnliche Verfahren hergestellt werden.

Die Verbindungen der Formel (I) werden in die Anstrichmittel oder in Vorprodukte zur Herstellung der Anstrichmittel nach üblichen Methoden, z.B. durch Vermischen der Wirkstoffe mit den anderen Komponenten, eingearbeitet.

Erfindungsgemäße Anstrichmittel enthalten daher neben mindestens einem fungiziden Wirkstoff der Formel (I) allgemein übliche Anstrichkomponenten in z.B. flüssiger, pastöser oder pulverförmiger Form wie z.B.
- Farbmittel, wie Pigmente oder Farbstoffe, bevorzugt Pigmente. Beispielsweise genannt sei Titandioxid, Zinkoxid und Eisenoxid.
- Bindemittel, wie beispielsweise oxidativ trocknende Alkydharze, Vinylpolymerisate und Vinylcopolymerisate, Acrylpolymerisate und Acrylcopolymerisate, Kunststoffpulver, Novolacke, Aminoharze, Polyesterharze, Epoxidharze, Silikonharze, Isocyanatharze bevorzugt sind Vinylpolymerisate und Vinylcopolymerisate, Acrylpolymerisate und Acrylcopolymerisate und andere in Wasser verdünnbaren Anstrichstoffen verwendbare Bindemittel.

Daneben enthalten die Anstriche gegebenenfalls folgende Zusatzstoffe
- Füllstoffe, wie beispielsweise Schwerspat, Calcit, Dolomit und Talk,
- Lösemittel, wie beispielsweise Alkohole, Ketone, Ester, Glykolether und aliphatische sowie aromatische Kohlenwasserstoffe,
- sowie Verdickungs- und Thixotropiermittel, Dispergier-und Netzmittel, Trockenstoffe, Hautverhütungsmittel, Verlaufmittel, Antischaummittel, Korrosionsinhibitoren, UV-Absorber, Duftstoffe, Antistatika, Frostschutzmittel.

Als Anstrichmittel bzw. Vorprodukte zur Herstellung von Anstrichmitteln seien vorzugsweise folgende genannt:
- Leime und Klebstoffe auf Basis der bekannten tierischen, pflanzlichen oder synthetischen Rohstoffe.
- Kunststoffdispersionen wie Latexdispersionen oder Dispersionen auf Basis anderer Polymere.
- Stärkelösungen, -dispersionen oder -slurries oder andere auf Basis von Stärke hergestellte Produkte wie z.B. Druckverdicker.
- Slurries anderer Rohstoffe wie Farbpigmente (z.B. Eisenoxidpigmente, Rußpigmente, Titandioxidpigmente) oder Slurries von Füllstoffen wie Kaolin oder Calciumcarbonat.
- Betonadditive beispielsweise auf Basis von Melasse oder Ligninsulfonaten.
- Bitumenemulsionen.
- Vor- und Zwischenprodukte der chemischen Industrie, z.B. bei der Farbstoffproduktion und -lagerung.
- Tinten oder Tuschen.
- Dispersionsfarben für die Anstrichindustrie.
- Schichten und Appreturen.

Die Wirksamkeit und das Wirkungsspektrum der Wirkstoffe der Formel (I) bzw. die daraus herstellbaren Mittel, Vorprodukte oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. des zusätzlichen Schutzes vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:
**Triazole wie:**
   Amitrole, Azocyclotin, BAS 480F, Bitertanol, Difenoconazole, Fenbuconazole, Fenchlorazole, Fenethanil, Fluquinconazole, Flusilazole, Flutriafol, Imibenconazole, Isozofos, Myclobutanil, Metconazole, Epoxyconazole, Paclobutrazol, Penconazole, Propioconazole, (+)-cis-1-(4-chlorphenyl)-2-(1H-1,2,4-triazol-1-yl)-cycloheptanol, Tetraconazole, Triadimefon, Triadimenol, Triapenthenol, Triflumizole, Triticonazole, Uniconazole sowie deren Metallsalze und Säureaddukte.
**Imidazole wie:**
   Imazalil, Pefurazoate, Prochloraz, Triflumizole, 2-(1-tert-Butyl)-1-(2-chlorphenyl)-3-(1,2,4-triazol-1-yl)-propan-2-ol, Thiazolcarboxanilide wie 2',6'-Dibromo-2-methyl-4-trifluoromethoxy-4'-trifluoromethyl-1,3-thiazole-5-carboxanilide, 1-Imidazolyl-1-(4'-chlorophenoxy)-3,3-dimethylbutan-2-on sowie deren Metallsalze und Säureaddukte.

Methyl(E)-2-[2-[6-(2-cyanophenoxy)pyrimidin-4-yloxy]phenyl]3-methoxyacrylate, methyl(E)-2-[2-[6-(2-thioamidophenoxy)pyrimidin-4-yloxy]phenyl]-3-methoxyacrylatemethyl(E)-2-[2-[6-(2-fluorophenoxy)pyrimidin-4-yloxy]phenyl]-3-methoxyacrylate, methyl(E)-2-[2-[6-(2,6-difluorophenoxy)pyrimidin-4-yloxy]phenyl]-3-methoxyacrylate, methyl(E)-2-[2-[3-(pyrimidin-2-yloxy)phenoxy]phenyl]-3-methoxyacrylatqethyl(E)-2-[2-[3-(5-methylpyrimidin-2-yloxy)-phenoxy]phenyl]-3-methoxyacrylatemethyl(E)-2-[2-[3-(phenyl-sulfonyloxy)phenoxy]phenyl]-3-methoxyacrylate,methyl(E)-2-[2-[3-(4-nitrophenoxy)phenoxy]phenyl]-3-methoxyacrylate, methyl(E)-2-[2-phenoxyphenyl]-3-methoxyacrylate, methyl(E)-2-[2-(3,5-dimethylbenzoyl)pyrrol-1-yl]-3-methoxyacrylate, methyl(E)-2-[2-(3-methoxyphenoxy)phenyl]-.3-methoxyacrylate, methyl(E)-2-[2-(2-phenylethen-1-yl)-phenyl]-3-methoxyacrylatemethyl(E)-2-[2-(3,5-dichlorophenoxy)pyridin-3-yl]-3-methoxyacrylate, methyl(E)-2-(2-(3-(1,1,2,2-tetrafluoroethoxy)phenoxy)phenyl)-3-methoxyacrylate, methyl(E)-2-(2-[3-(alpha-hydroxybenzyl)phenoxy]phenyl)-3-methoxyacrylate, methyl(E)-2-(2-(4-phenoxypyridin-2-yloxy)phenyl)-3-methoxyacrylatemethyl(E)-2-[2-(3-n-propyloxyphenoxy)phenyl]3-methoxyacrylate, methyl(E)-2-[2-(3-isopropyloxyphenoxy)phenyl]-3-methoxyacrylatemethyl(E)-2-[2-[3-(2-fluorophenoxy)pehnoxy]phenyl]-3-methoxyacrylate, methyl(E)-2-[2-(3-ethoxyphenoxy)phenyl]-3-methoxyacrylatemethyl(E)-2-[2-(4-tert.-butylpyridin-2-yloxy)phenyl]-3-methoxyacrylate, methyl(E)-2-[2-[3-(3-cyanophenoxy)phenoxy]phenyl]-3-methoxyacrylatemethyl(E)-2-[2-(3-methylpyridin-2-yloxymethyl)phenyl]-3-methoxyacrylatemethyl(E)-2-[2-[6-(2-methylphenoxy)pyrimidin-4-yloxy]phenyl]-3-methoxyacrylate,methyl(E)-2-[2-(5-bromopyridin-2-yloxymethyl)phenyl]-3-methoxyacrylate, methyl(E)-2-[2-(3-(3-iodopyridin-2-yloxy)phenoxy)phenyl]-3-methoxyacrylate, methyl(E)-2-[2-[6-(2-chloropyridin-3-yloxy)pyrimidin-4-yloxy]phenyl]-3-methoxyacrylate, (E),(E)methyl-2-[2-(5,6-dimethylpyrazin-2-ylmethyloximinomethyl)phenyl]-3-methoxyacrylate(E)-methyl-2-{2-[6-(6-methylpyridin-2-yloxy)pyrimidin-4-yloxy]phenyl}-3-methoxyacrylate, (E),(E)methyl-2-{2-(3-methoxyphenyl)methyloximinomethyl]phenyl}-3-methoxyacrylate(E)methyl-2-{2-(6-(2-azidophenoxy)-pyrimidin-4-yloxy]phenyl}3-methoxyacrylate,(E),(E)methyl-2-{2-[6-phenylpyrimidin-4-yl)-methyloximinomethyl]phenyl }-3-methoxyacrylate, (E),(E)methyl-2-{2-[(4-chlorophenyl)-methyloximinomethyl]phenyl}-3-methoxyacrylate, (E)methyl-2-{2-[6-(2-n-propylphenoxy)-1,3,5-triazin-4-yloxy]phenyl}-3-methoxyacrylate, (E),(E)methyl-2-{2-[(3-nitrophenyl)methyloximinomethyl]phenyl}-3-methoxyacrylate;
**Succinat-Dehydrogenase Inhibitoren wie:**
   Fenfuram, Furcarbanil, Cyclafluramid, Furmecyclox, Seedvax, Metsulfovax, Pyrocarbolid, Oxycarboxin, Shirlan, Mebenil (Mepronil), Benodanil, Flutolanil (Moncut); Naphthalin-Derivate wie Terbinafine, Naftifine, Butenafine, 3-Chloro-7-(2-aza-2,7,7-trimethyl-oct-3-en-5-in);
   Sulfenamide wie Dichlofluanid, Tolylfluanid, Folpet, Fluorfolpet; Captan, Captofol; Benzimidazole wie Carbendazim, Benomyl, Furathiocarb, Fuberidazole, Thiophonatmethyl, Thiabendazole oder deren Salze;
   Morpholinderivate wie Tridemorph, Fenpropimorph, Falimorph, Dimethomorph, Dodemorph, Aldimorph, Fenpropidin und ihre arylsulfonsauren Salze, wie z.B. p-Toluolsulfonsäure und p-Dodecylphenyl-sulfonsäure;
   Dithiocarbamate, Cufraneb, Ferbam, Mancopper, Mancozeb, Maneb, Metam, Metiram, Thiram Zeneb, Ziram;
   Benzthiazole wie 2-Mercaptobenzothiazol;
   Benzamide wie 2,6-Dichloro-N-(4-trifluoromethylbenzyl)-benzamide; Borverbindungen wie Borsäure, Borsäureester, Borax;
   Formaldehyd und Formaldehydabspaltende Verbindungen wie Benzylalkoholmono(poly)-hemiformal, Oxazolidine, Hexa-hydro-S-triazine, N-Methylolchloracetamid, Paraformadehyd, Nitropyrin, Oxolinsäure, Tecloftalam;
   Tris-N-(cyclohexyldiazeniumdioxy)-aluminium, N-(Cyclo-hexyldiazeniumdioxy)-tributylzinn bzw. K-Salze, Bis-N-(cyclohexyldiazeniumdioxy)-kupfer;
   N-Methylisothiazolin-3-on,5-Chlor-N-methylisothiazolin-3-on,4,5-Dichloro-N-octylisothiazolin-3-on, N-Octyl-isothiazolin-3-on, 4,5-Trimethylen-isothiazolinone, 4,5-Benzisothiazolinone, N-Methylolchloracetamid;
   Aldehyde wie Zimtaldehyd, Formaldehyd, Glutardialdehyd, β-Bromzimtaldehyd; Thiocyanate wie Thiocyanatomethylthiobenzothiazol, Methylenbisthiocyanat, usw; quartäre Ammoniumverbindungen wie Benzyldimethyltetradecylammoniumchlorid, Benzyldimethyldodecylammoniumchlorid, Didecyldimethaylammoniumchlorid;
   Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyclohexylcarbamat, 3-Iod-2-propinyl-phenylcarbamat;
   Phenolderivate wie Tribromphenol, Tetrachlorphenol, 3-Methyl-4-chlorphenol, 3,5-Dimethyl-4-chlorphenol, Phenoxyethanol, Dichlorphen, o-Phenylphenol, m-Phenylphenol, p-Phenylphenol, 2-Benzyl-4-chlorphenol und deren Alkali- und Erdalkalimetallsalze;
   Mikrobizide mit aktivierter Halogengruppe wie Chloracetamid, Bronopol, Bronidox, Tectamer wie 2-Brom-2-nitro-1,3-propandiol, 2-Brom-4'-hydroxy-acetophenon, 2,2-Dibrom-3-nitril-propionamid, 1,2-Dibrom-2,4-dicyanobutan, β-Brom-β-nitrostyrol;
   Pyridine wie 1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
   Metallseifen wie Zinn-, Kupfer-, Zinknaphtenat, -octoat, 2-ethylhexanoat, -oleat, -phosphat, -benzoat;
   Metallsalze wie Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
   Oxide wie Tributylzinnoxid, Cu₂O, CuO, ZnO;
   Dialkyldithiocarbamate wie Na- und Zn-Salze von Dialkyldithiocarbamaten, Tetramethylthiuramdisulfid, Kalium-N-methyl-dithiocarbamat;
   Nitrilewie2,4,5,6-Tetrachlorisophthalodinitril,Dinatrium-cyano-dithioimidocarbamat; Chinoline wie 8-Hydroxychinolin und deren Cu-Salze;
   Mucochlorsäure, 5-Hydroxy-2(5H)-furanon;
   4,5-Dichlorodithiazolinon, 4,5-Benzdithiazolinon, 4,5-Trimethylendithiazolinon, 4,5-Dichlor-(3H)-1,2-dithiol-3-on, 3,5-Dimethyl-tetrahydro-1,3,5-thiadiazin-2-thion, N-(2-p-Chlorbenzoylethyl)-hexaminiumchloridKalium-N-hydroxymethyl-N'-methyldithiocarbamat,
   2-Oxo-2-(4-hydroxy-phenyl)acethydroximsäure-chlorid,
   Phenyl-(2-chlor-cyan-vinyl)sulfon,
   Phenyl-(1,2-dichlor-2-cyan-vinyl)sulfon;
   Ag, Zn oder Cu-haltige Zeolithe allein oder eingeschlossen in polymere Wirkstoffe.
Ganz besonders bevorzugt sind Mischungen mit
   Azaconazole, Bromuconazole, Cyproconazole, Dichlobutrazol, Diniconazole, Hexaconazole, Metconazole, Penconazole, Propiconazole, Tebuconazole, Methyl-(E)-methoximino[α-(o-tolyloxy)-o-tolyl)]acetateMethyl-(E)-2-{2-[6-(2-cyanphenoxy)-pyrimidin-4-yl-oxy]phenyl}-3-methoxyacrylat, Methfuroxam, Carboxin, Fenpiclonil, 4-(2,2-Difluoro-1,3-benzodioxol-4-yl)-1H-pyrrol-3-carbonitril, Butenafine, Imazalil, N-Methyl-isothiazolin-3-on, 5-Chlor-N-methylisothiazolin-3-on, N-Octylisothiazolin-3-on, Benzisothiazolinone, N-(2-Hydroxypropyl)-amino-methanol, Benzylalkohol(hemi)-formal, Glutaraldehyd, Omadine, Dimethyldicarbonat, und/oder 3-Iodo-2-propinyl-n-butylcarbamate.

Desweiteren werden auch gut wirksame Mischungen mit den folgenden Wirkstoffen hergestellt:
**Fungizide:**
   Acypetacs, 2-Aminobutane, Ampropylfos, Anilazine, Benalaxyl, Bupirimate, Chinomethionat, Chloroneb, Chlozolinate, Cymoxanil, Dazomet, Diclomezine, Dichloram, Diethofencarb, Dimethirimol, Diocab, Dithianon, Dodine, Drazoxolon, Edifenphos, Ethirimol, Etridiazole, Fenarimol, Fenitropan, Fentin acetate, Fentin Hydroxide, Ferimzone, Fluazinam, Fluromide, Flusulfamide, Flutriafol, Fosetyl, Fthalide, Furalaxyl, Guazatine, Hymexazol, Iprobenfos, Iprodione, Isoprothiolane, Metalaxyl, Methasulfocarb, Nitrothal-isopropyl, Nuarimol, Ofurace, Oxadiyl, Perflurazoate, Pencycuron, Phosdiphen, Pimaricin, Piperalin, Procymidone, Propamocarb, Propineb, Pyrazophos, Pyrifenox, Pyroquilon, Quintozene, Tar Oils, Tecnazene, Thicyofen, Thiophanate-methyl, Tolclofos-methyl, Triazoxide, Trichlamide, Tricyclazole, Triforine, Vinclozolin.
**Insektizide:**
   Phosphorsäureester wie Azinphos-ethyl, Azinphos-methyl, α-1(4-Chlorphenyl)-4-(O-ethyl, S-propyl)phosphoryloxy-pyrazol, Chlorpyrifos, Coumaphos, Demeton, Demeton-S-methyl, Diazinon, Dichlorvos, Dimethoate, Ethoate, Ethoprophos, Etrimfos, Fenitrothion, Fenthion, Heptenophas, Parathion, Parathion-methyl, Phosalone, Phoxim, Pirimiphos-ethyl, Pirimiphos-methyl, Profenofos, Prothiofos, Sulfprofos, Triazophos und Trichlorphon;
   Carbamate wie Aldicarb, Bendiocarb, α-2-(1-Methylpropyl)-phenylmethylcarbamat, Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb; Organosiliciumverbindungen,vorzugsweiseDimethyl(phenyl)silyl-methyl-3-phenoxybenzylether wie Dimethyl-(4-ethoxyphenyl)-silylmethyl-3-phenoxybenzylether oder (Dimethylphenyl)-silyl-methyl-2-phenoxy-6-pyridylmethyletherwiez.B.Dimethyl-(9-ethoxy-phenyl)-silylmethyl-2-phenoxy-6-pyridylmethylether oder [(Phenyl)-3-(3-phenoxyphenyl)-propyl](dimethyl)-silane wie z.B. (4-Ethoxyphenyl)-[3-(4-fluoro-3-phenoxyphenyl-propyl]dimethyl-silan, Silafluofen;
   Pyrethroide wie Allethrin, Alphamethrin, Bioresmethrin, Byfenthrin, Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluor-methylvinyl)cyclopropancarboxylat, Fenpropathrin, Fenfluthrin, Fenvalerate, Flucythrinate, Flumethrin, Fluvalinate, Permethrin, Resmethrin und Tralomethrin;
   Nitroimine und Nitromethylene wie 1-[(6-Chlor-3 -pyridinyl)-methyl]-4,5-dihydro-N-nitro-1H-imidazol-2-amin (Imidacloprid), N-[(6-Chlor-3-pyridyl)methyl-]N²-cyano-N¹-methylacetamide (NI-25);
   Abamectin, AC 303, 630, Acephate, Acrinathrin, Alanycarb, Aldoxycarb, Aldrin, Amitraz, Azamethiphos, Bacillus thuringiensis, Phosmet, Phosphamidon, Phosphine, Prallethrin, Propaphos, Propetamphos, Prothoate, Pyraclofos, Pyrethrins, Pyridaben, Pyridafenthion, Pyriproxyfen, Quinalphos, RH-7988, Rotenone, Sodium fluoride, Sodium hexafluorosilicate, Sulfotep, Sulfuryl fluoride, Tar Oils, Teflubenzuron, Tefluthrin, Temephos, Terbufos, Tetrachlorvinphos, Tetramethrin, O-2-tert.-Butylpyrimidin-5-yl-o-isopropyl-phosphorothiate, Thiocyclam, Thiofanox, Thiometon, Tralomethrin, Triflumuron, Trimethacarb, Vamidothion, Verticillium Lacanii, XMC, Xylylcarb, Benfuracarb, Bensultap, Bifenthrin, Bioallethrin, MERbioallethrin (S)-cyclopentenyl isomer, Bromophos, Bromophos-ethyl, Buprofezin, Cadusafos, Calcium Polysulfide, Carbophenothion, Cartap, Chinomethionat, Chlordane, Chlorfenvinphos, Chlorfluazuron, Chlormephos, Chloropicrin, Chlorpyrifos, Cyanophos, Beta-Cyfluthrin, Alpha-cypermethrin, Cyophenothrin, Cyromazine, Dazomet, DDT, Demeton-S-methylsulphon, Diafenthiuron, Dialifos, Dicrotophos, Diflubenzuron, Dinoseb, Deoxabenzofos, Diaxacarb, Disulfoton, DNOC, Empenthrin, Endosulfan, EPN, Esfenvalerate, Ethiofencarb, Ethion, Etofenprox, Fenobucarb, Fenoxycarb, Fensulfothion, Fipronil, Flucycloxuron, Flufenprox, Flufenoxuron, Fonofos, Formetanate, Formothion, Fosmethilan, Furathiocarb, Heptachlor, Hexaflumuron, Hydramethylnon, Hydrogen Cyanide, Hydroprene, IPSP, Isazofos, Isofenphos, Isoprothiolane, Isoxathion, Iodfenphos, Kadethrin, Lindane, Malathion, Mecarbam, Mephosfolan, Mercurous, chloride, Metam, Metarthizium, anisopliae, Methacrifos, Methamidophos, Methidathion, Methiocarb, Methoprene, Methoxychlor, Methyl isothiocyanate, Metholcarb, Mevinphos, Monocrotophos, Naled, Neodiprion sertifer NPV, Nicotine, Omethoate, Oxydemeton-methyl, Pentachlorophenol, Petroleum oils, Phenothrin, Phenthoate, Phorate;
**Molluscicide:**
   Fentinacetate, Metaldehyde, Methiocarb. Niclosamide, Thiodicarb, Trimethacarb.
**Algicide:**
   Coppersulfate, Dichlororphen, Endothal, Fentinacetate, Quinoclamine.
**Herbicide:**
   acetochlor, acifluorfen, aclonifen, acrolein, alachlor, alloxydim, ametryn, amidosulfuron, amitrole, ammonium sulfamate, anilofos, asulam atrazine, aziptrotryne, benazolin, benfluralin, benfuresate, bensulfuron, bensulfide, bentazone, benzofencap, benzthiazuron, bifenox, bilanafos, borax, dichlorprop, dichlorprop-P, diclofop, diethatyl, difenoxuron, difenzoquat, diflufenican, dimefuron, dimepiperate, dimethachlor, dimethametryn, dimethipin, dimethylarsinic acid, dinitramine, dinoseb, dinoseb, dinoseb acetate, dinoseb, bromacil, bromobutide, bromofenoxim, bromoxynil, butachlor, butamifos, fuenachlor, butralin, butylate, carbetamide, CGA 184927, chlormethoxyfen, chloramben, chlorbromuron, chlorbutam, chlorfurenol, chloridazon, chlorimuron, chlornitrofen, chloroacetic acid, achloropicrin, chlorotoluron, chloroxuron, chlorprepham, chlorsulfuron, chlorthal, chlorthiamid, cinmethylin, cinofulsuron, clethodim, clomazone, clomeprop, clopyralid, cyanamide, cyanazine, dinoseb acetate, dinoterb, diphenamid, dipropetryn, diquat, dithiopyr, diduron, DNOC, PPX-A 788, DPX-E96361, DSMA, eglinazine, endothal, EPTC, esprocarb, ethalfluralin, ethidimuron, ethofumesate, fenoxaprop, fenoxaprop-P, fenuron, flamprop, flamprop-M, flazasulfuron, fluazifop, fluazifop-P, fluchloralin, flumeturon, fluorocgycofen, fluoronitrofen, flupropanate, flurenol, fluridone, flurochloridone, fluoroxypyr, cycloate, cycloxydim, 2,4-D, daimuron, dalapon, dazomet, 2,4-DB, desmedipham, desmetryn, dicamba, dichlorbenil, isoproturon, isouron, isoxaben, isoxapyrifop, lactofen, lenacil, linuron, LS830556, MCPA, MCPA-thioethyl, MCPB, mecoprop, mecoprop-P, mefenacet, mefluidide, metam, metamitron, metazachlor, methabenzthiazuron, methazole, methoproptryne, methyldymron, methylisothiocyanate, metobromuron, fomosafen, fosamine, furyloxyfen, glufosinate, glyphosate, haloxyfop, hexazinone, imazamethabenz, imazapyr, imazaquin, imazethapyr, ioxynil, isopropalin, propyzamide, prosulfocab, pyrazolynate, pyrazolsulfuron, pyrazoxyfen, pyributicarb, pyridate, quinclorac, quinmerac, quinocloamine, quizalofop, quzizalofop-P, S-23121, sethoxydim, sifuron, simazine, simetryn, SMY 1500, sodium chlorate, sulfometuron, tar oils, TCA, metolachlor, metoxuron, metribzin, metsulfuron, molinate, monalide, monolinuron, MSMA, naproanilide, napropamide, naptalam, neburon, nicosulfuron, nipyraclofen, norflurazon, orbencarb, oaryzalin, oxadiazon, oxyfluorfen, paraquat, pebulate, pendimethalin, pentachlorophenol, pentaochlor, petroleum oils, phenmedipham, picloram, piperophos, pretilachlor, primisulfuron, prodiamine, proglinazine, propmeton, prometryn, propachlor, tebutam, tebuthiuron, terbacil, terbumeton, terbuthylazine, terbutryn, thiazafluoron, thifensulfuron, thiobencarb, thiocarbazil, tioclorim, tralkoxydim, tri-allate, triasulfuron, tribenzuron, triclopyr, tridiphane, trietazine, trifluralin, IBI-C4874 vernolate, propanil, propaquizafop, propazine, propham.

Die Gewichtsverhältnisse der Wirkstoffe in diesen Wirkstoffkombinationen können in relativ großen Bereichen variiert werden.

Vorzugsweise erhalten die Wirkstoffkombinationen den Wirkstoff zu 0,1 bis 99,9 %, insbesondere zu 1 bis 75 %, besonders bevorzugt 5 bis 50 %, wobei der Rest zu 100 % durch einen oder mehrere der obengenannten Mischungspartner ausgefüllt wird.

Die zum Schutz der technischen Materialien verwendeten mikrobiziden Mittel oder Konzentrate enthalten den Wirkstoff bzw. die Wirkstoffkombination in einer Konzentration von 0,01 und 95 Gew.-%, insbesondere 0,1 bis 60 Gew.-%.

Die Anwendungskonzentrationen der zu verwendenden Wirkstoffe bzw. der Wirkstoffkombinationen richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden.

Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe bzw. Mittel ermöglichen in vorteilhafter Weise, die bisher verfügbaren mikrobiziden Mittel durch effektivere zu ersetzen. Sie zeigen eine gute Stabilität und haben in vorteilhafter Weise ein breites Wirkungsspektrum.

Die nachfolgenden Beispiele dienen zur Verdeutlichung der Erfindung. Die Erfindung ist nicht auf die Beispiele beschränkt.

### Herstellungsbeispiele

### Beispiel 1 (Vorstufe der Formel (II))

10,3 g (0,06 Mol) α-Formyl-hexansäureethylester und 5,5 g (0,06 Mol) Thiosemicarbazid werden in 200 ml Ethanol vorgelegt und 3 Stunden bei 80°C gerührt.

Dann wird auf Raumtemperatur gebracht und unter Rühren 6,9 g Kalium-tert.-butylat zugegeben, um danach 4 Stunden bei Raumtemperatur weiterzurühren. Anschließend wird der Kolbeninhalt in ein Gemisch aus 800 ml Wasser und 50 ml conc. Salzsäure eingerührt und der erhaltene Niederschlag abgesaugt. Nach gründlichem Waschen mit Wasser wird das Produkt im Trockenschrank bis zur Gewichtskonstanz belassen. Durch Umkristallisieren aus Ethanol kann die Verbindung gereinigt werden.
Ausbeute: 9,4 g (80,3 % d. Th.)
Fp.: 139-141°C

### (Endstufe der Formel (I))

47,1 g (0,236 mol) der Vorstufe in 450 ml Tetrahydrofuran werden bei 25°C mit 26,48 g (0,236 mol) Kalium-tert.-butylat versetzt. Nach Kühlen auf 0°C werden 30 ml 50 % Chloracetaldehyd zugetropft und 16 Stunden bei 25°C gerührt. Nach Eingießen in 750 ml 10 % HCl wird mit Methylenchlorid extrahiert, getrocknet, das Lösungsmittel verdampft und der Rückstand mit Diisopropylether verrührt.

Man erhält 26,1 g der Zielverbindung I/007 vom Schmelzpunkt 89 bis 91°C. Rekristallisation erhält den Schmelzpunkt auf 91°C.

Analog Beispiel 1 und gemäß den allgemeinen Beschreibungen werden die folgenden Verbindungen der Formel (I) erhalten, dabei ist es auch möglich, die Aufarbeitung der Vorstufe direkt und unter Einsparung der Base die Endstufe herzustellen:

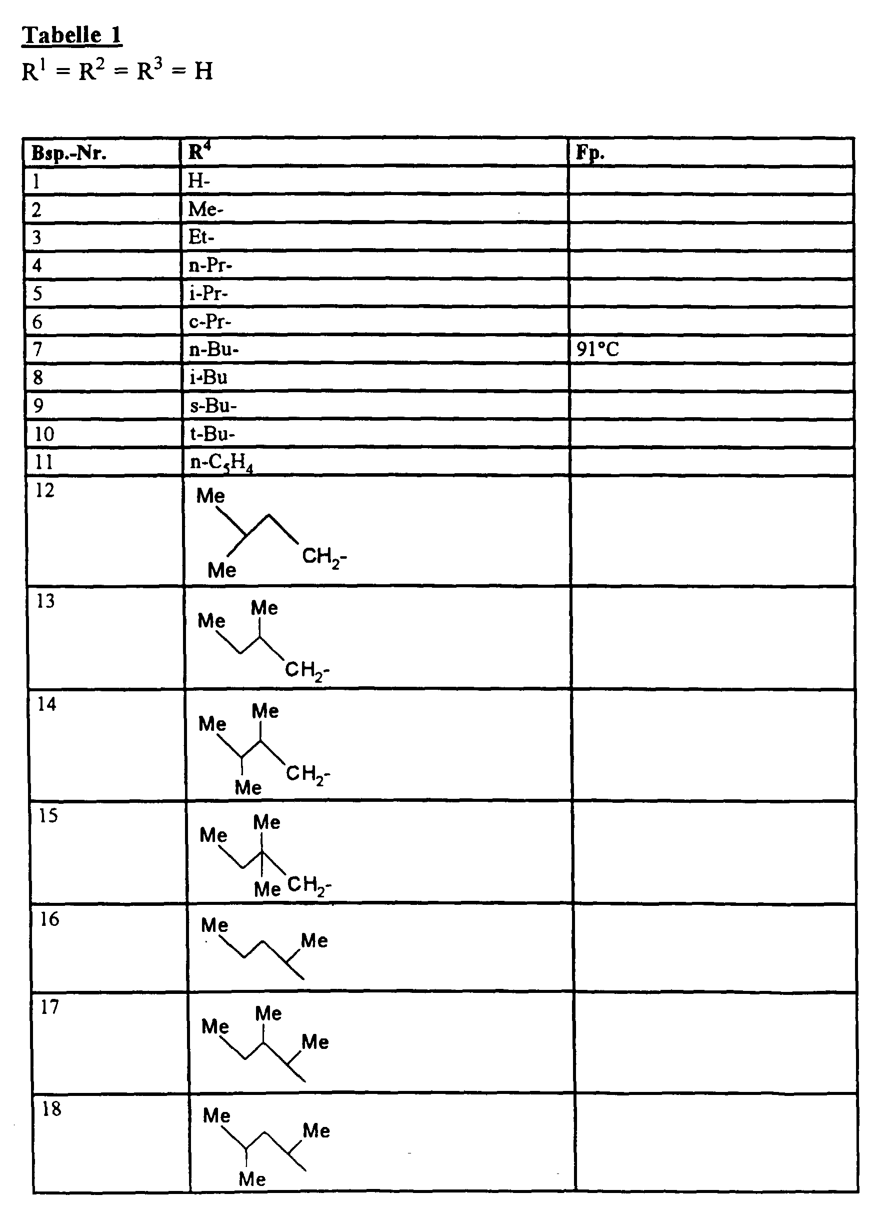

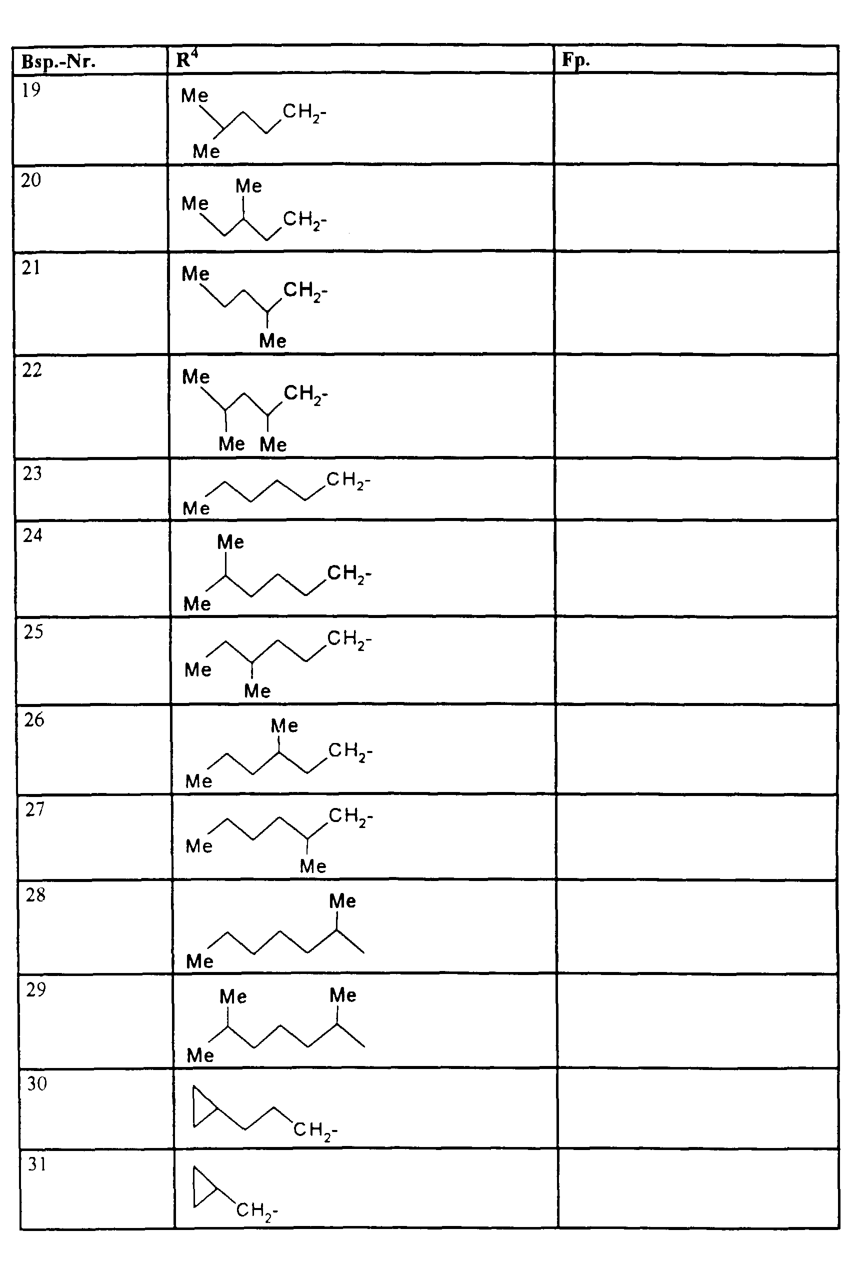

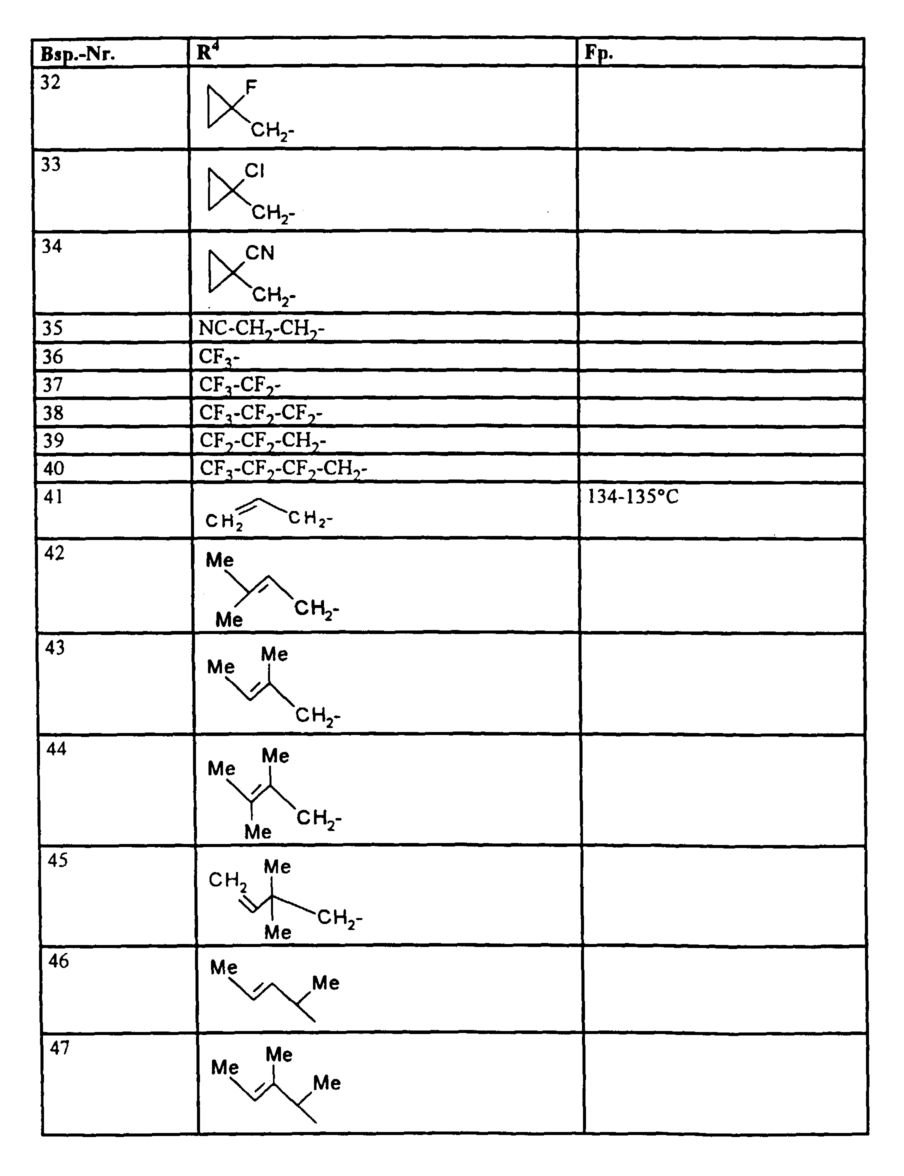

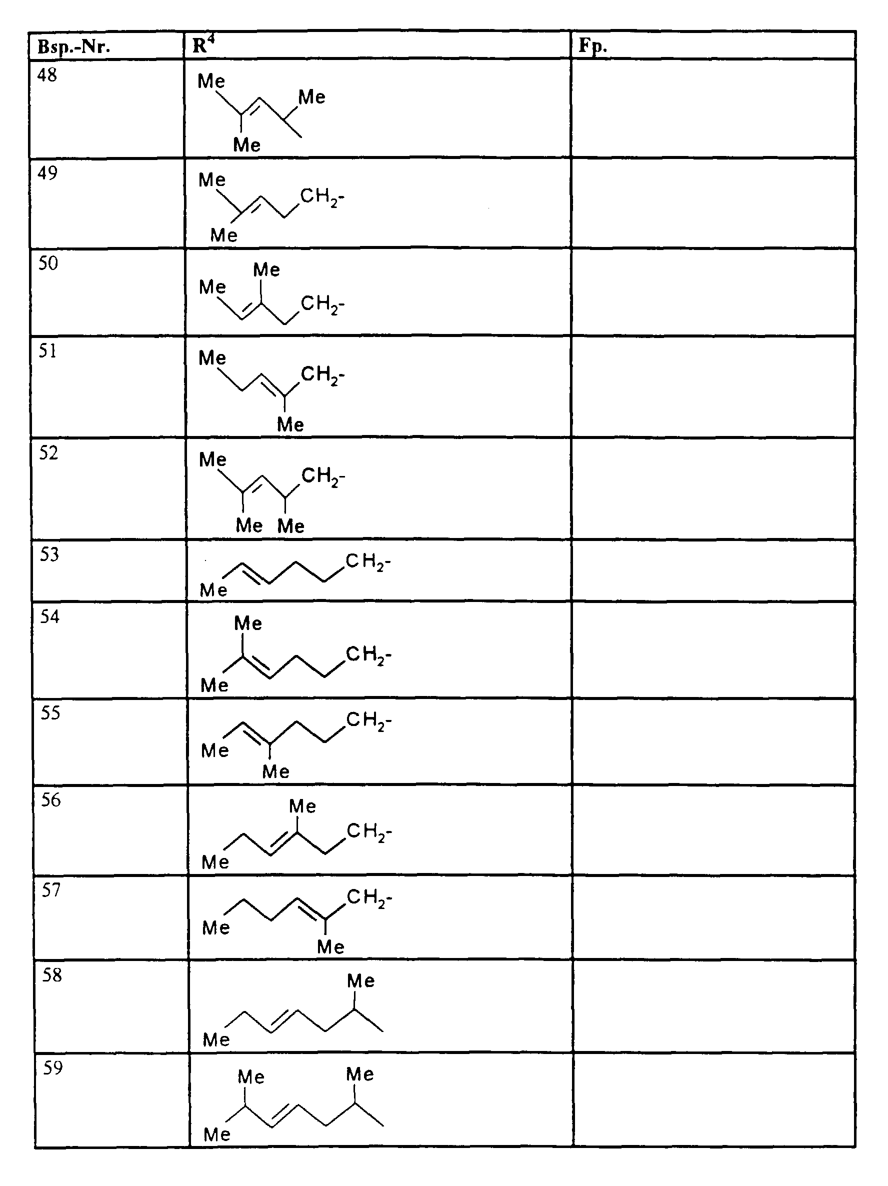

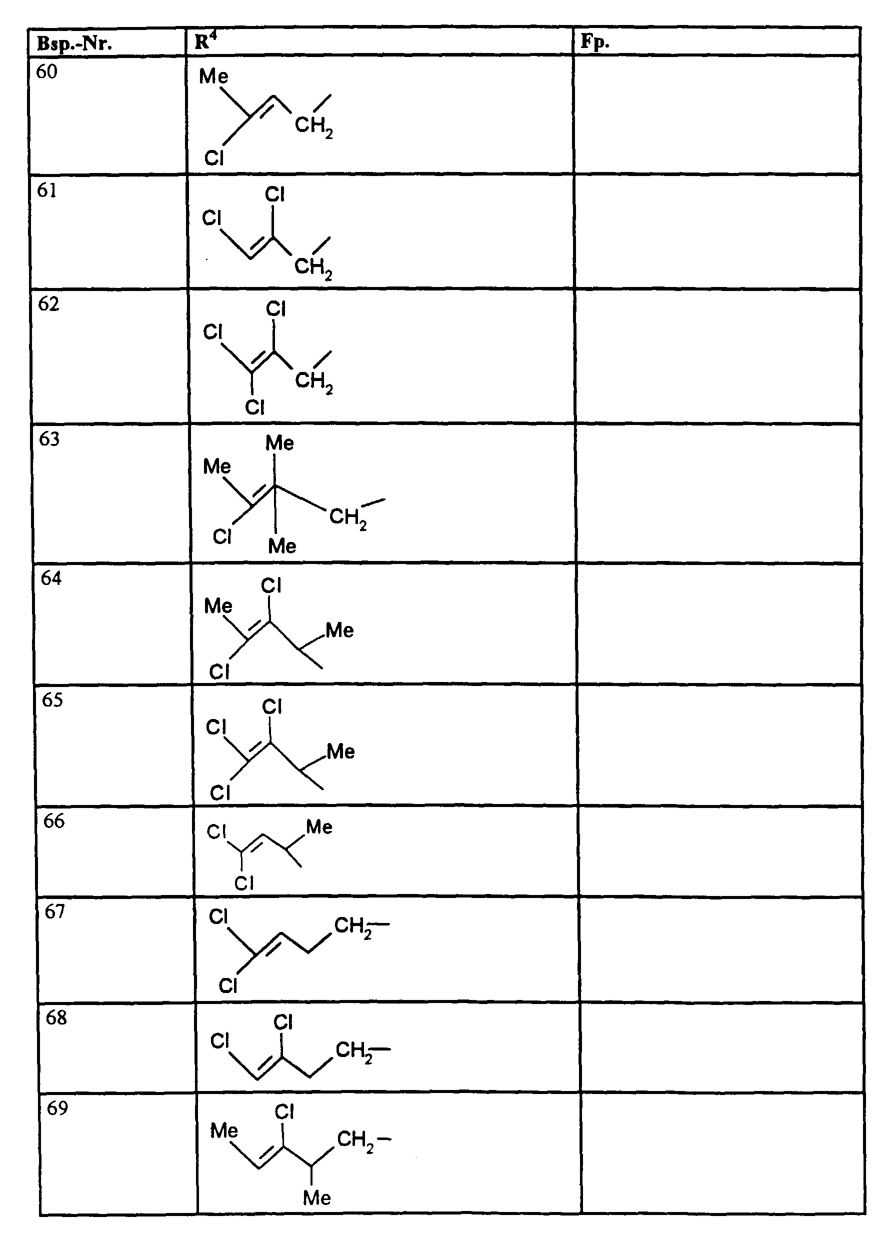

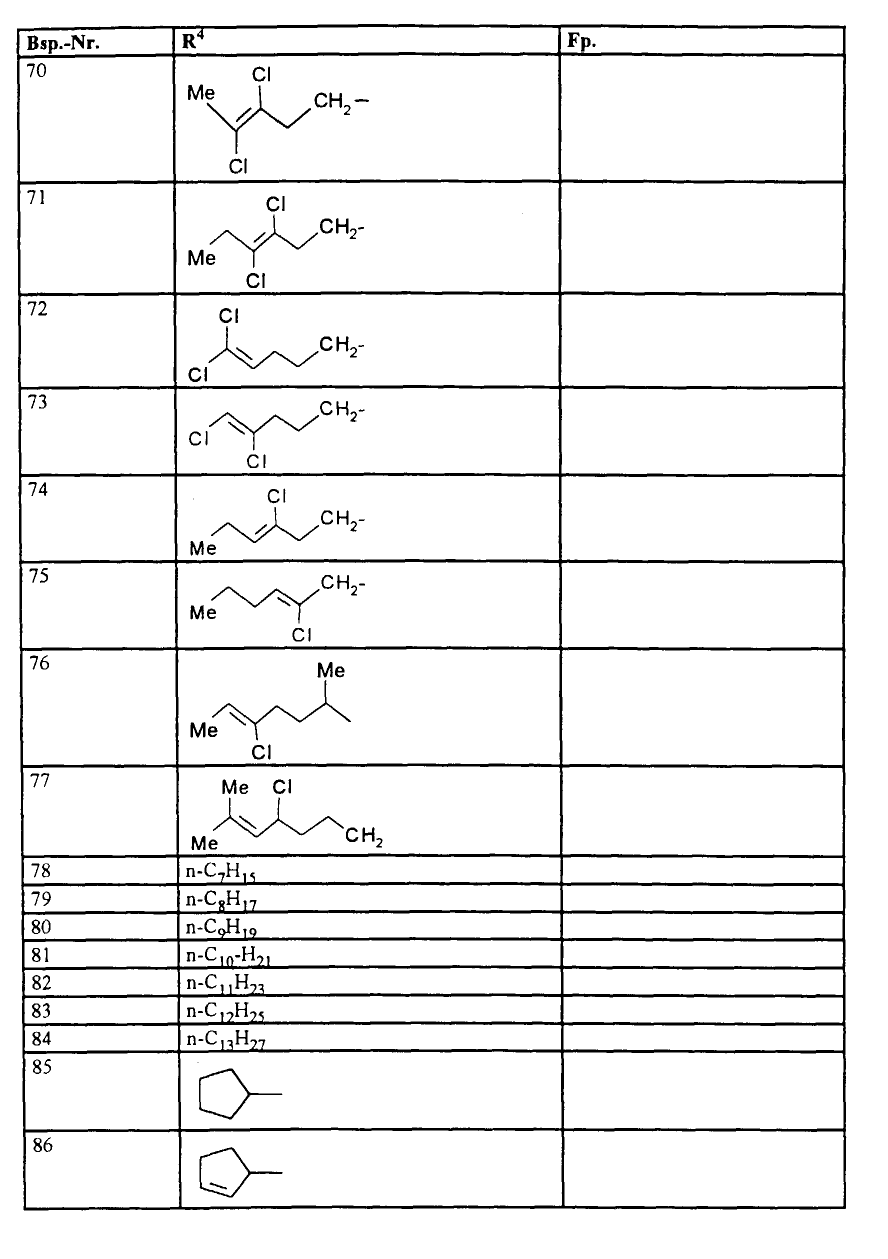

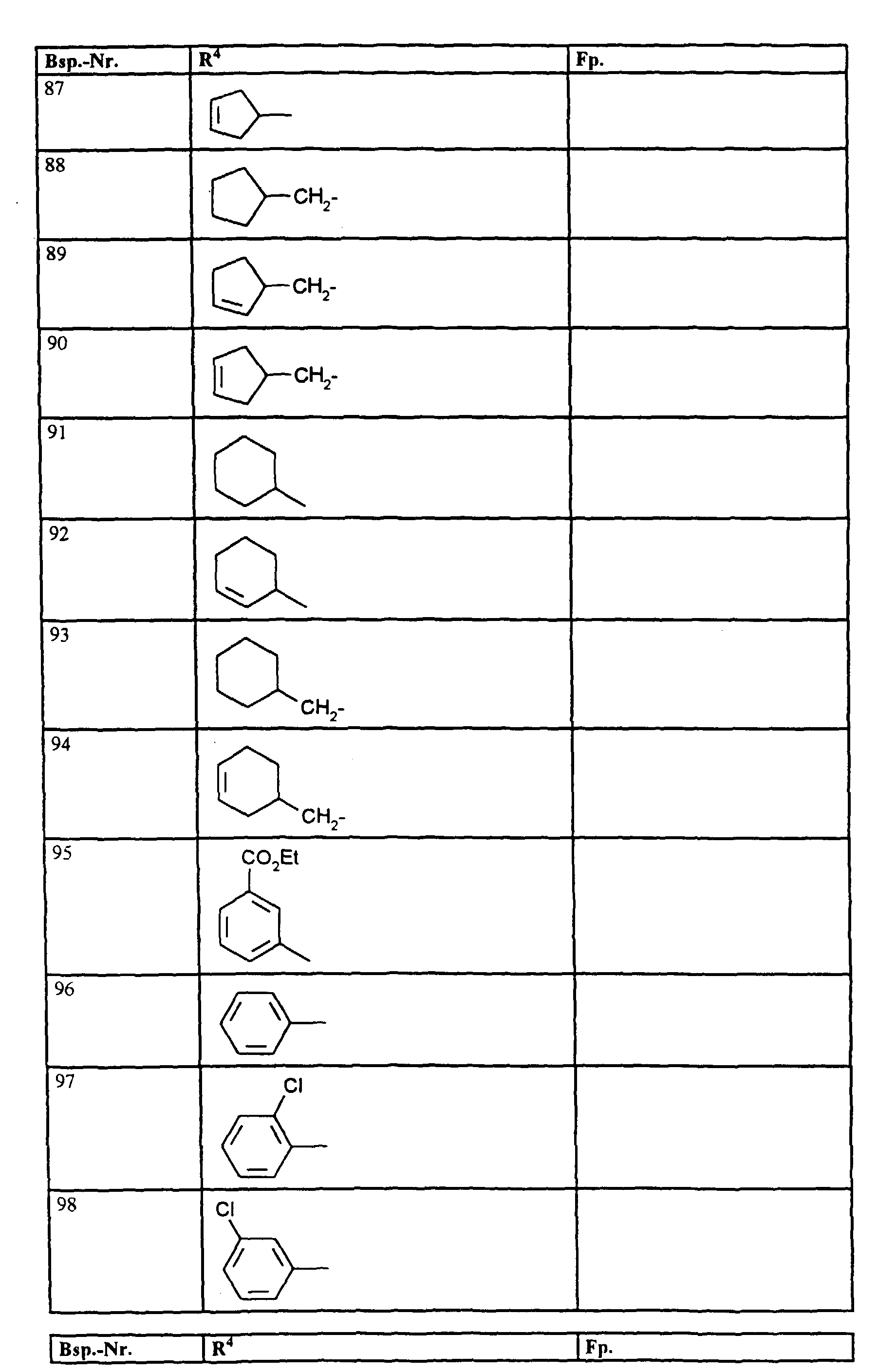

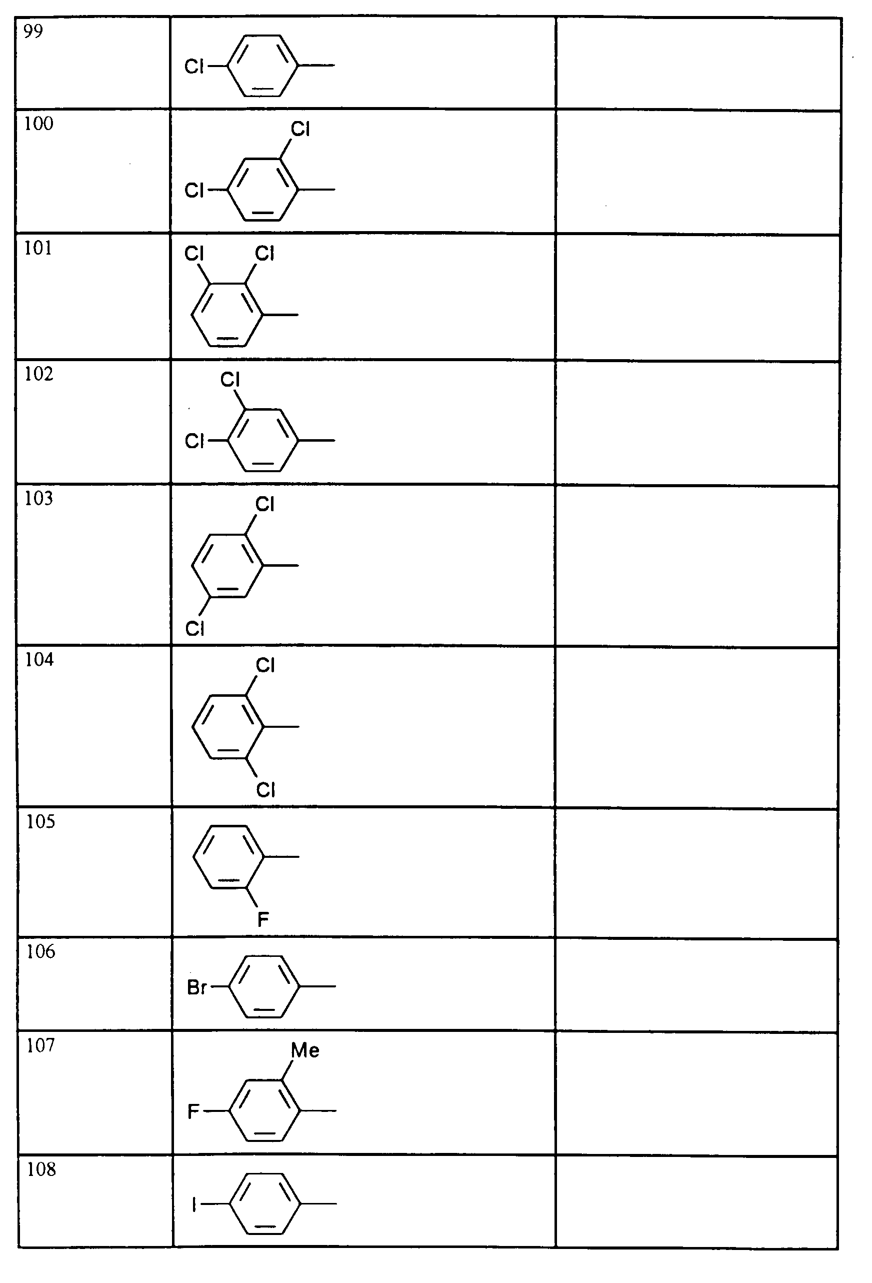

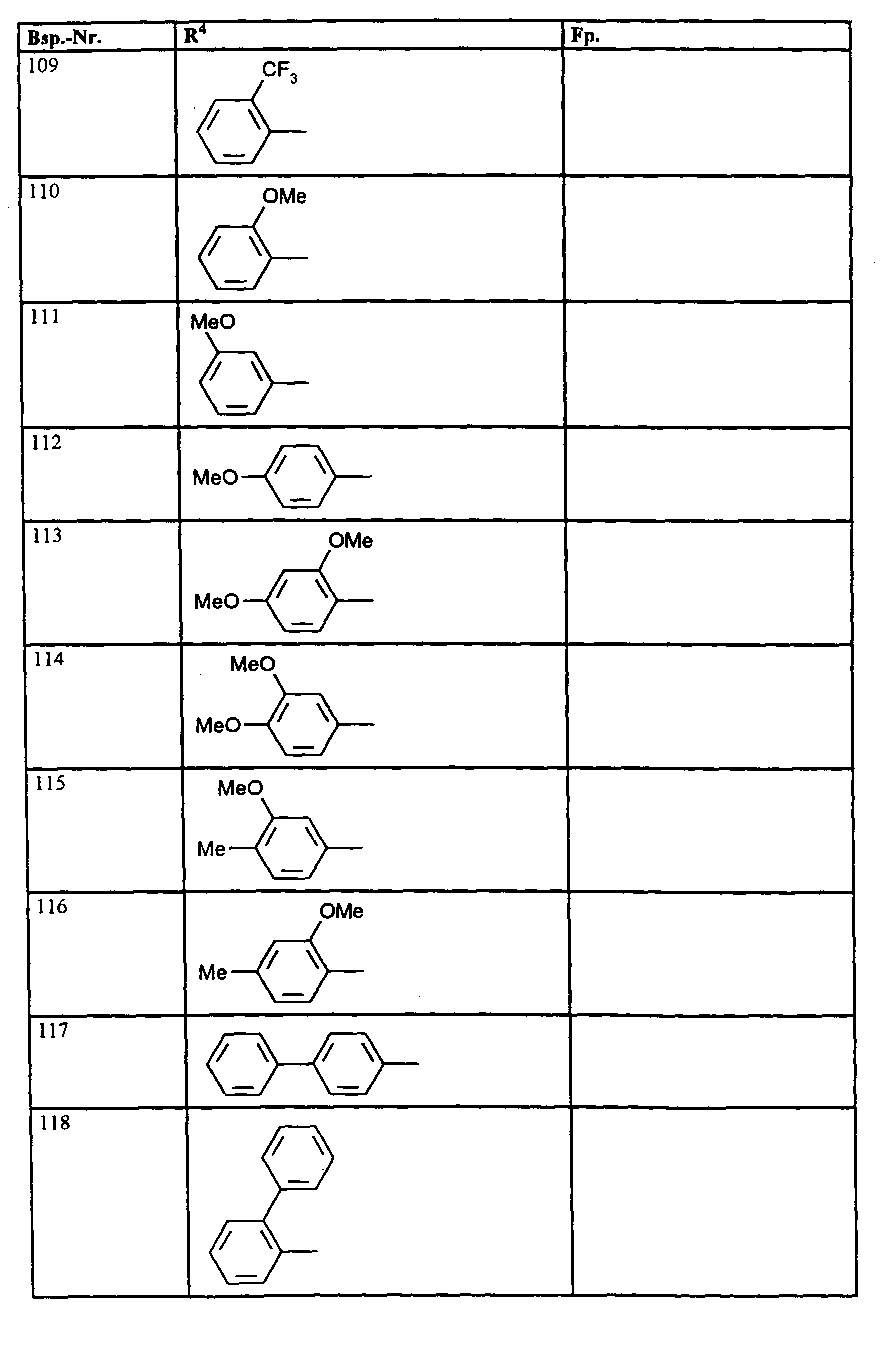

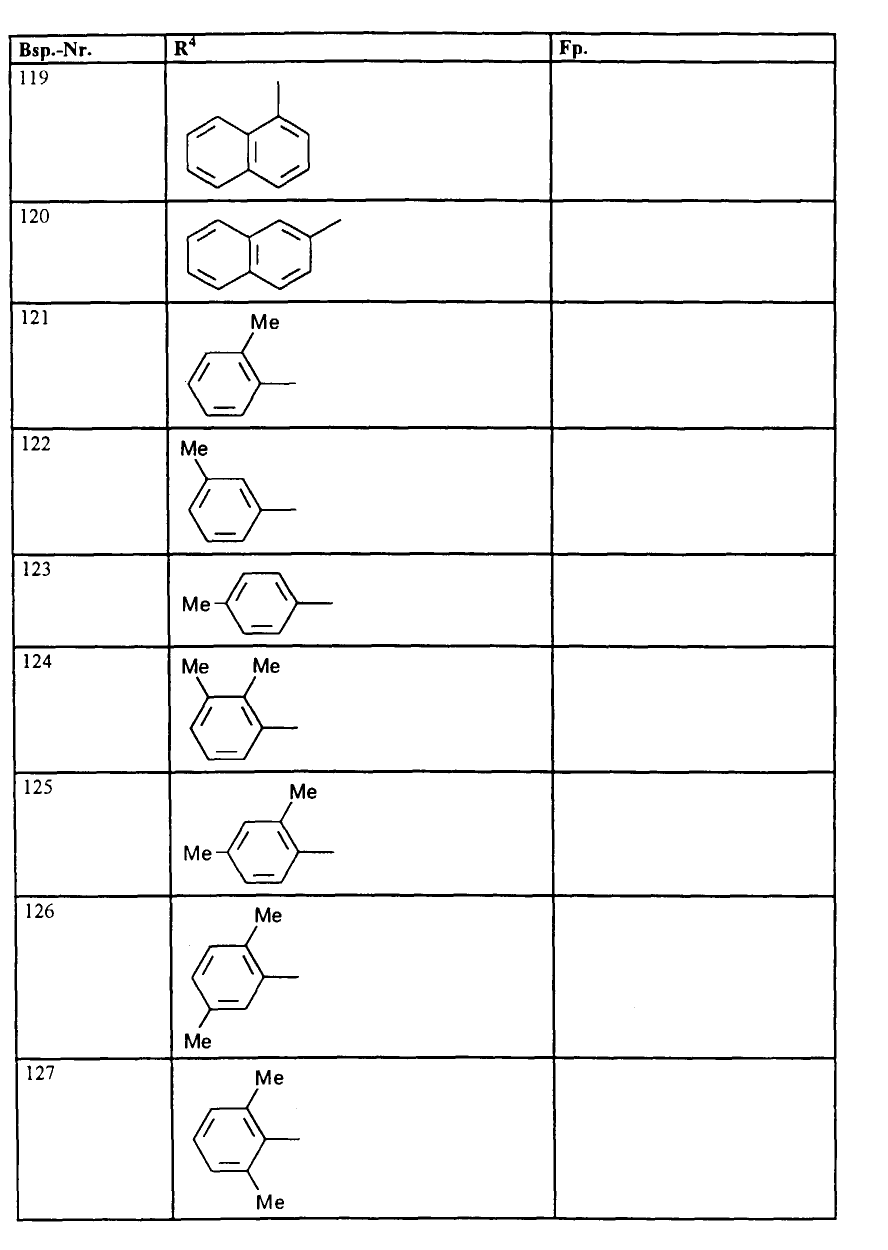

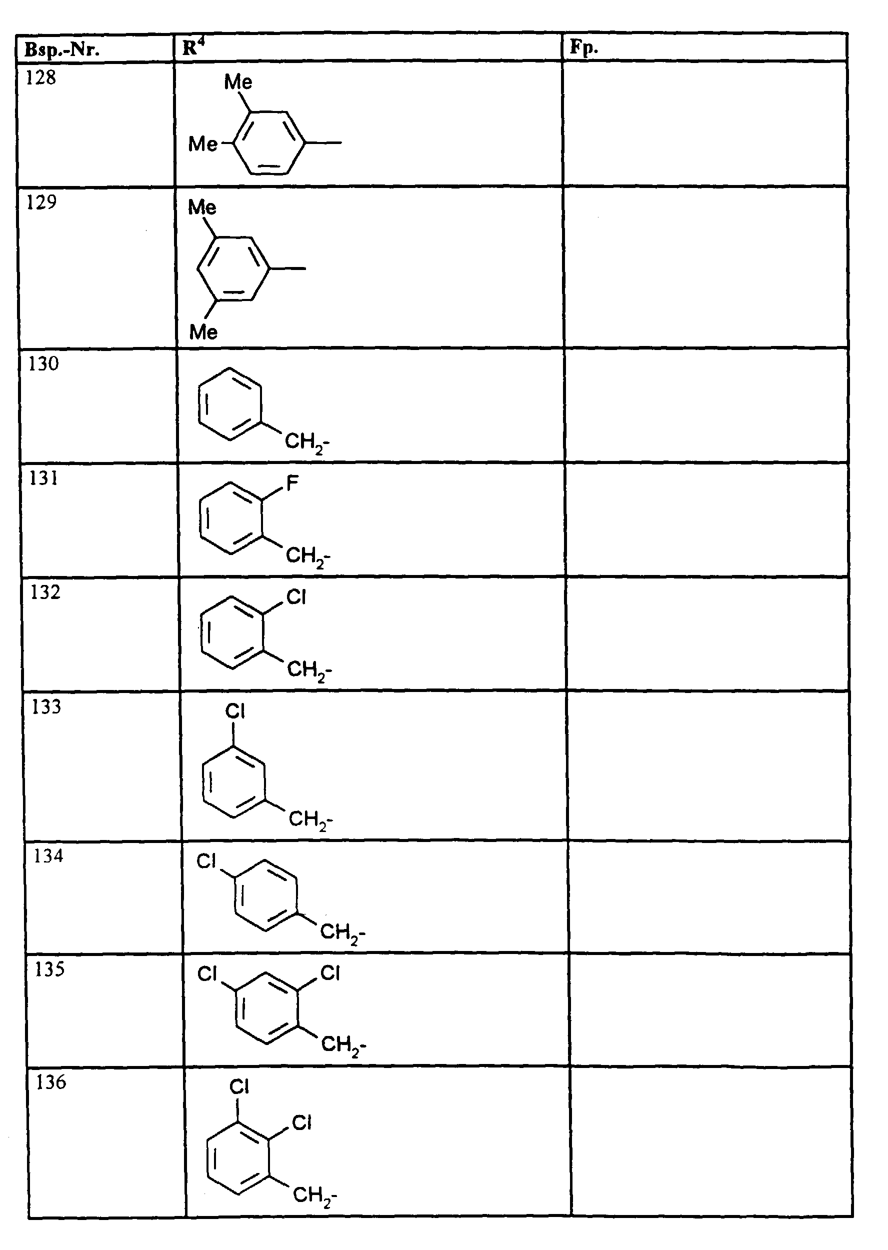

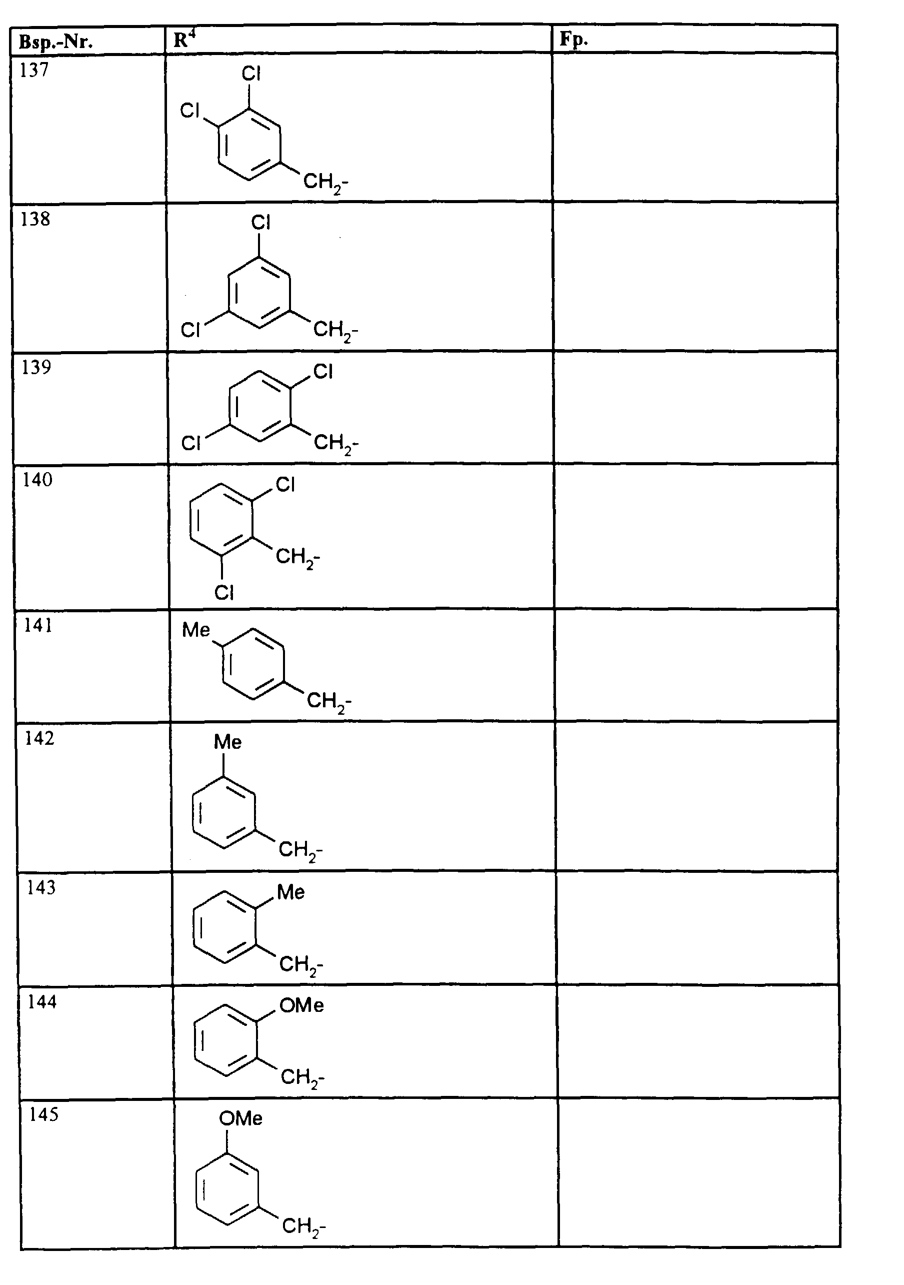

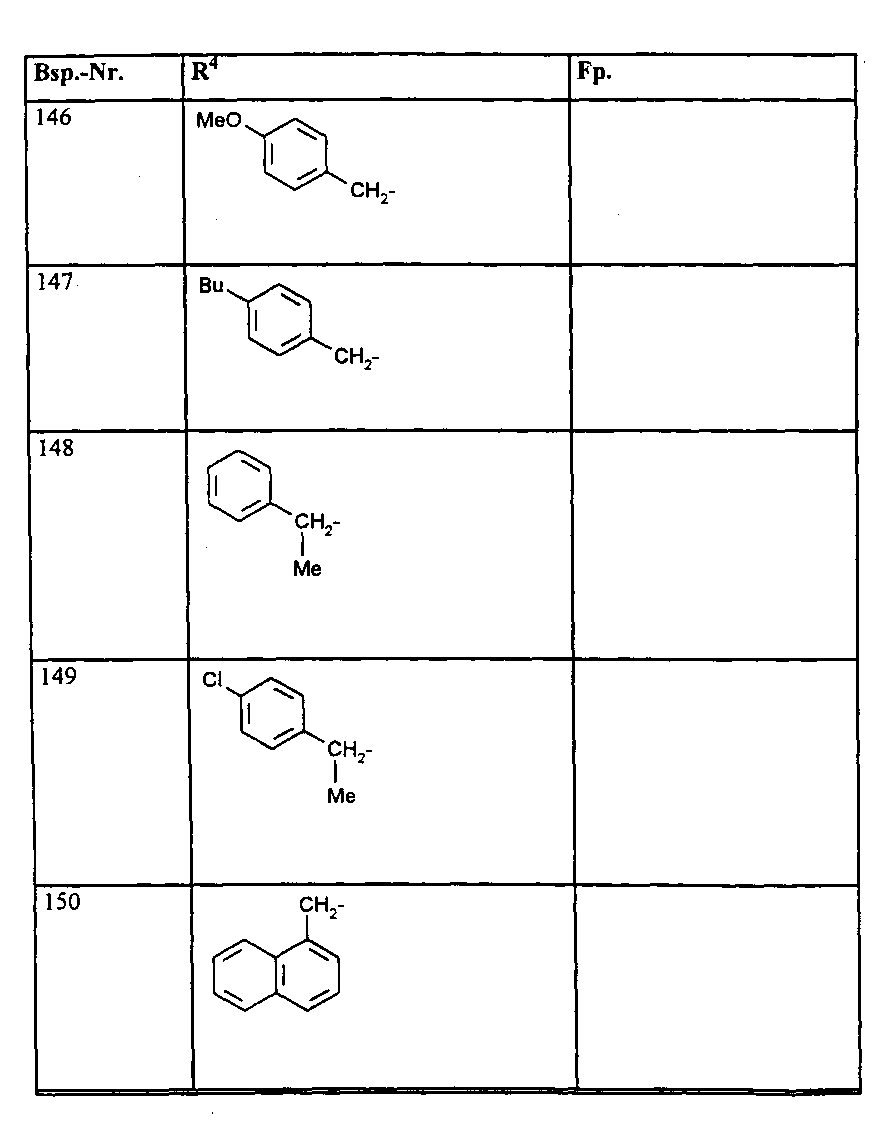

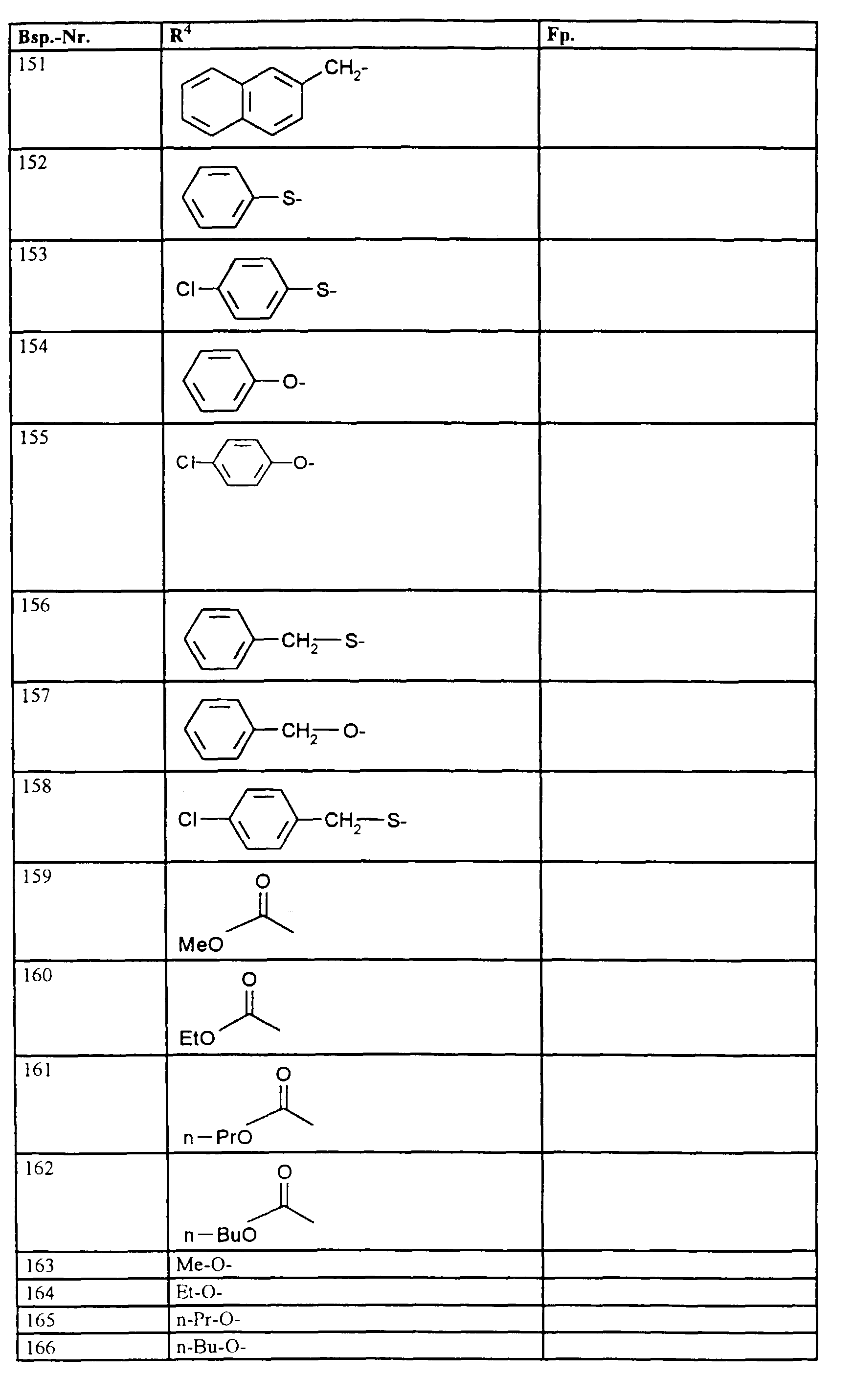

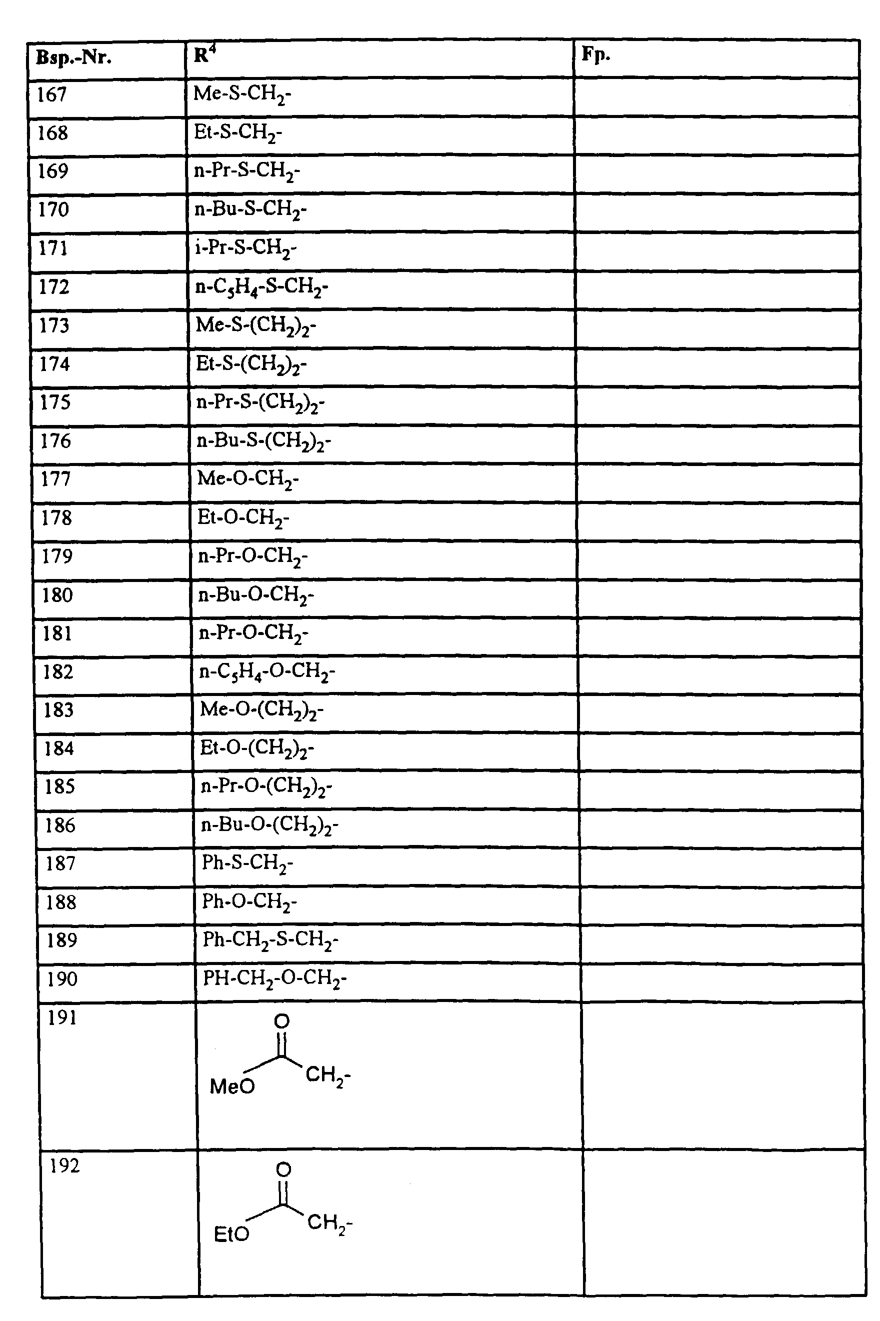

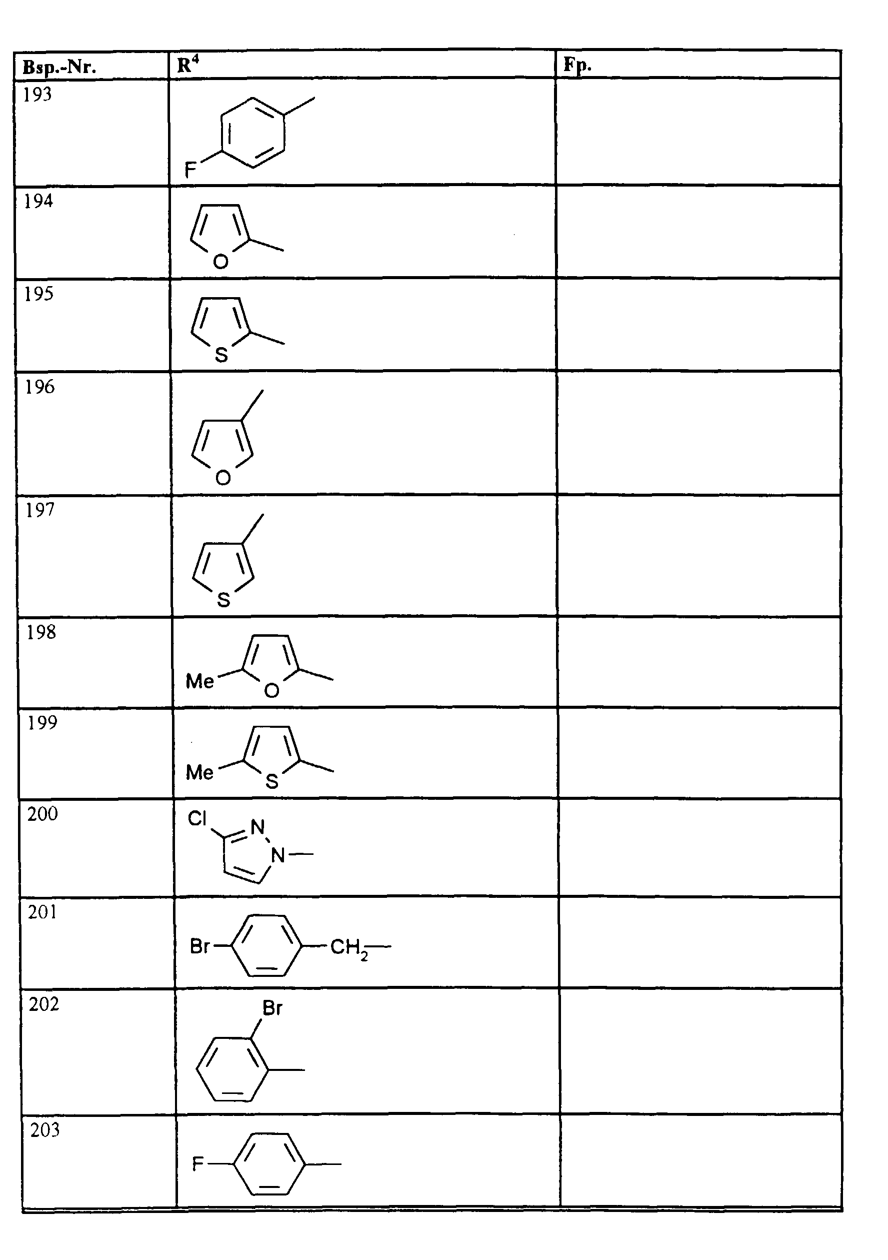

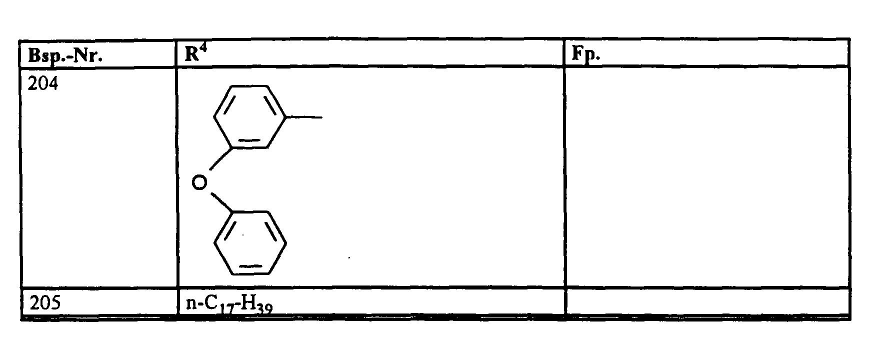

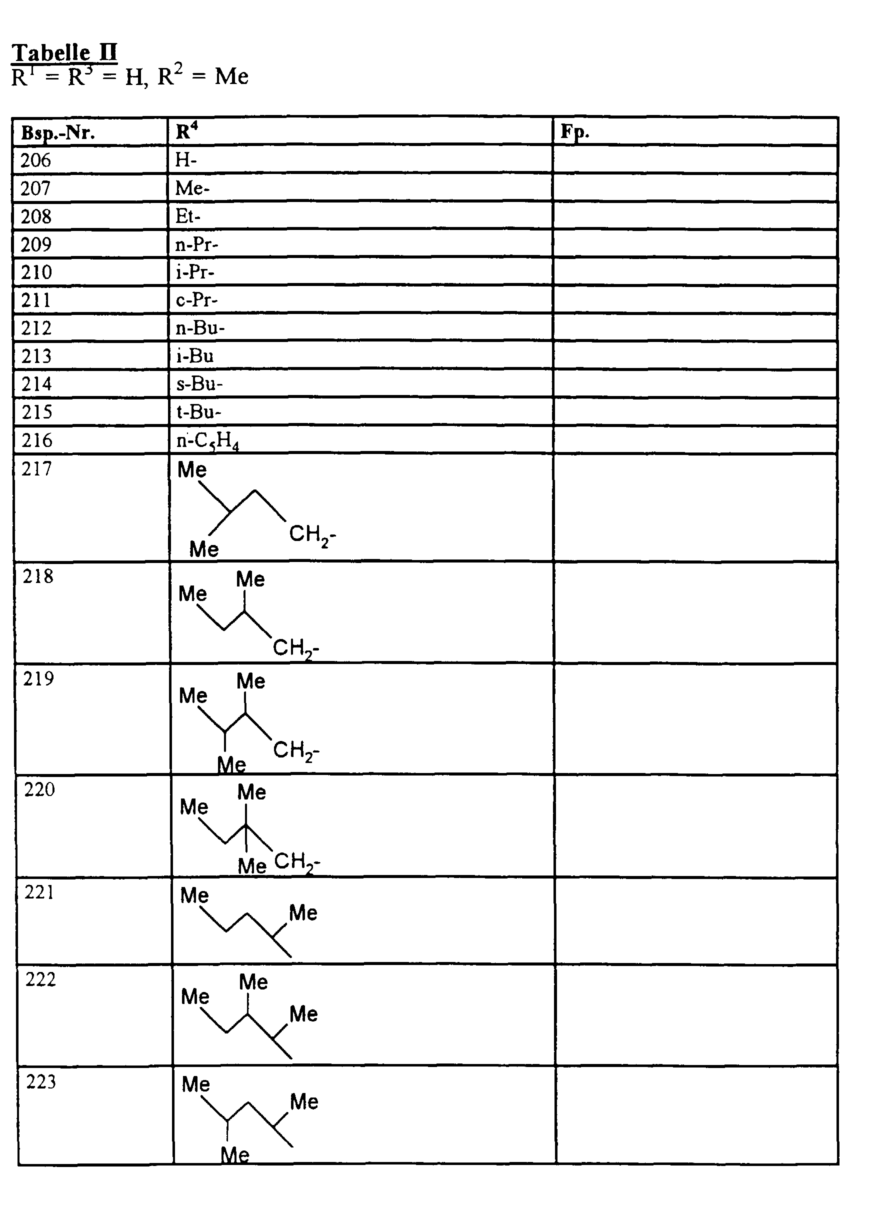

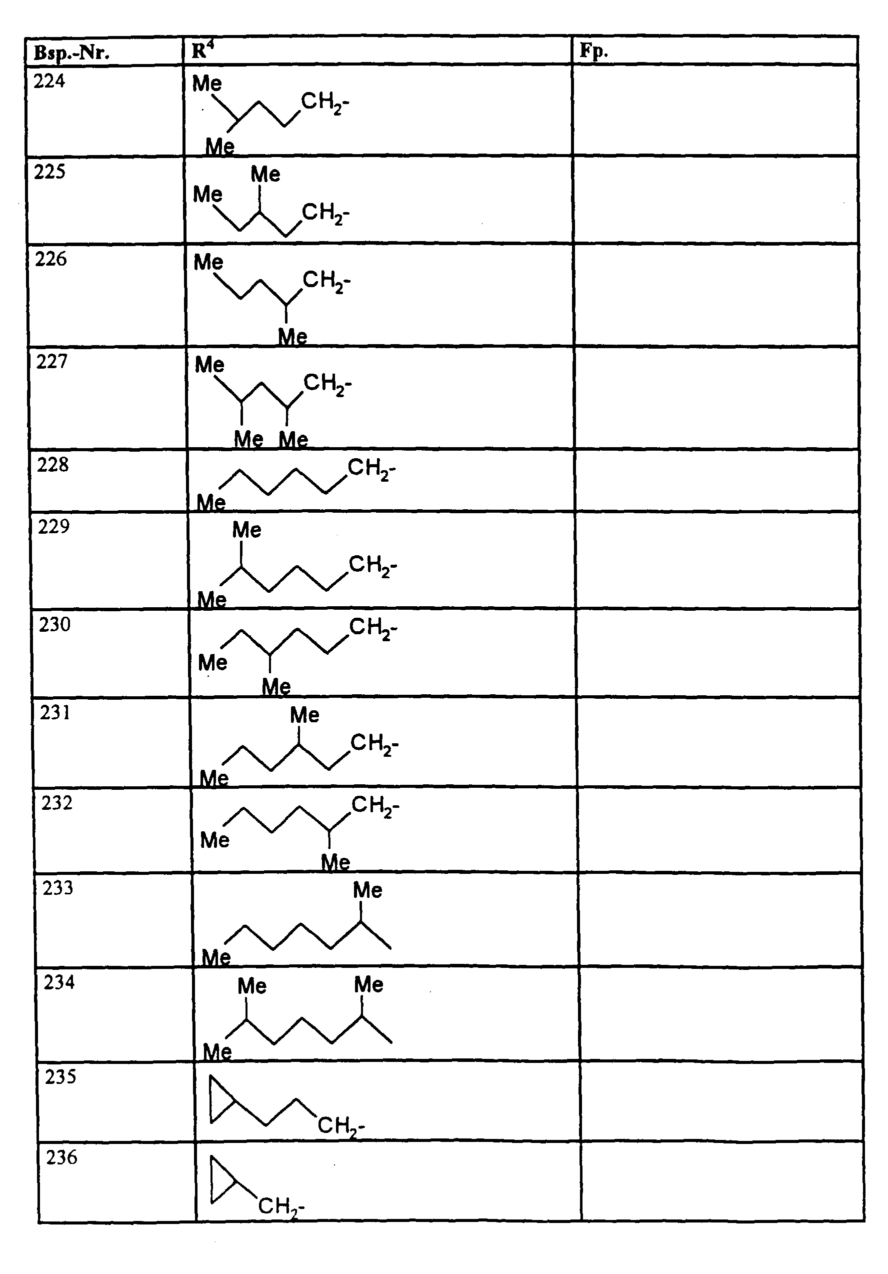

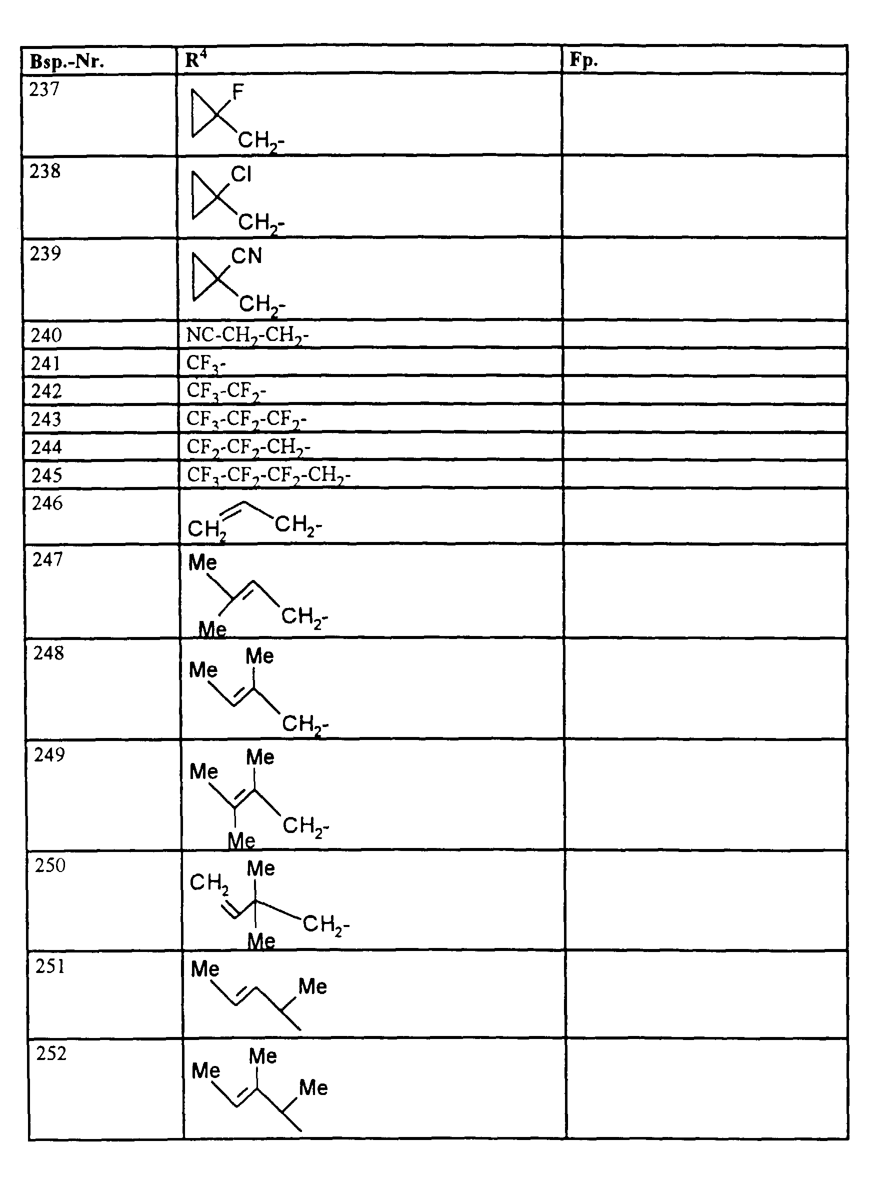

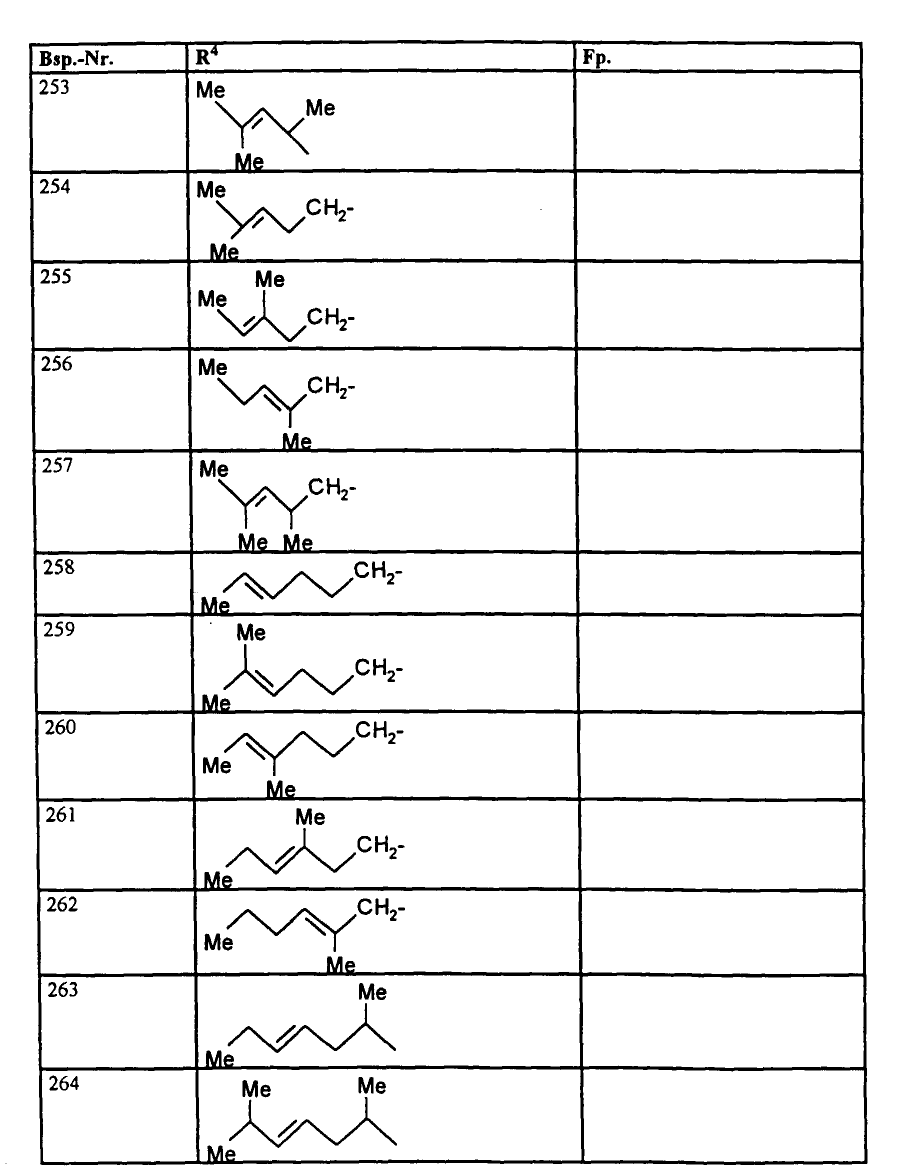

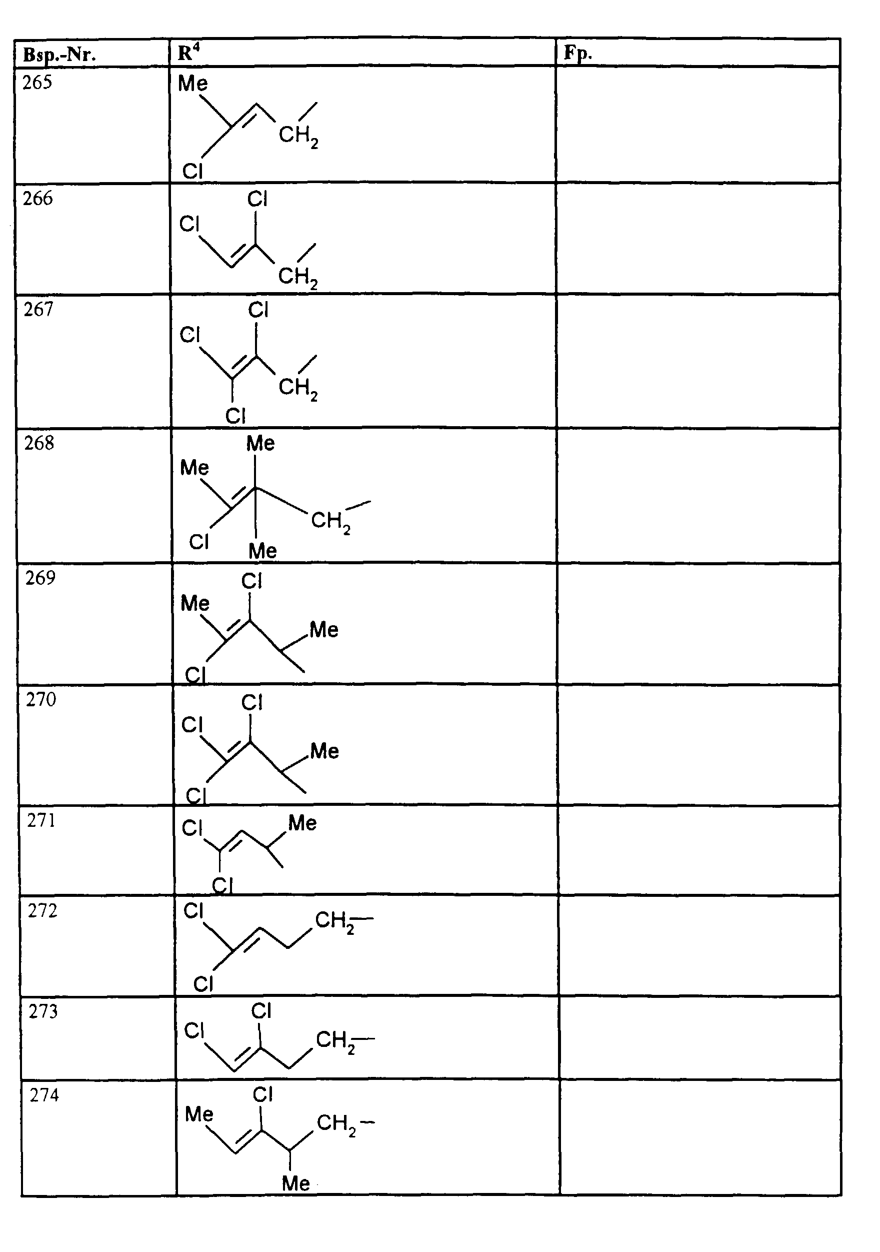

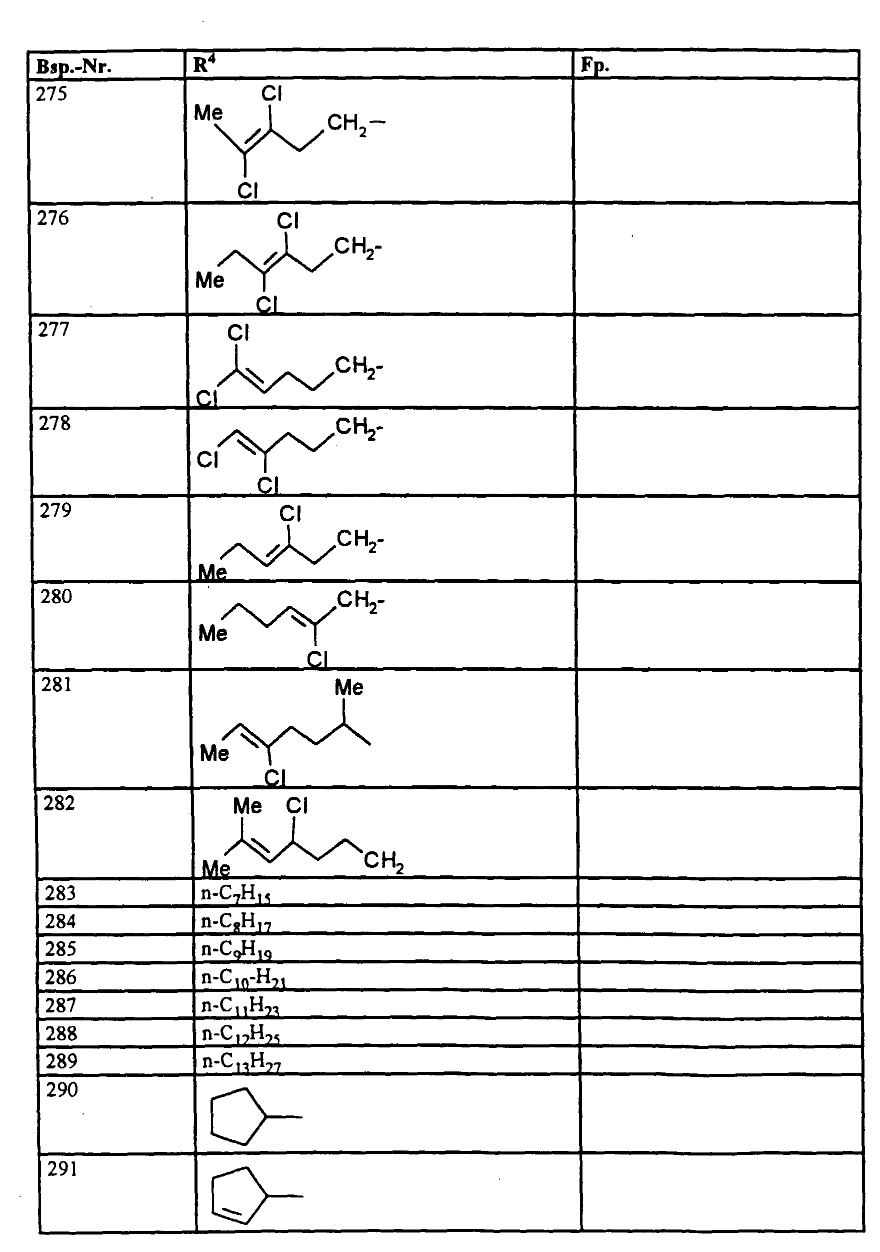

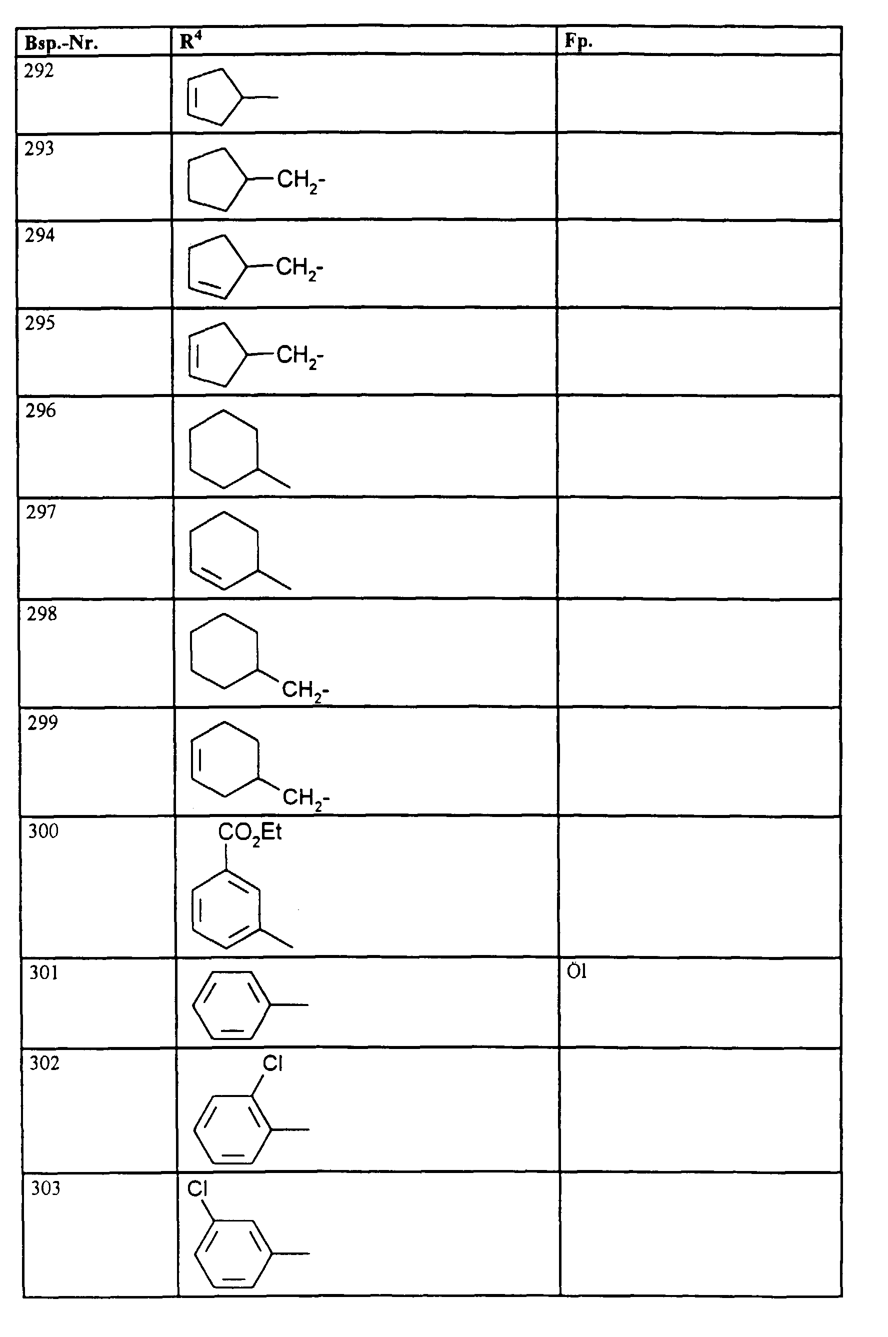

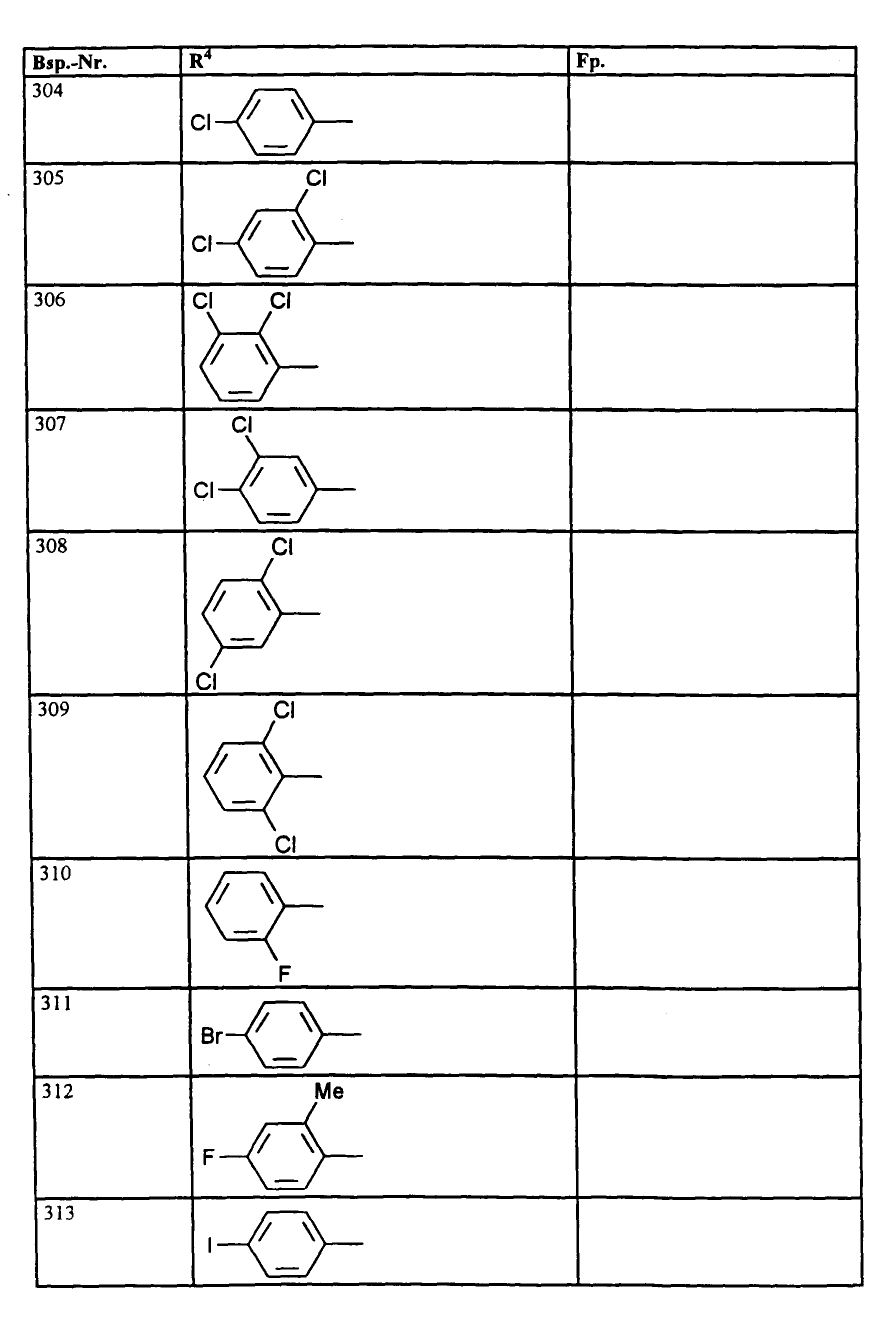

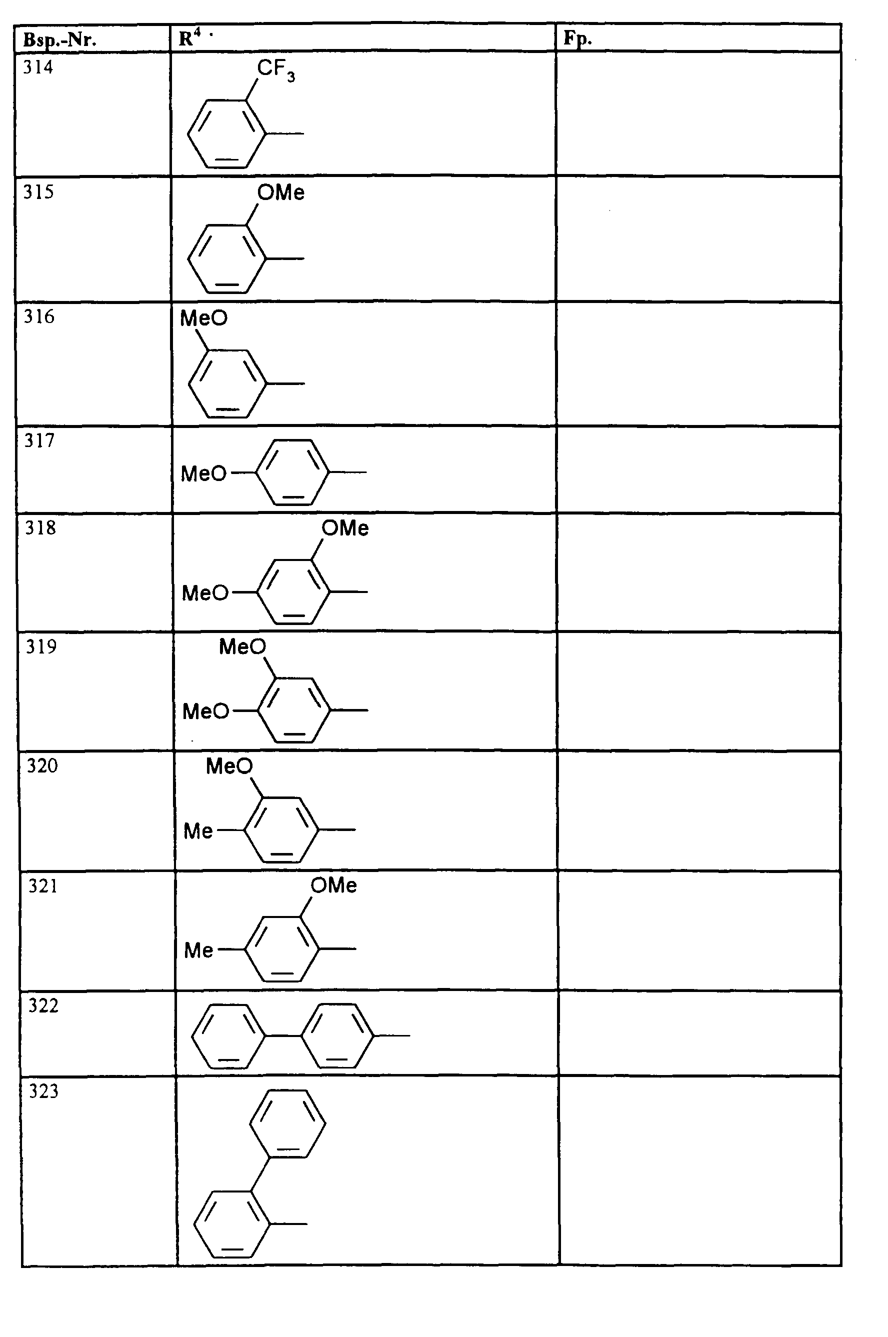

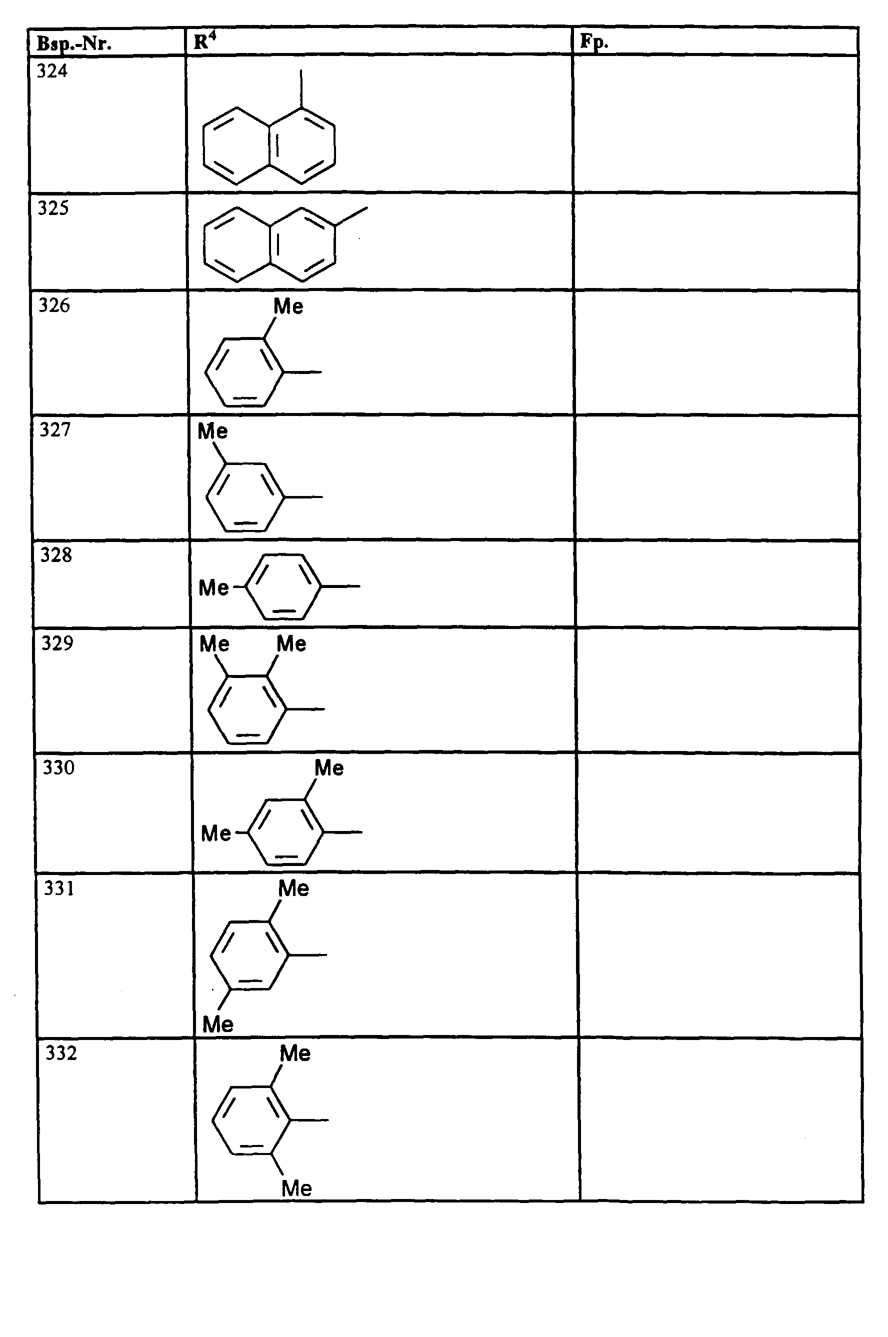

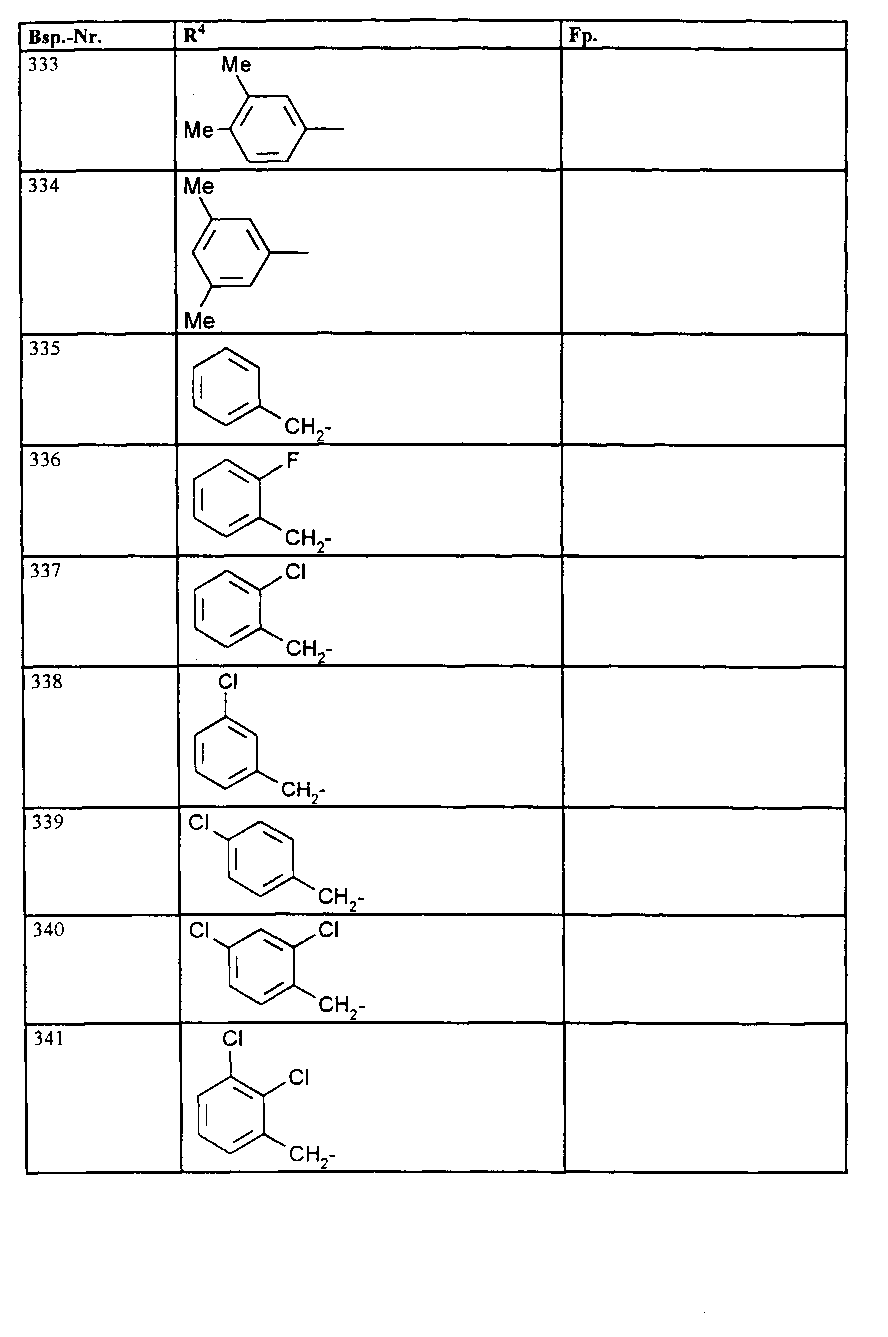

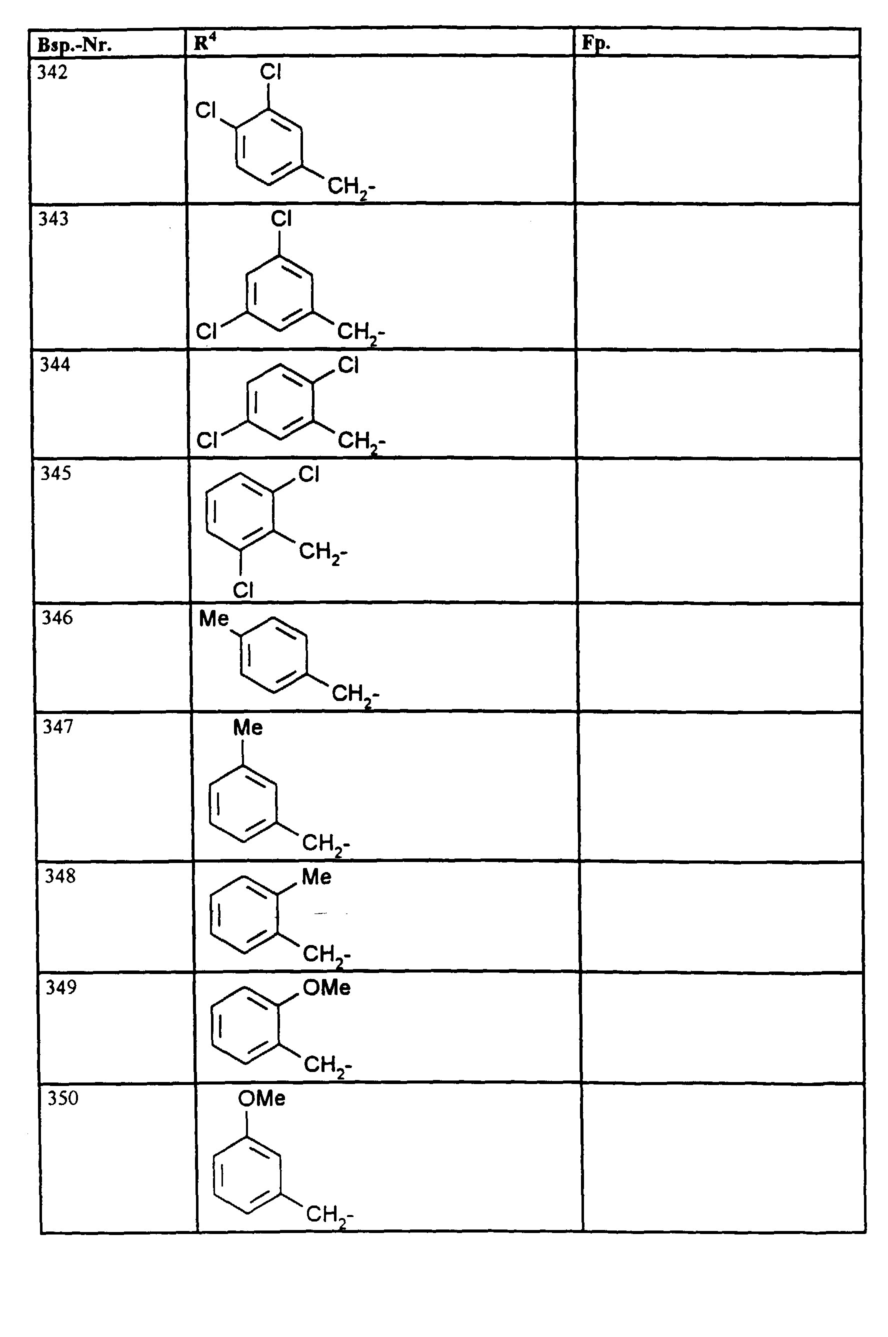

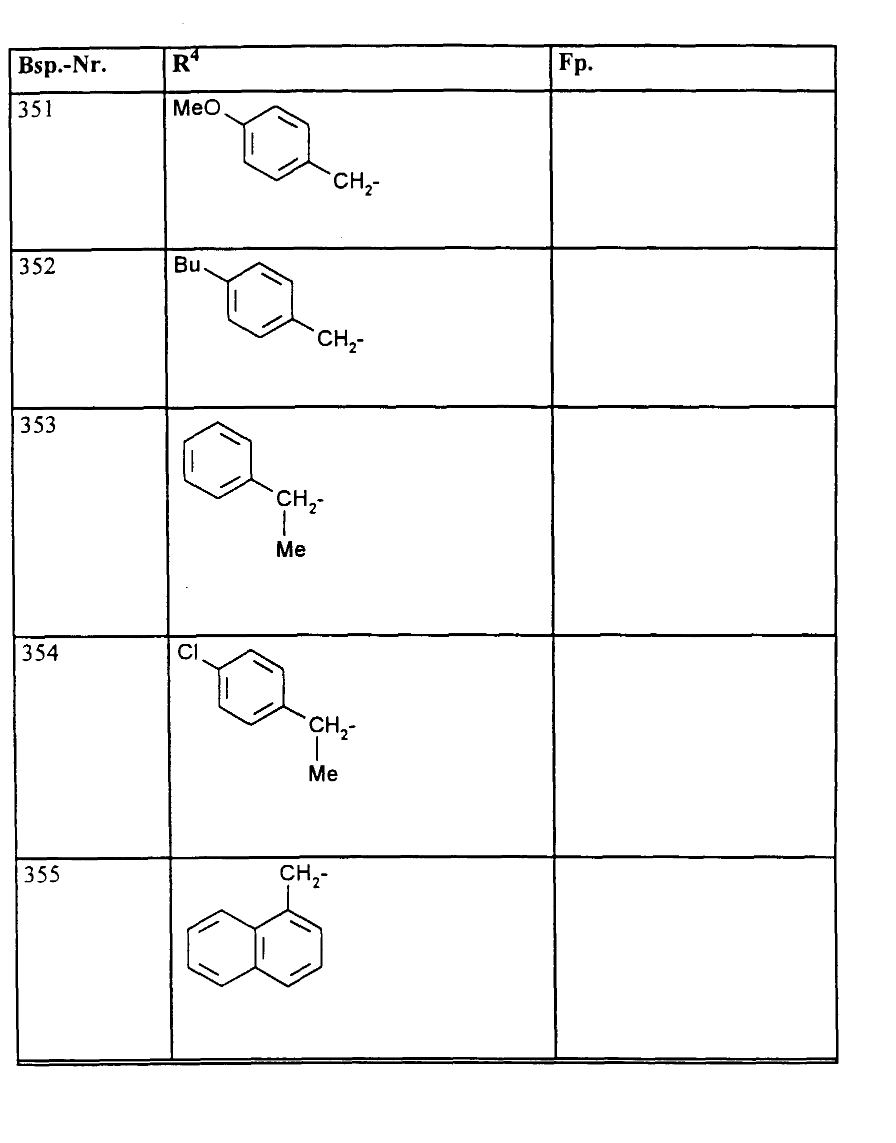

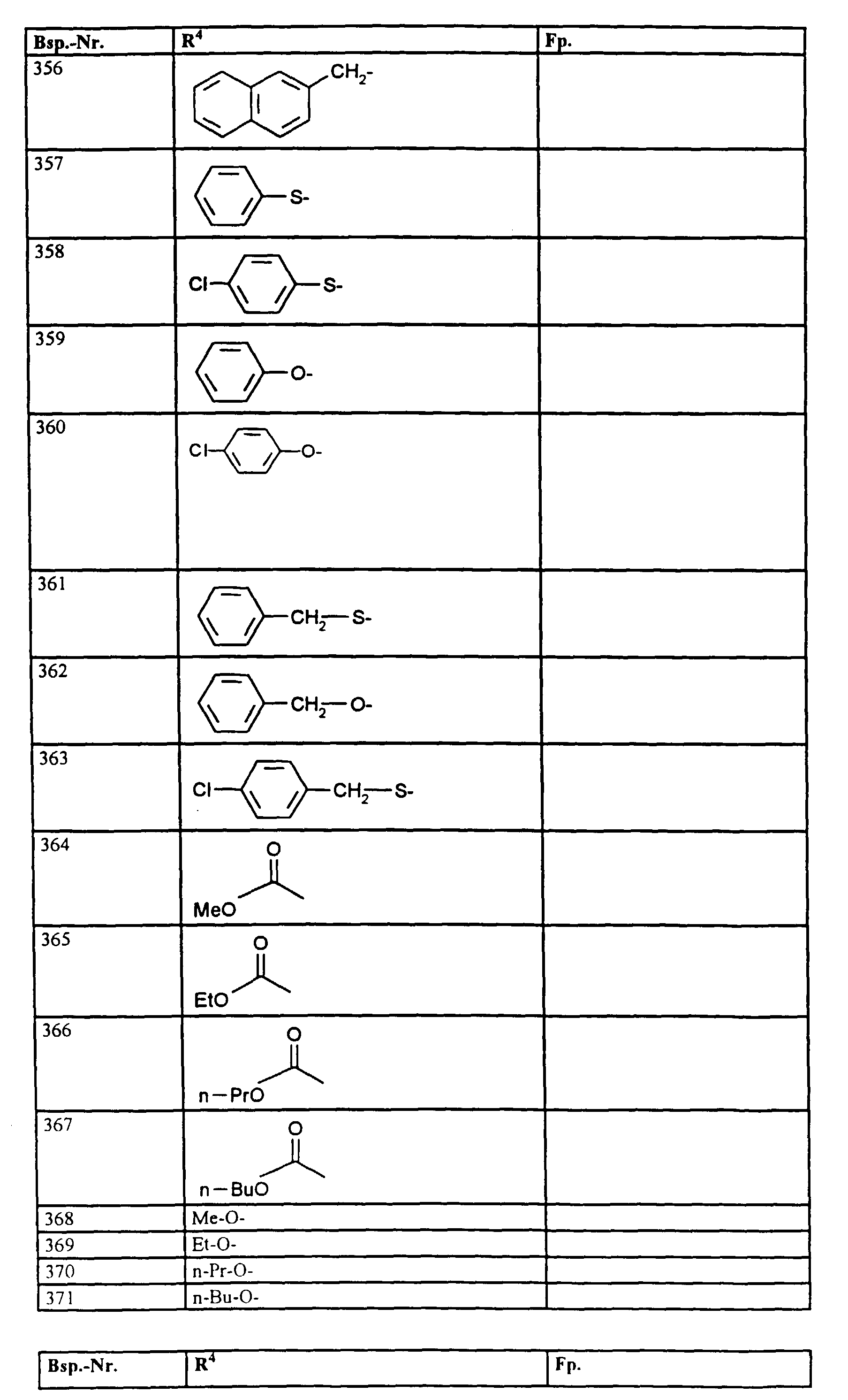

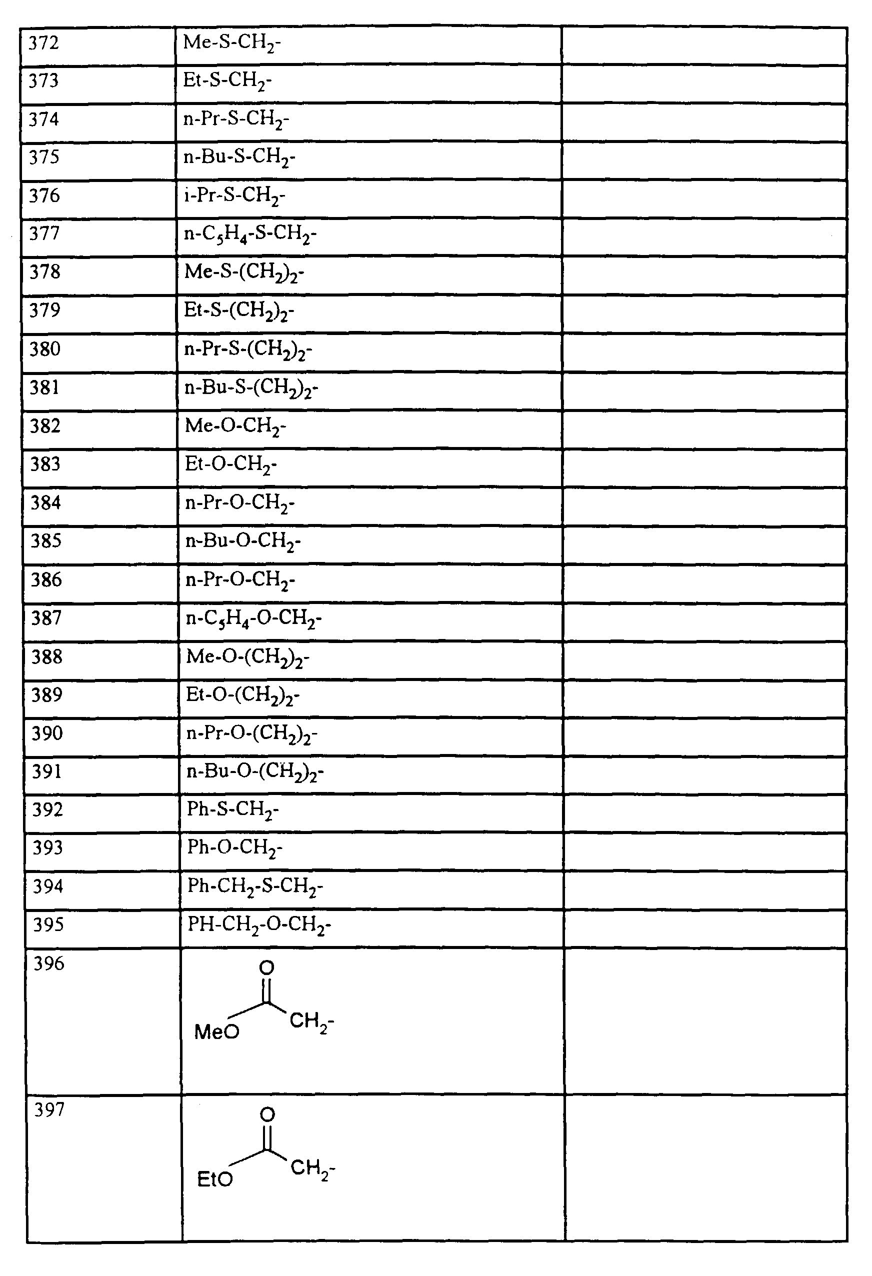

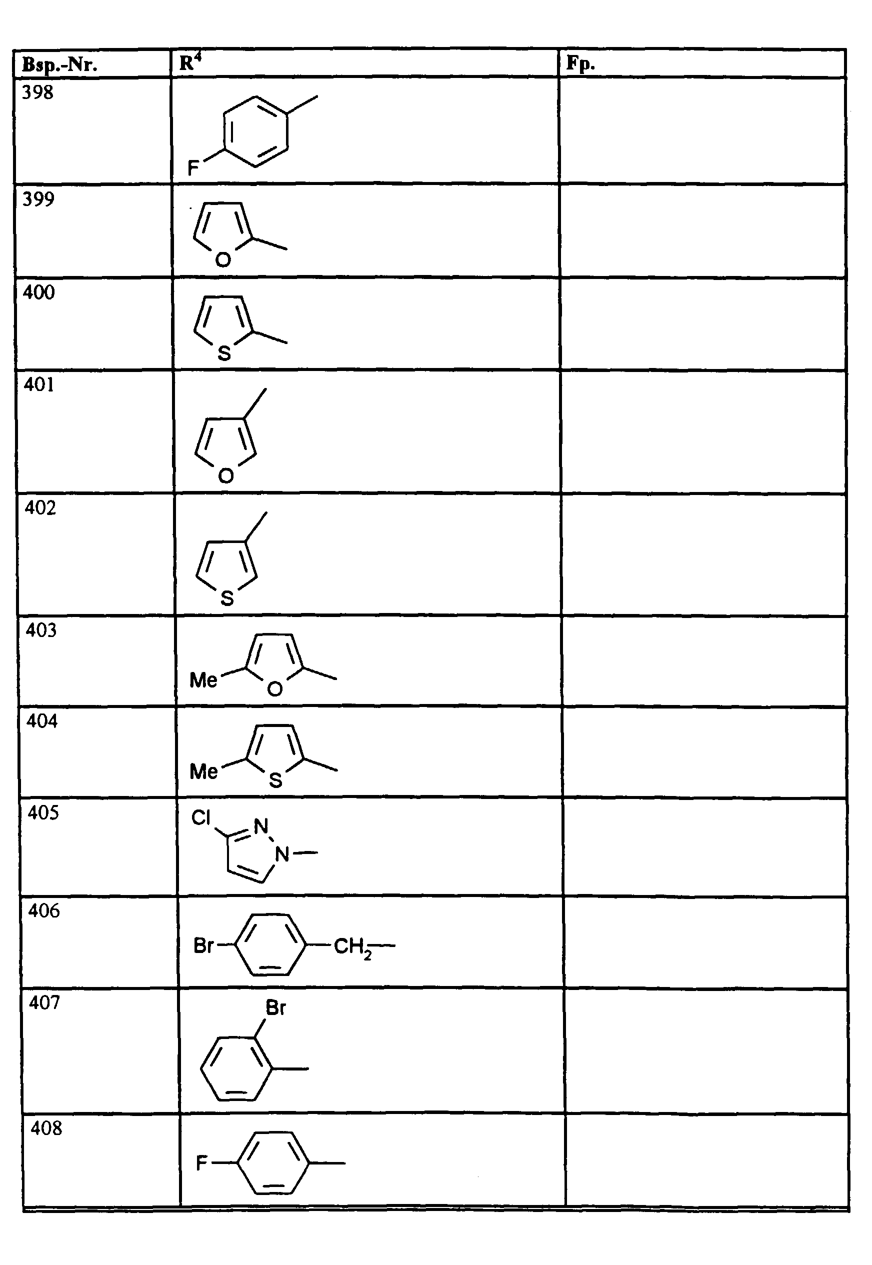

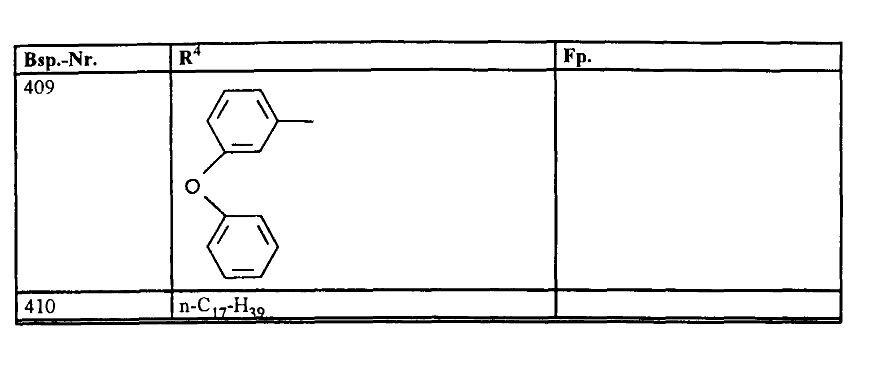

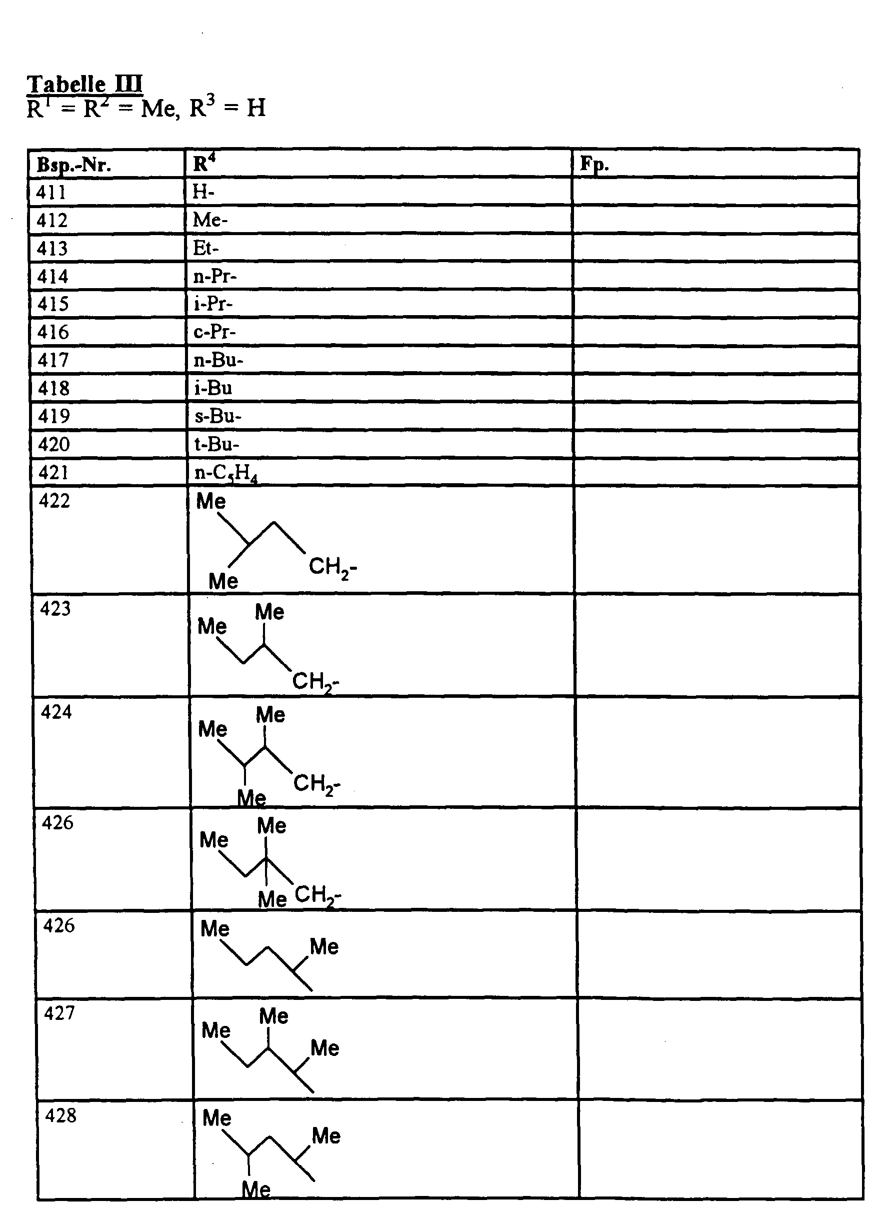

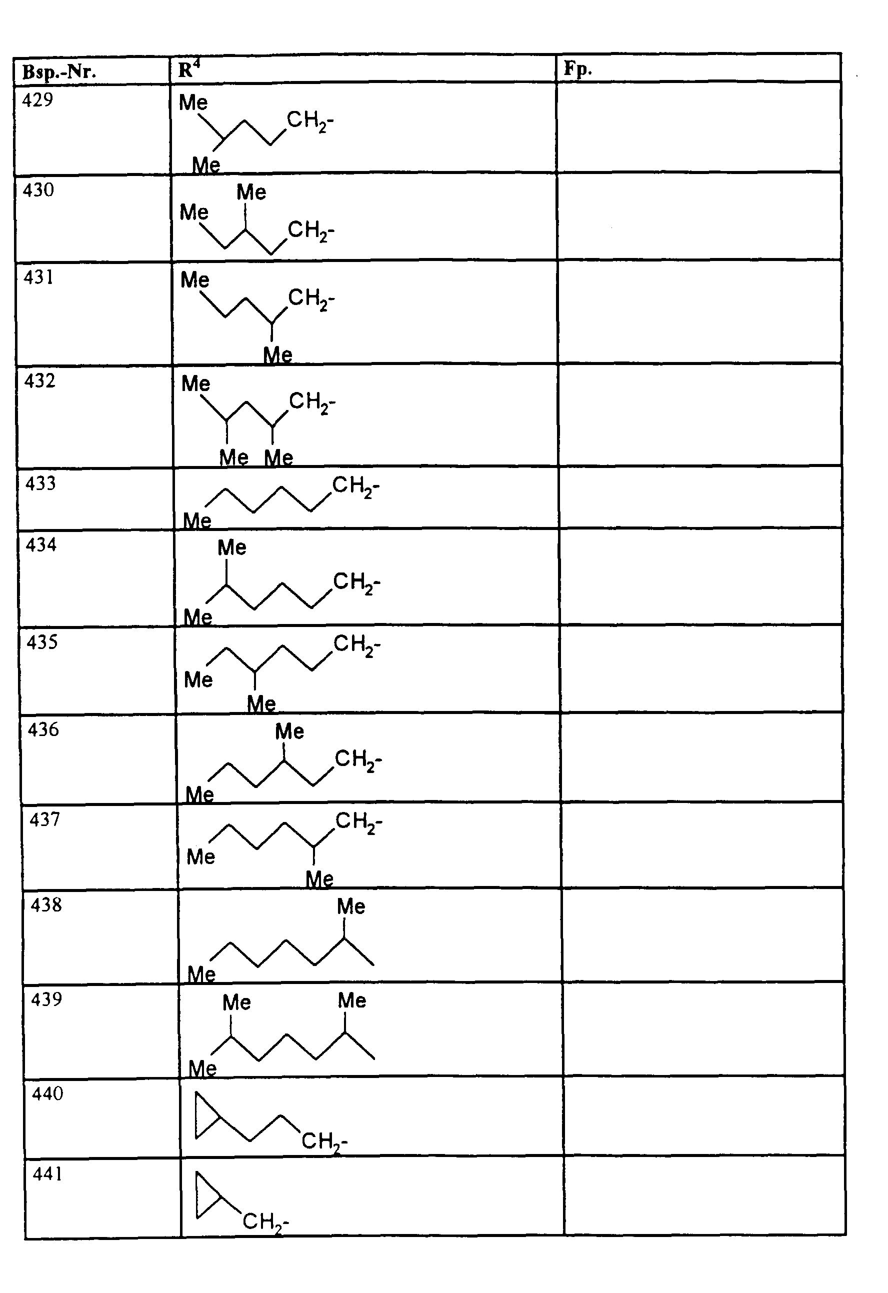

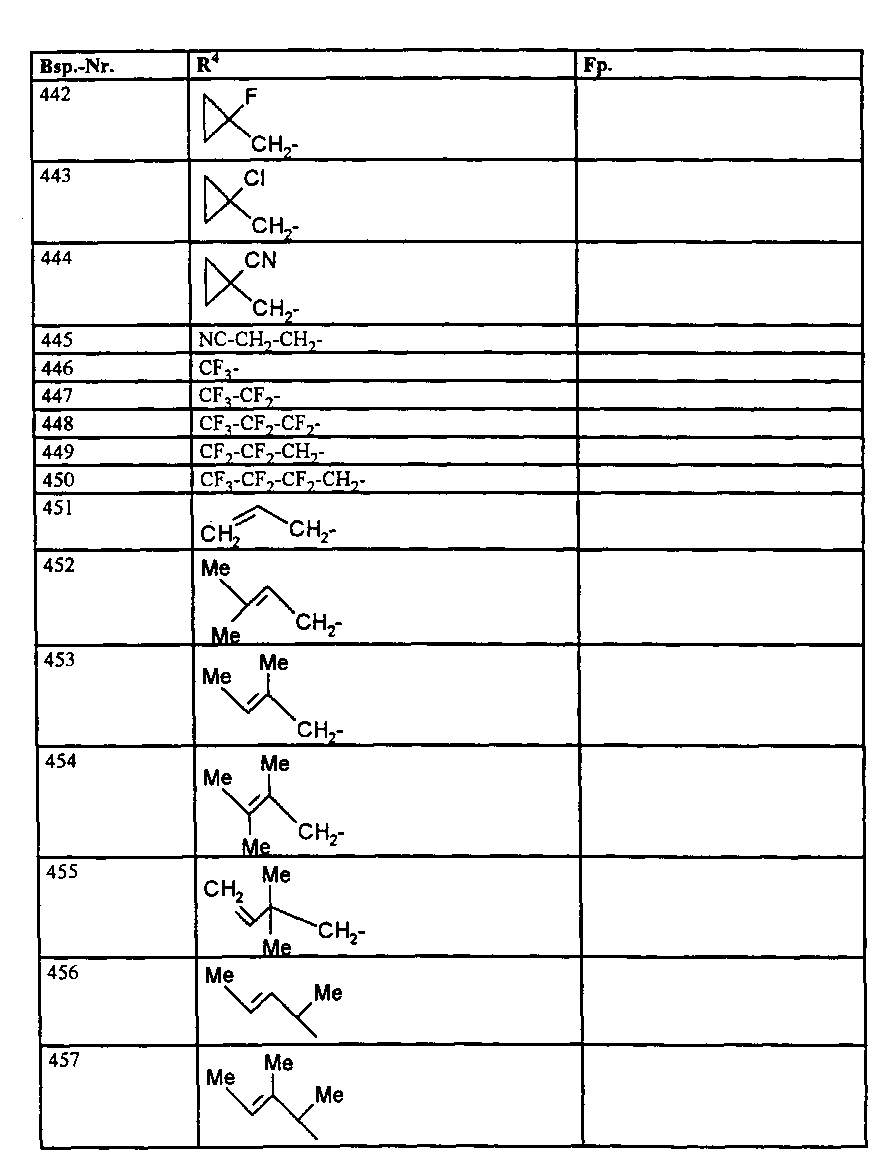

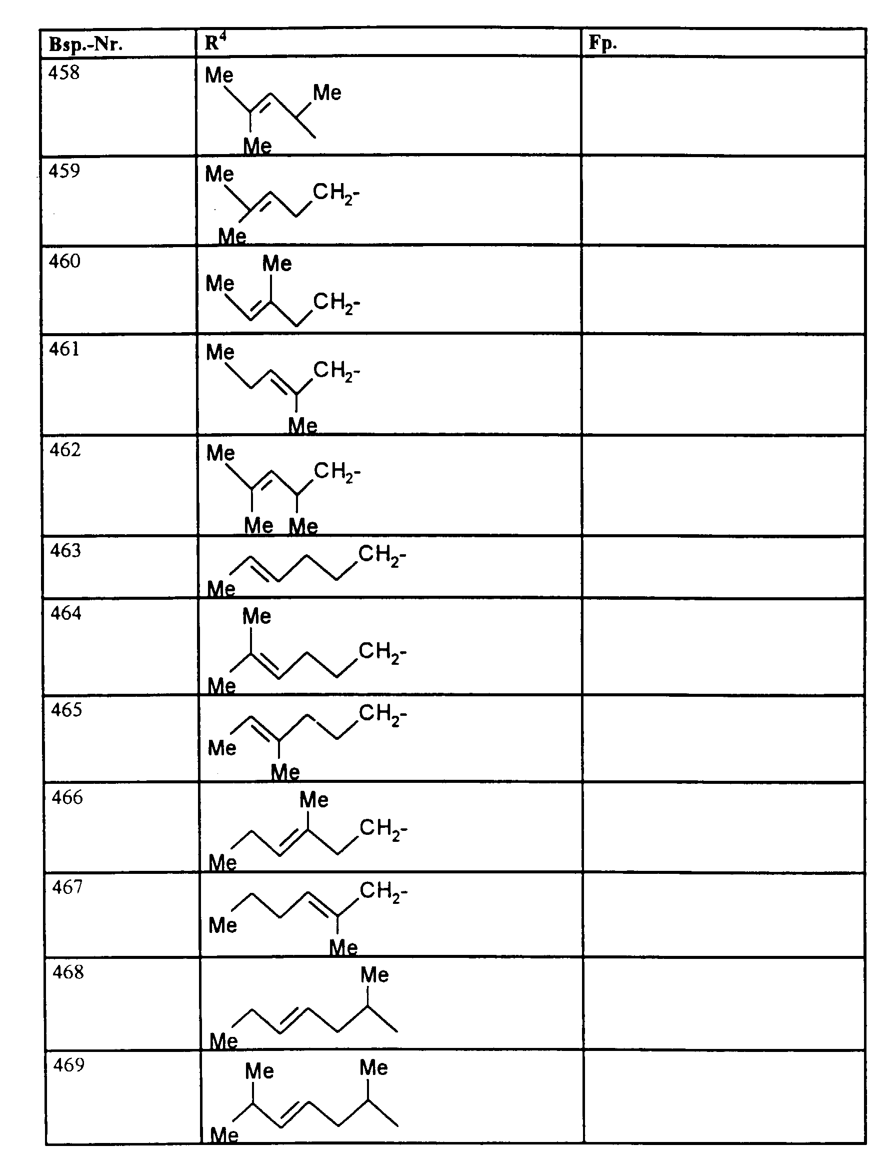

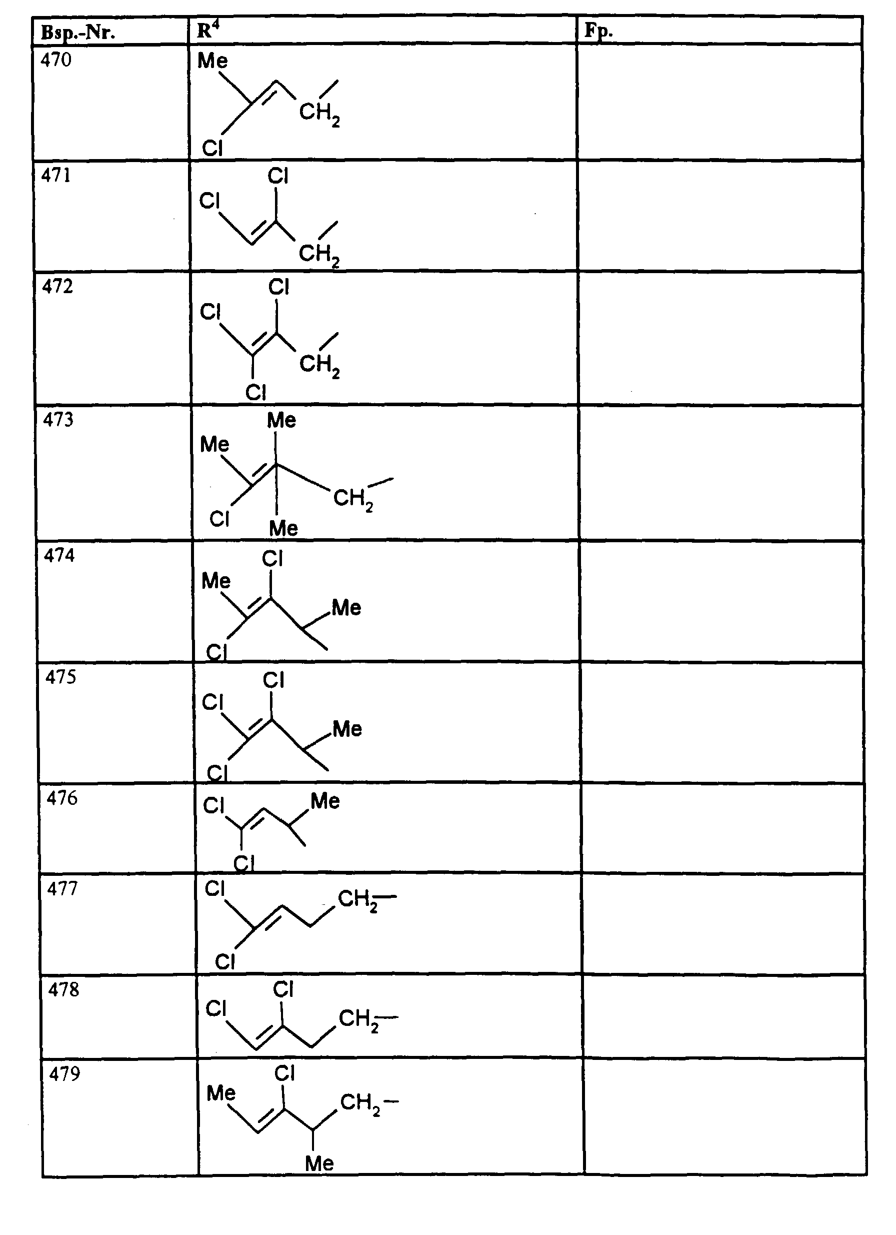

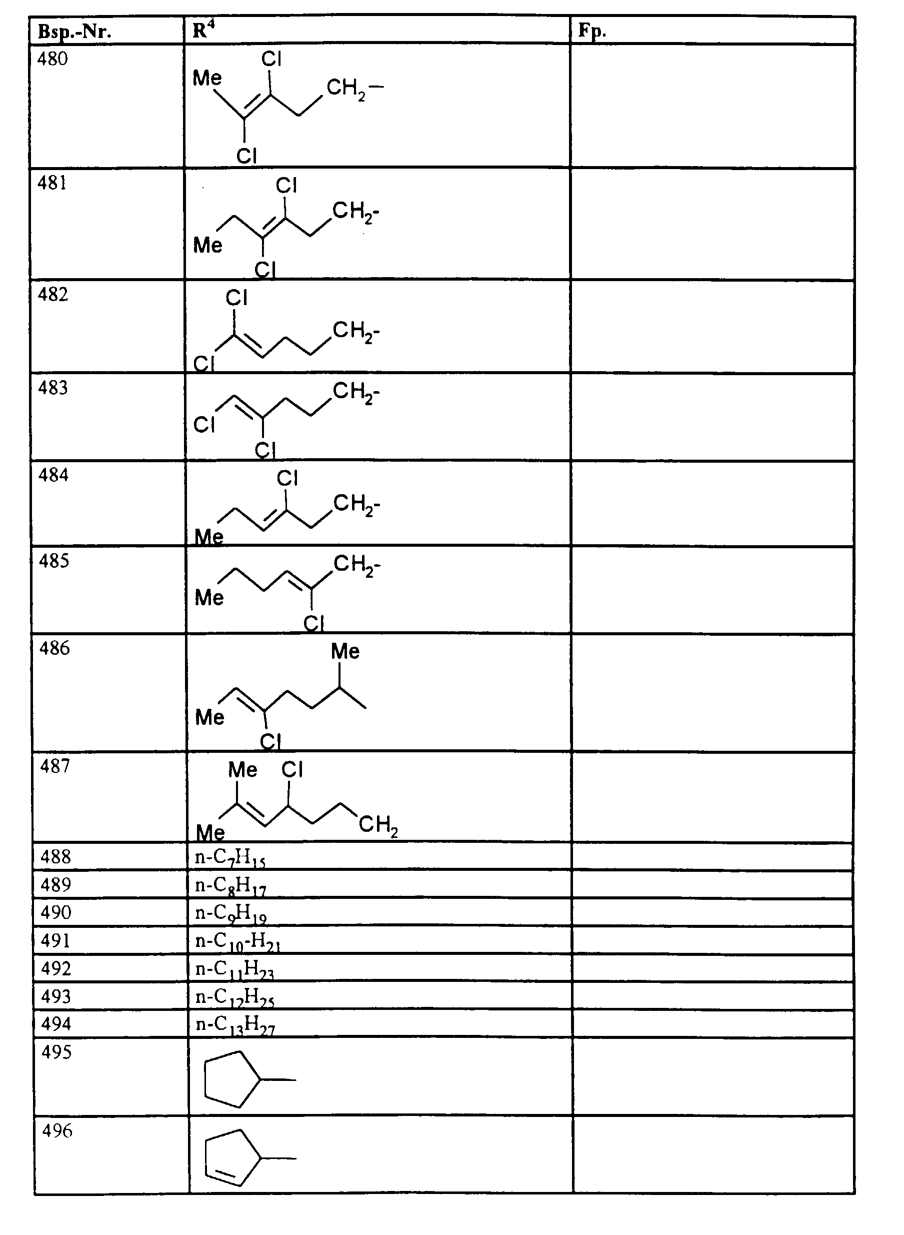

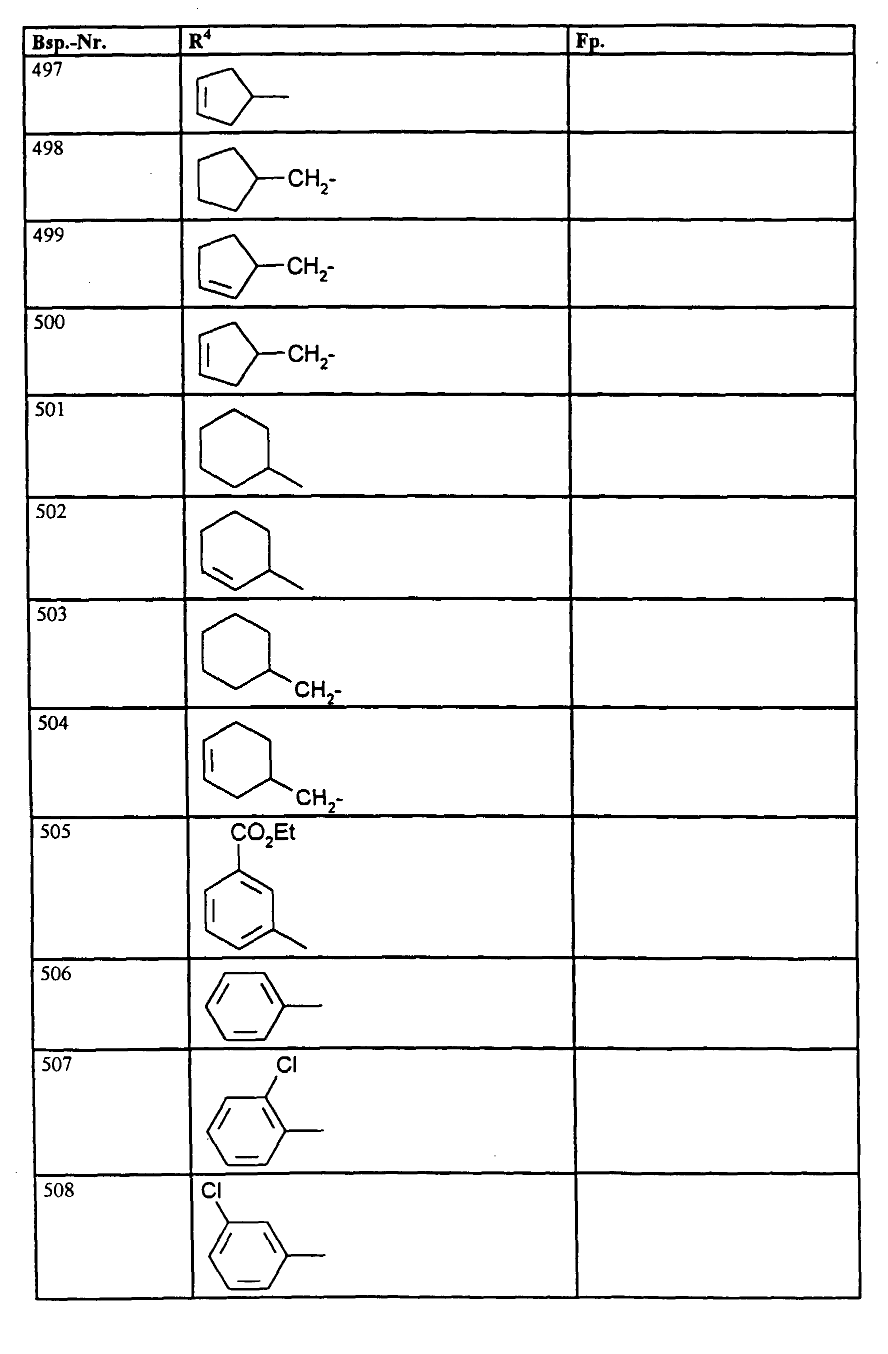

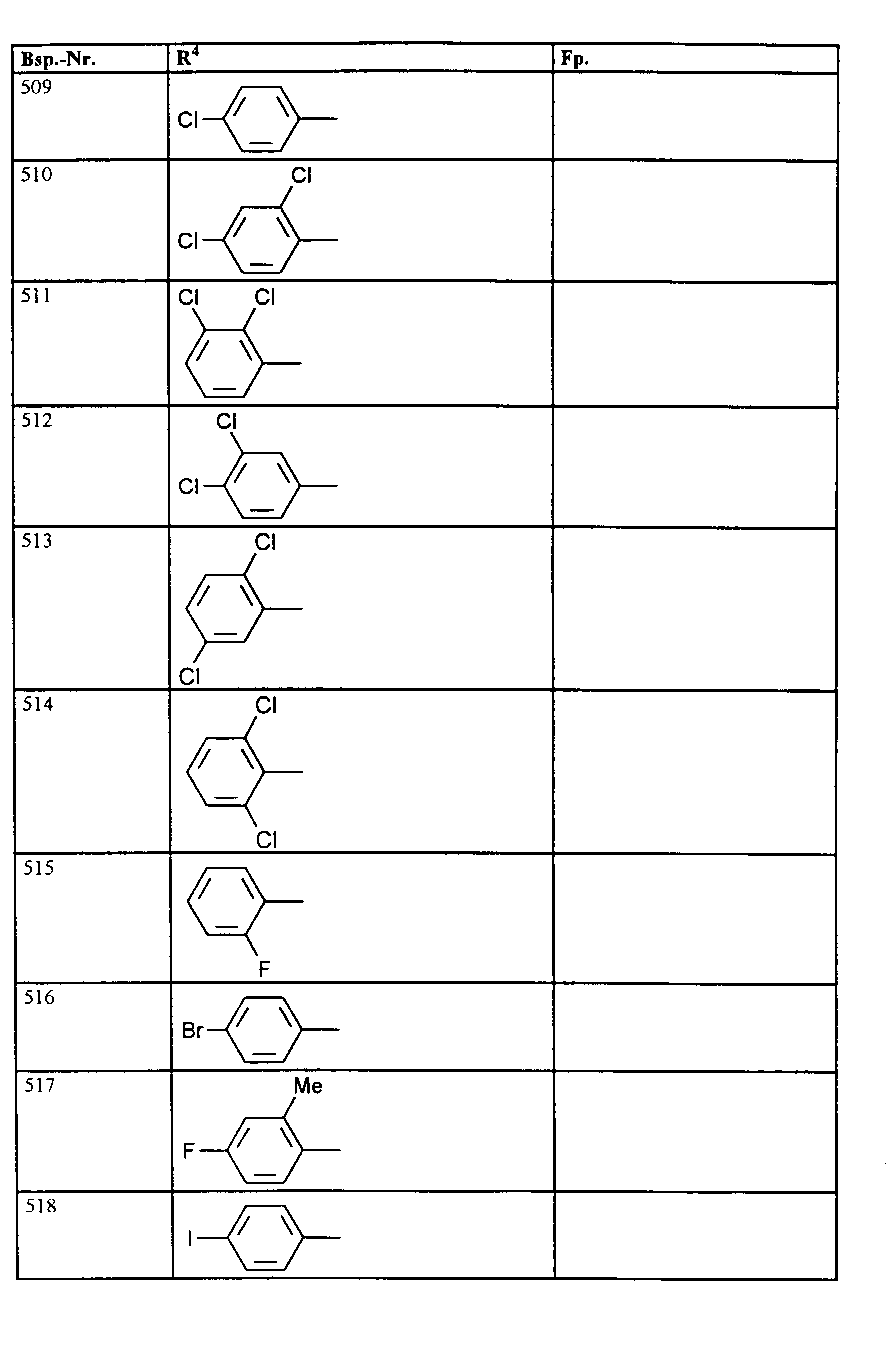

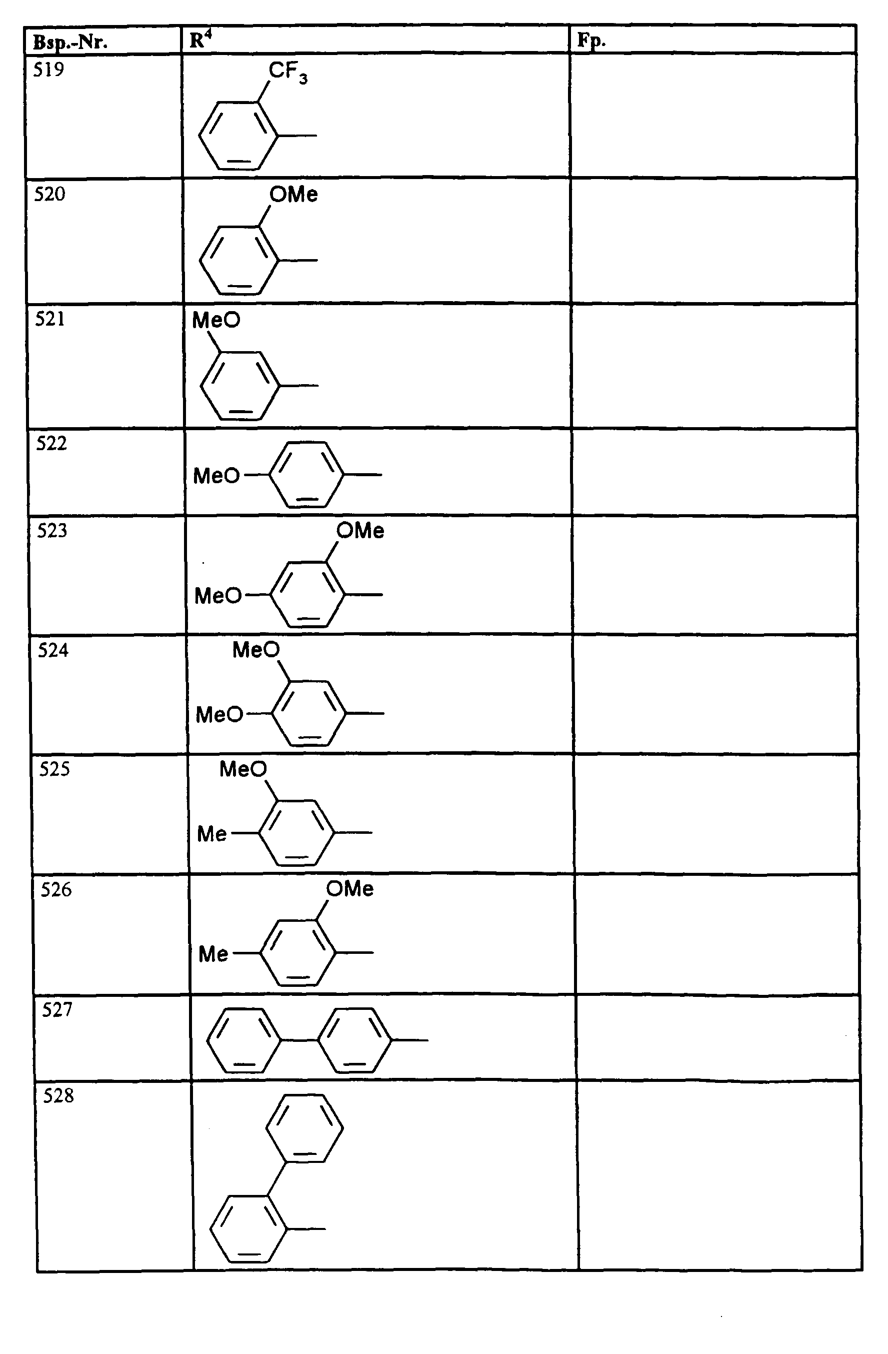

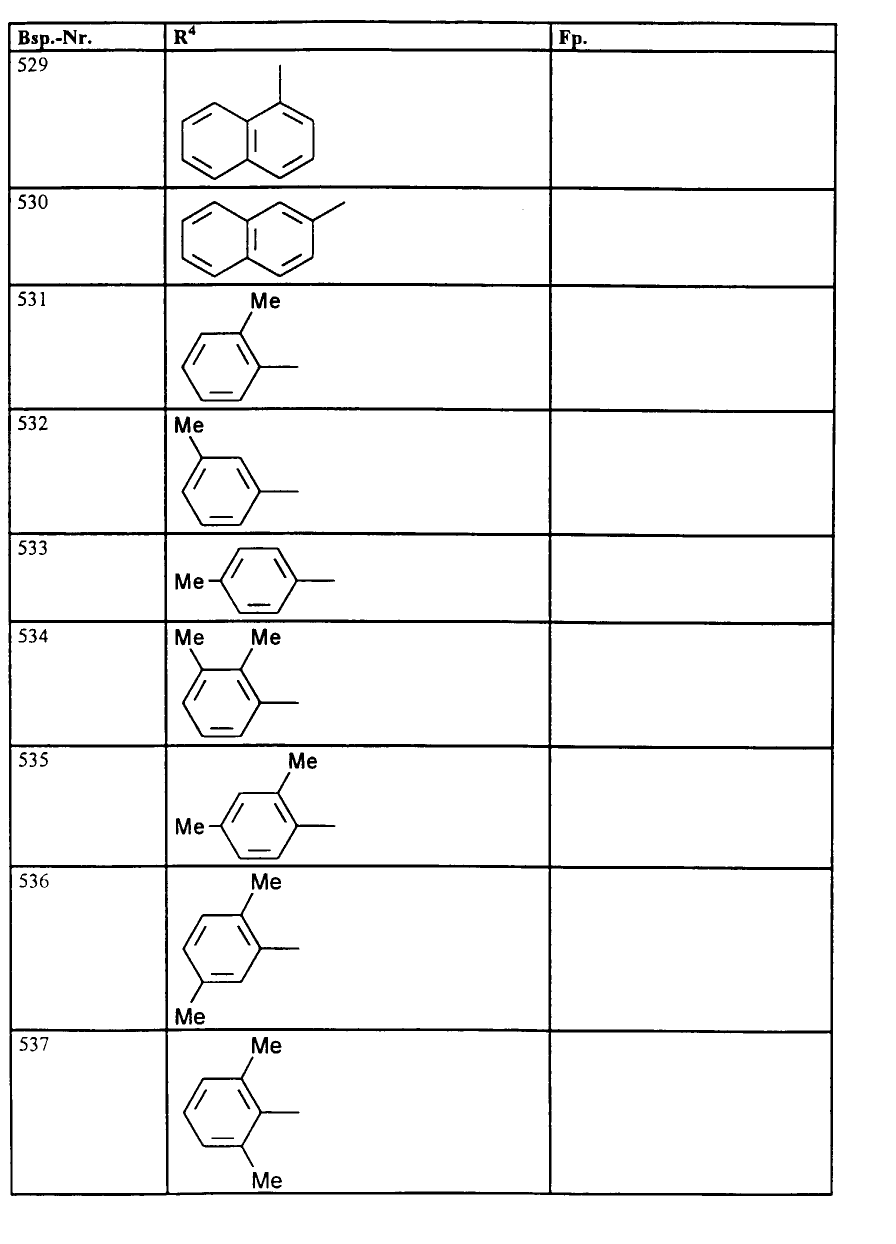

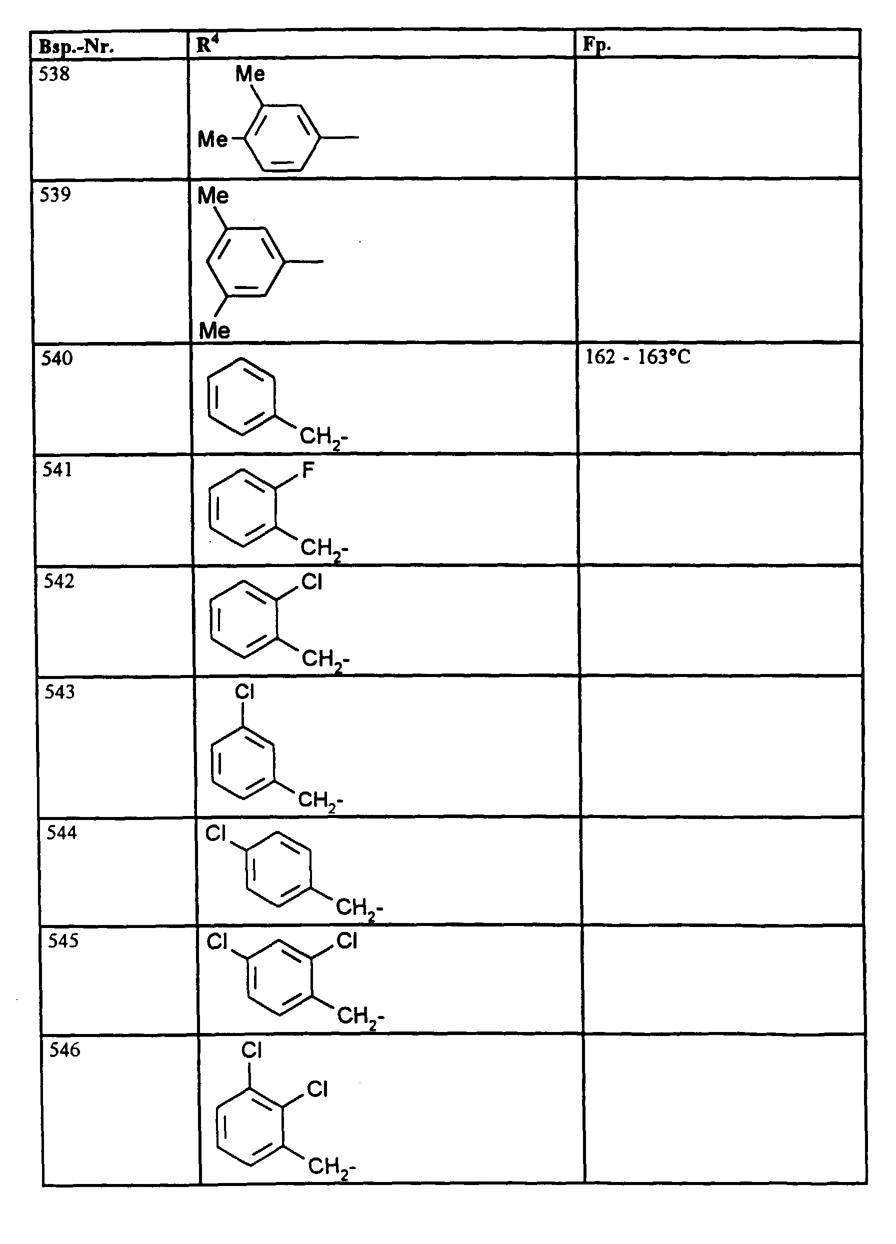

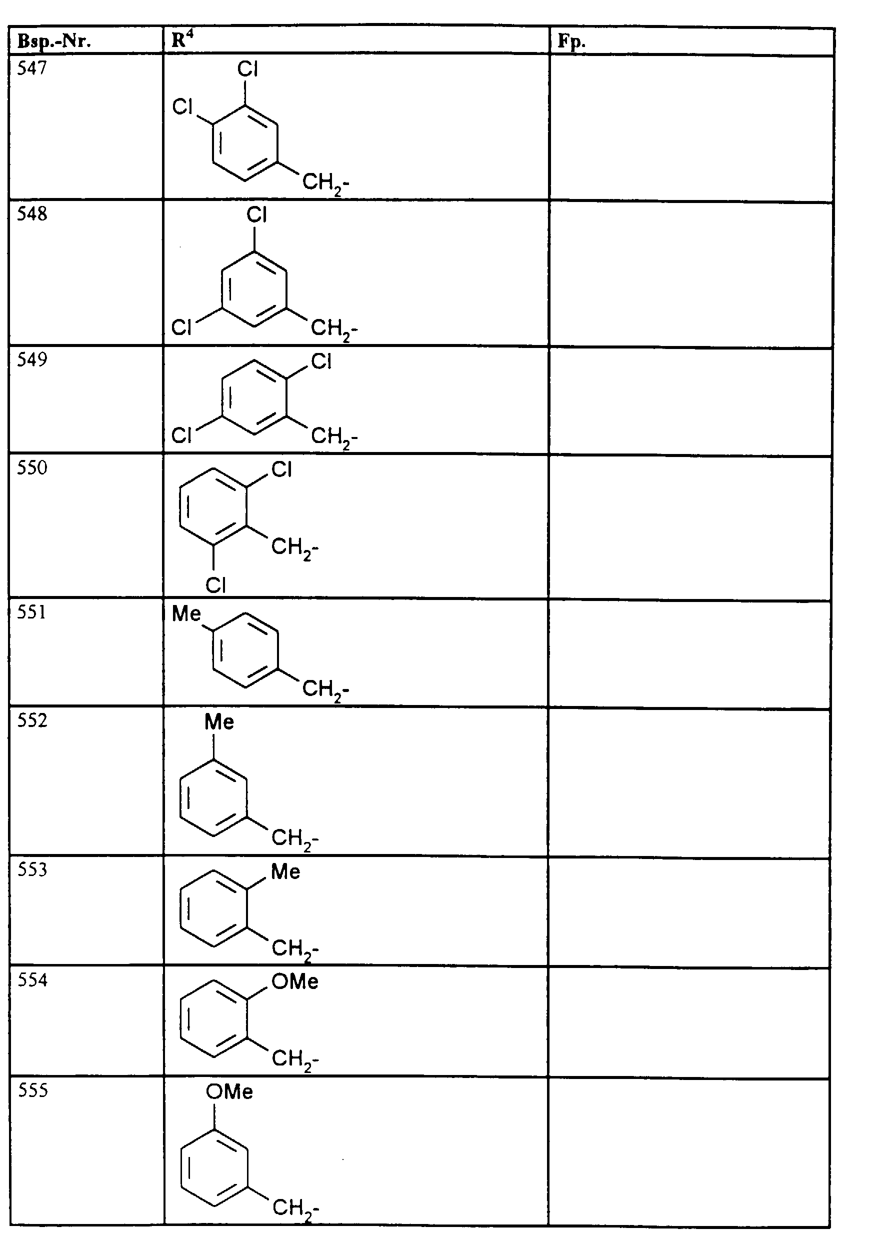

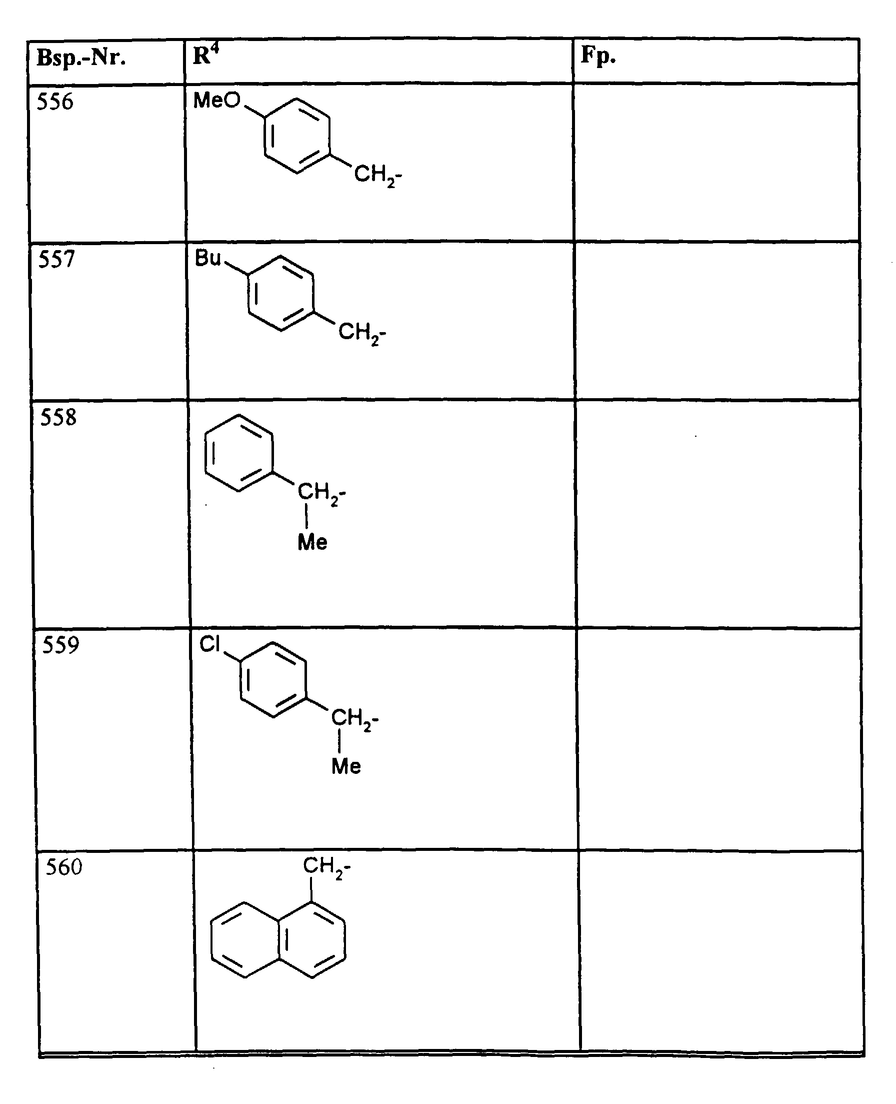

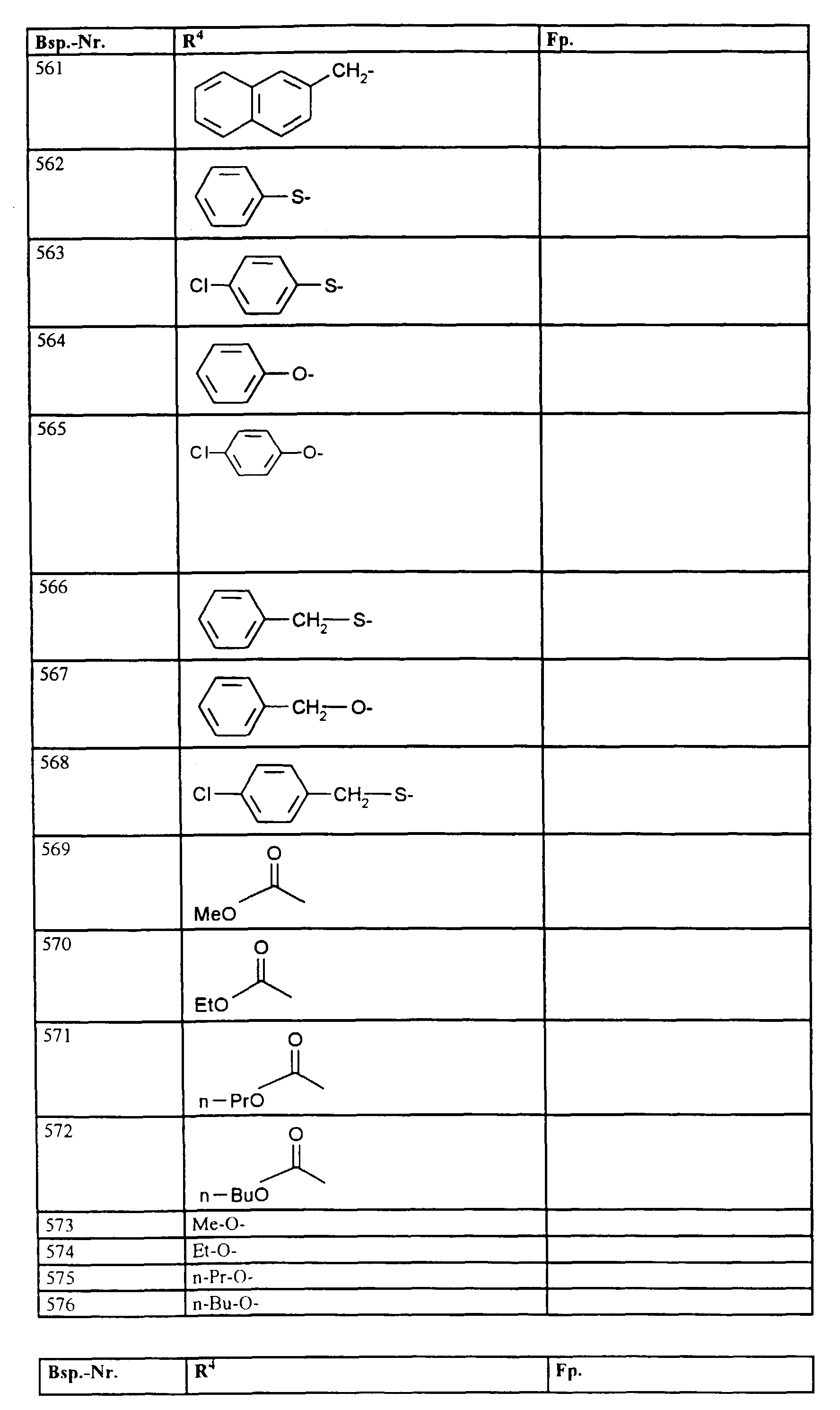

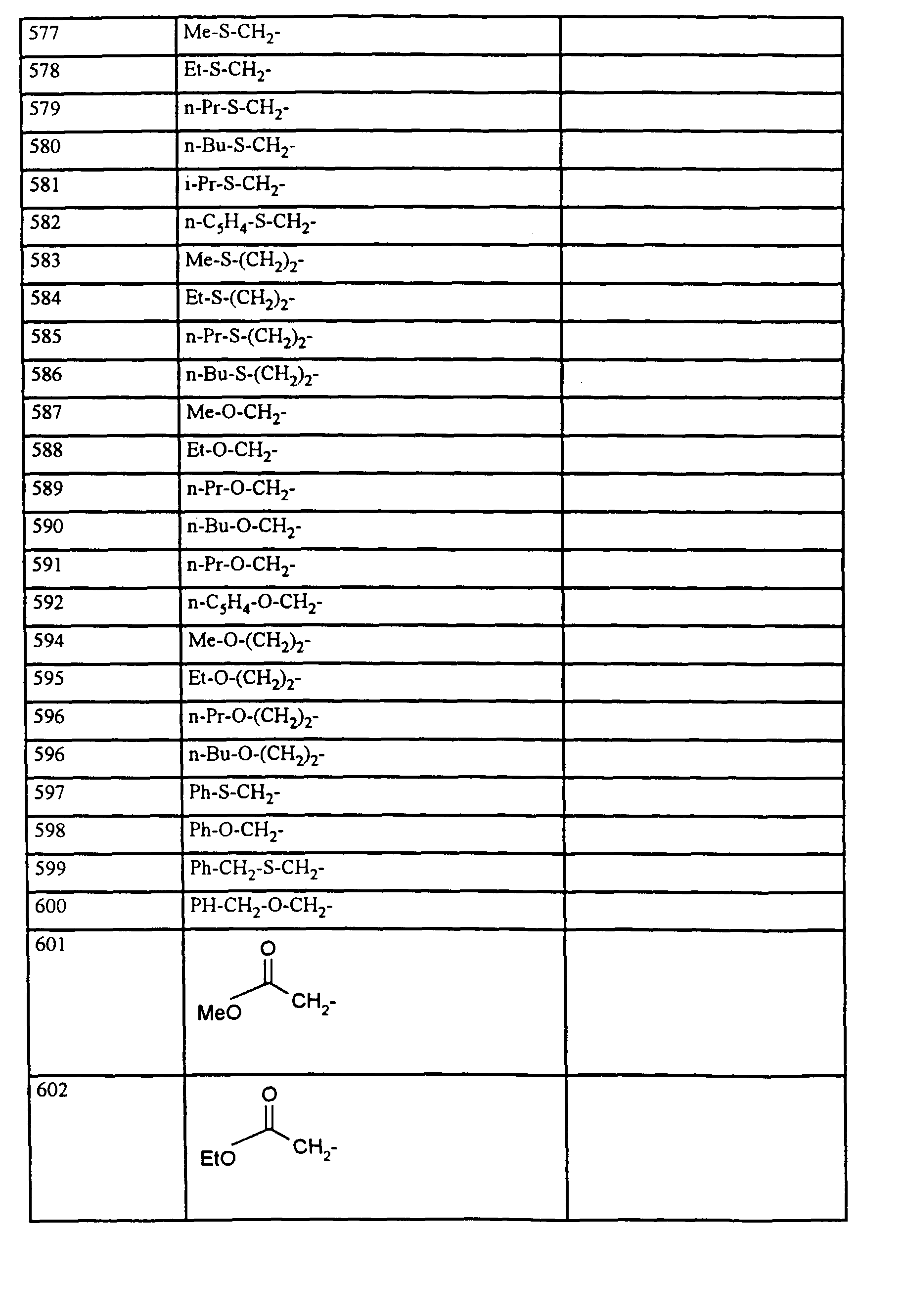

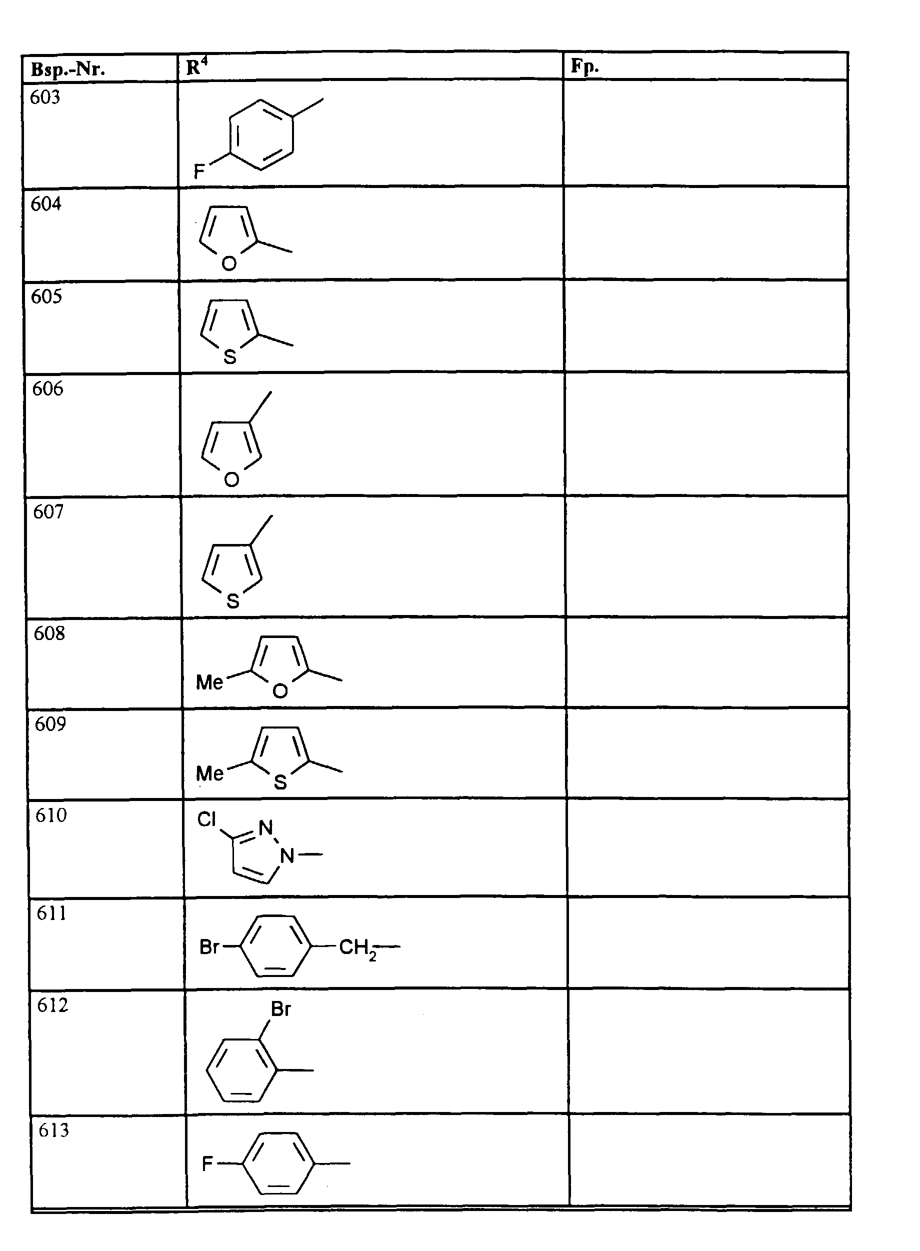

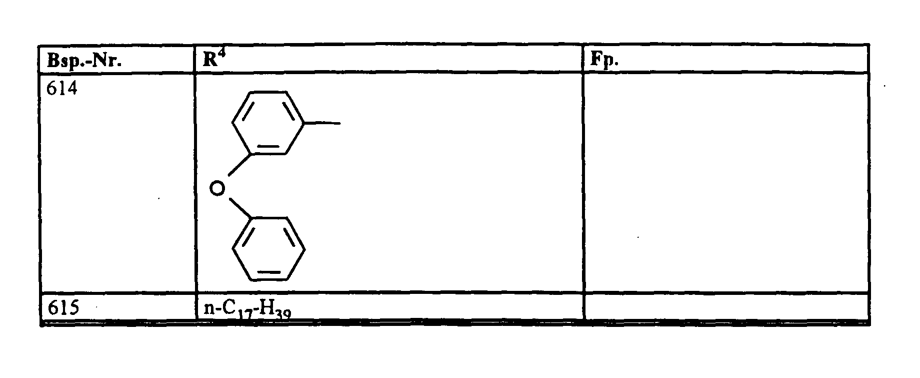

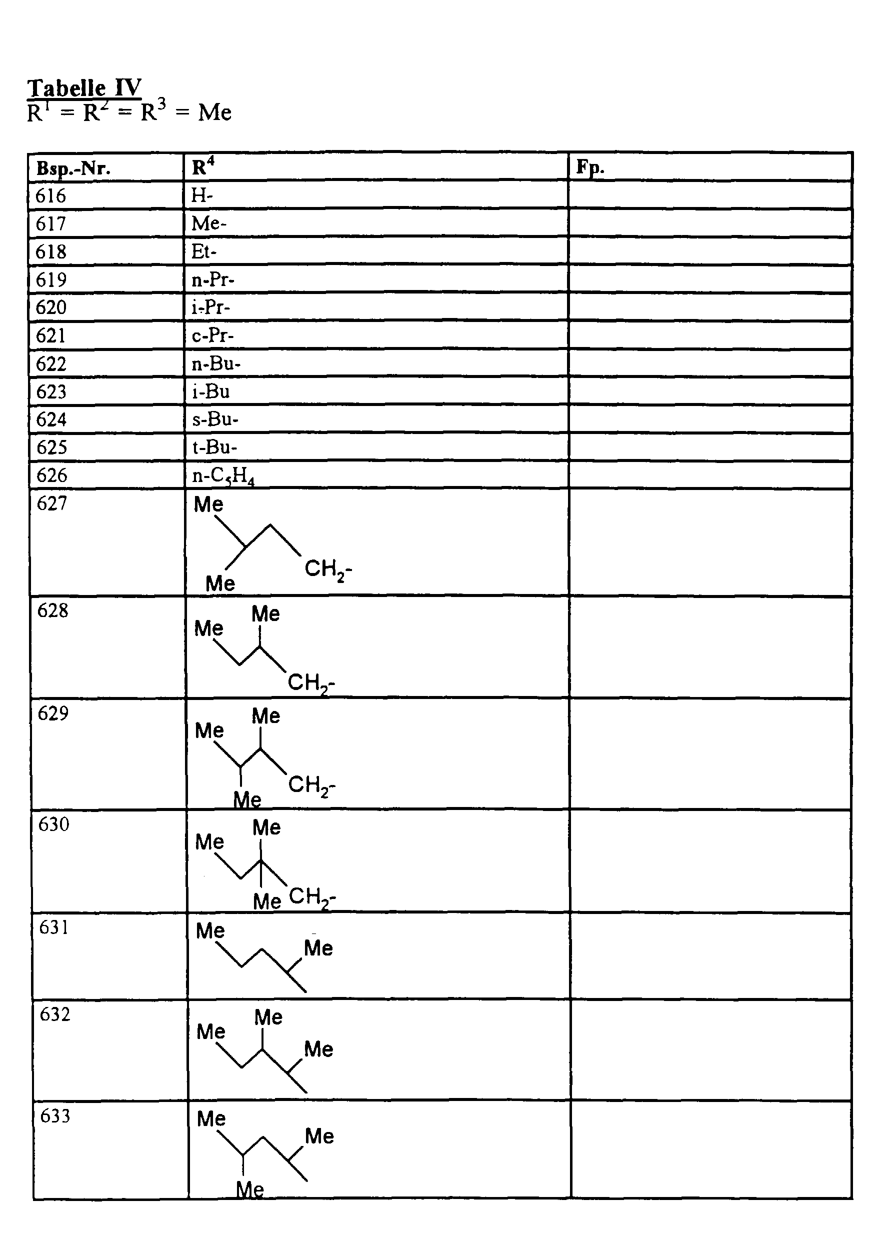

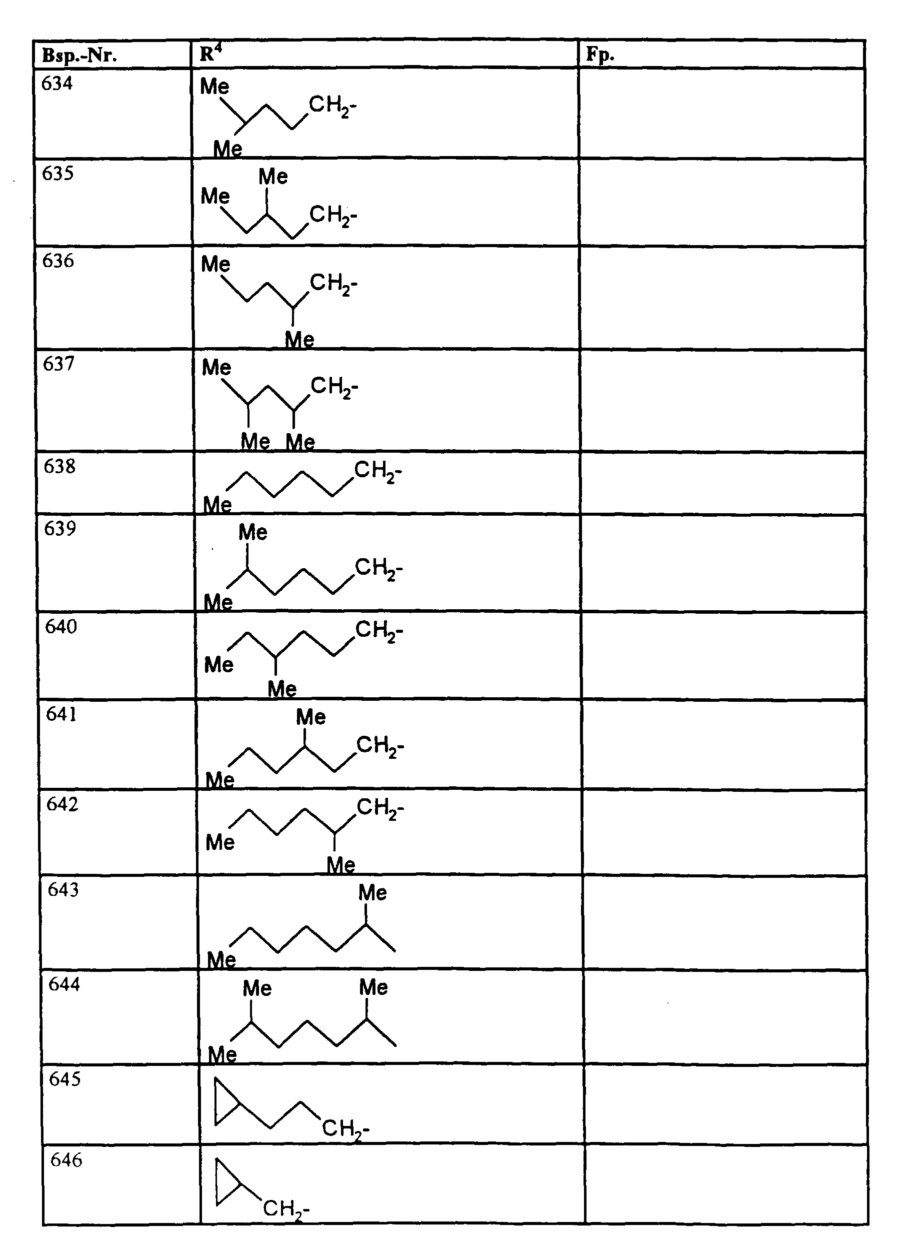

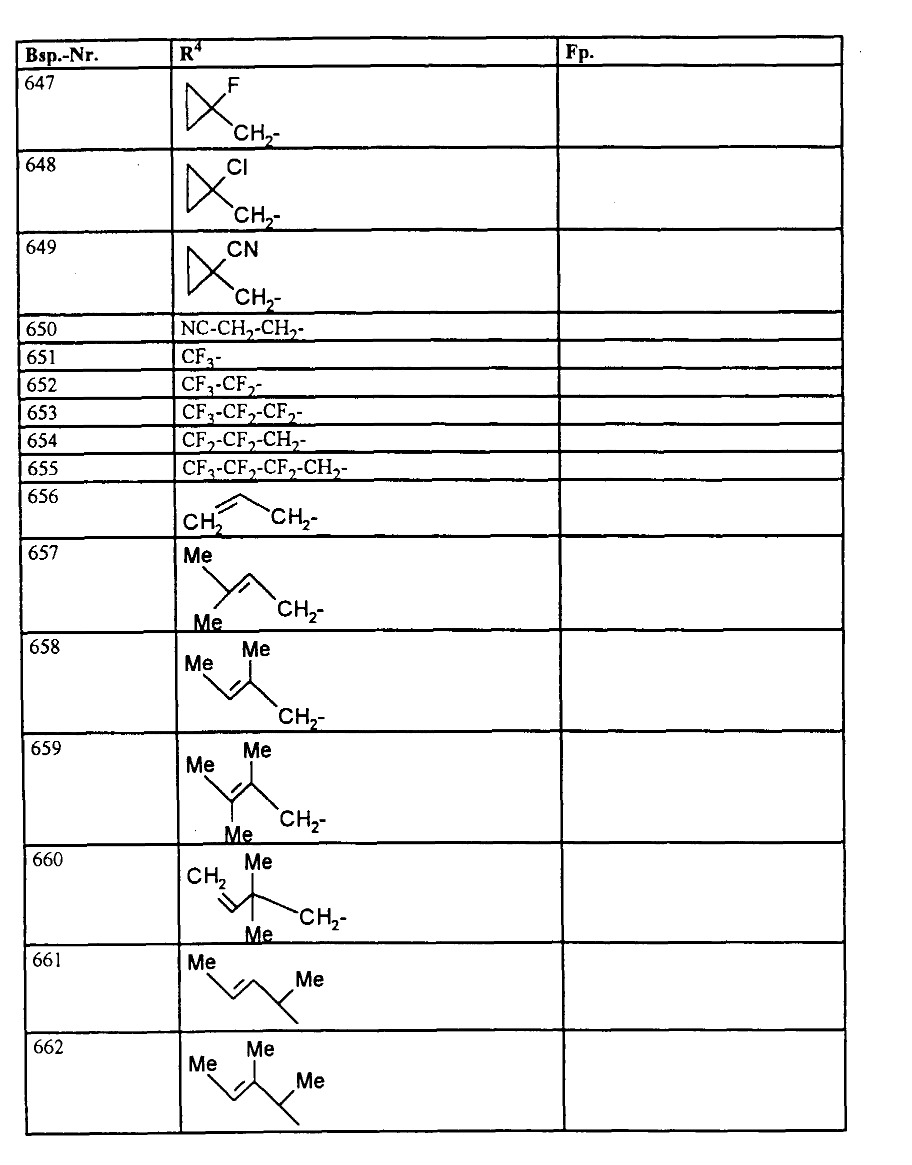

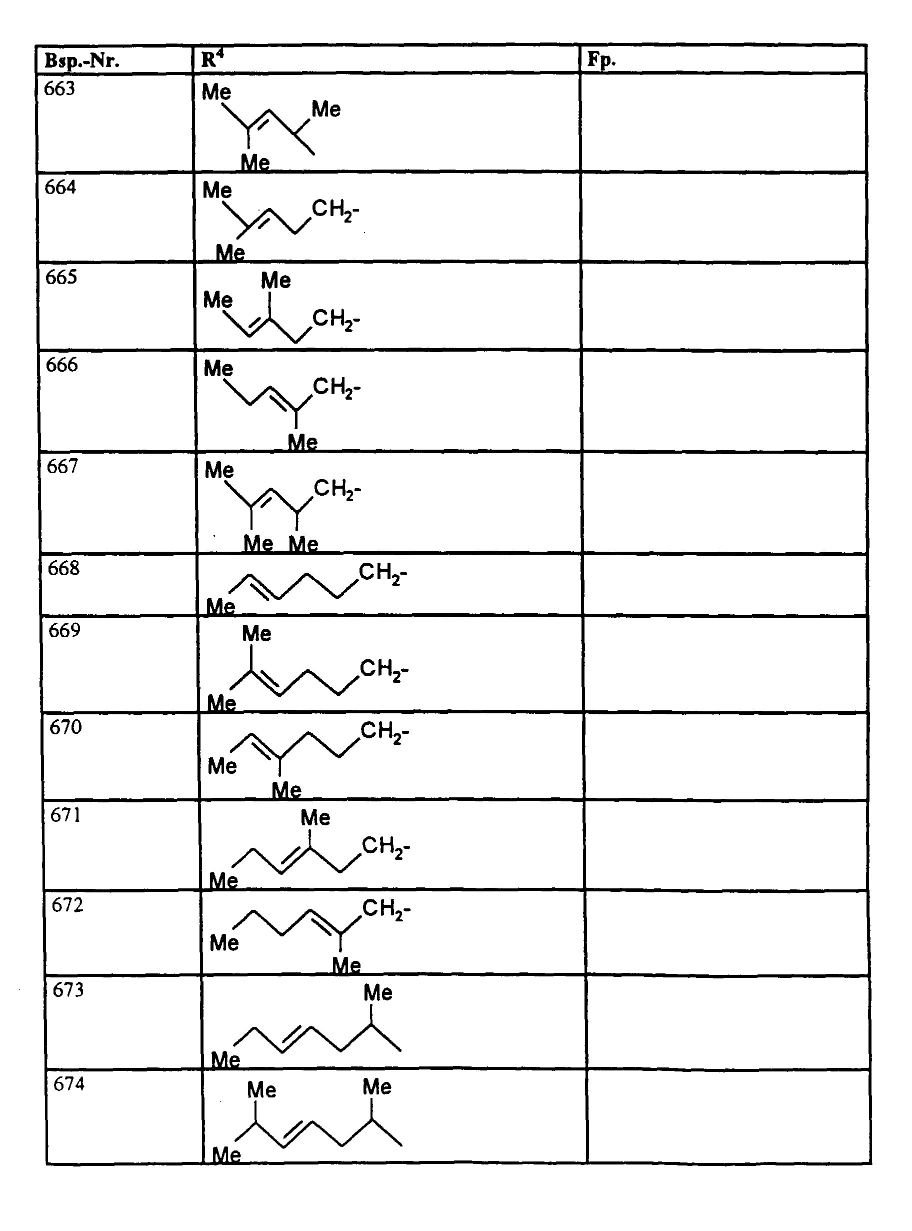

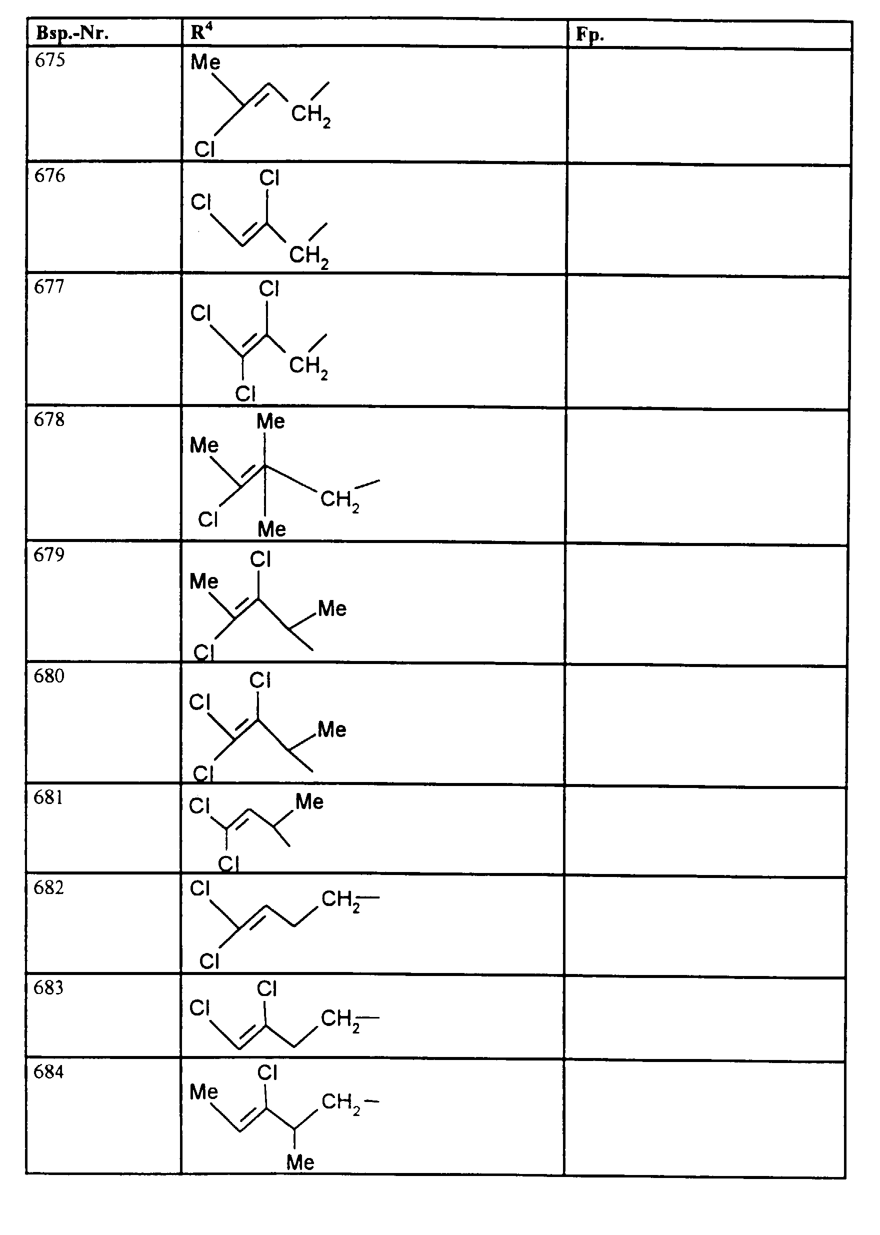

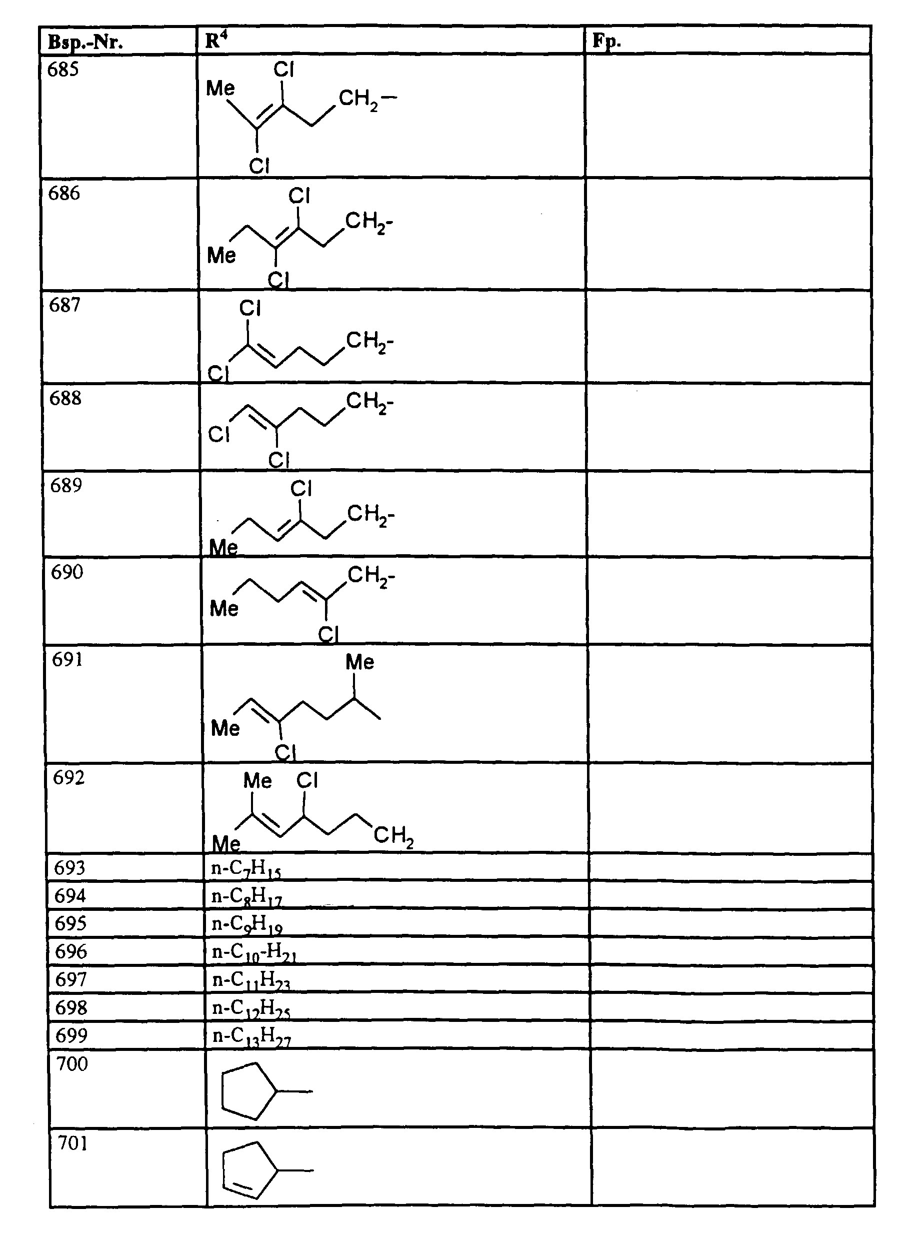

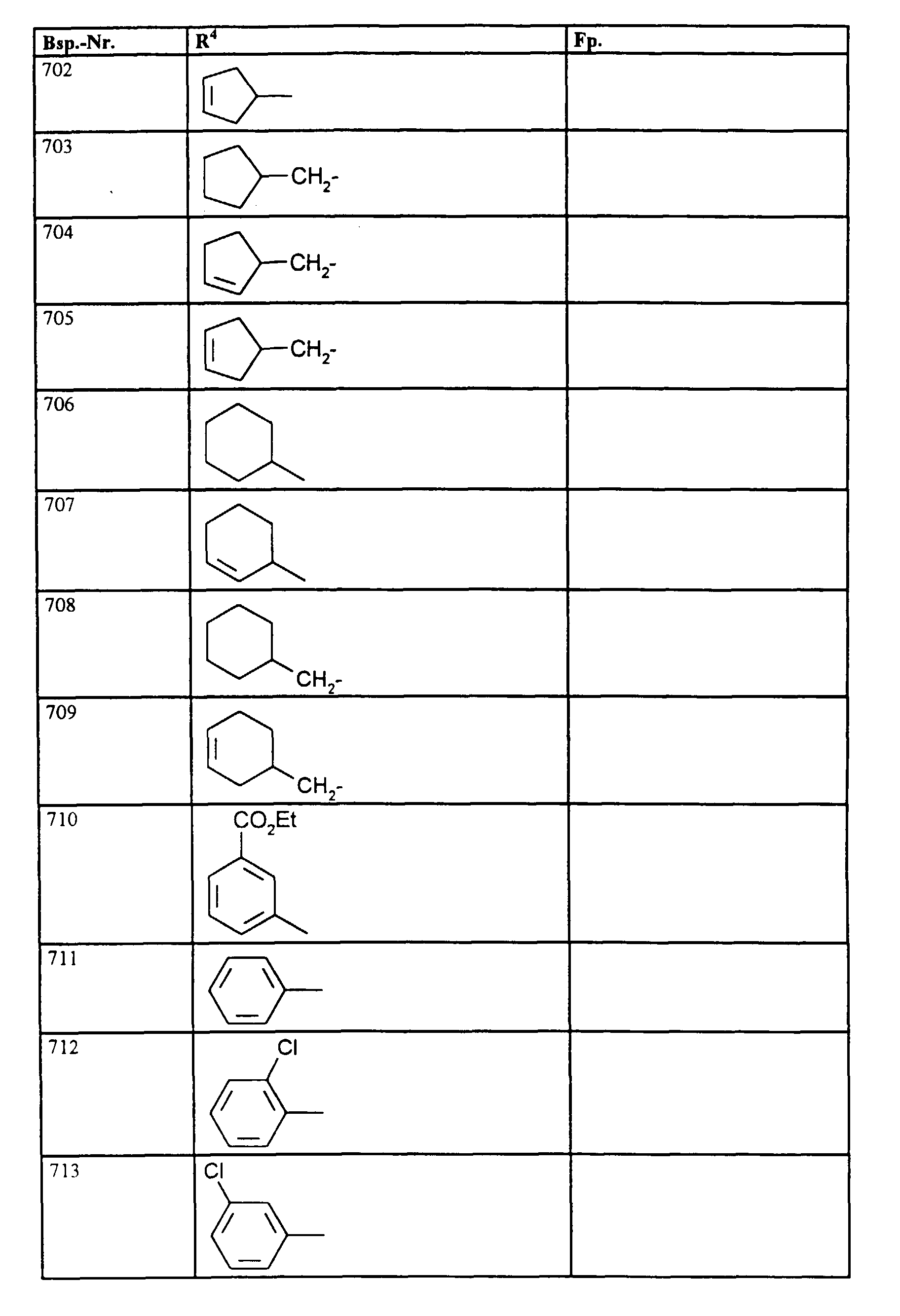

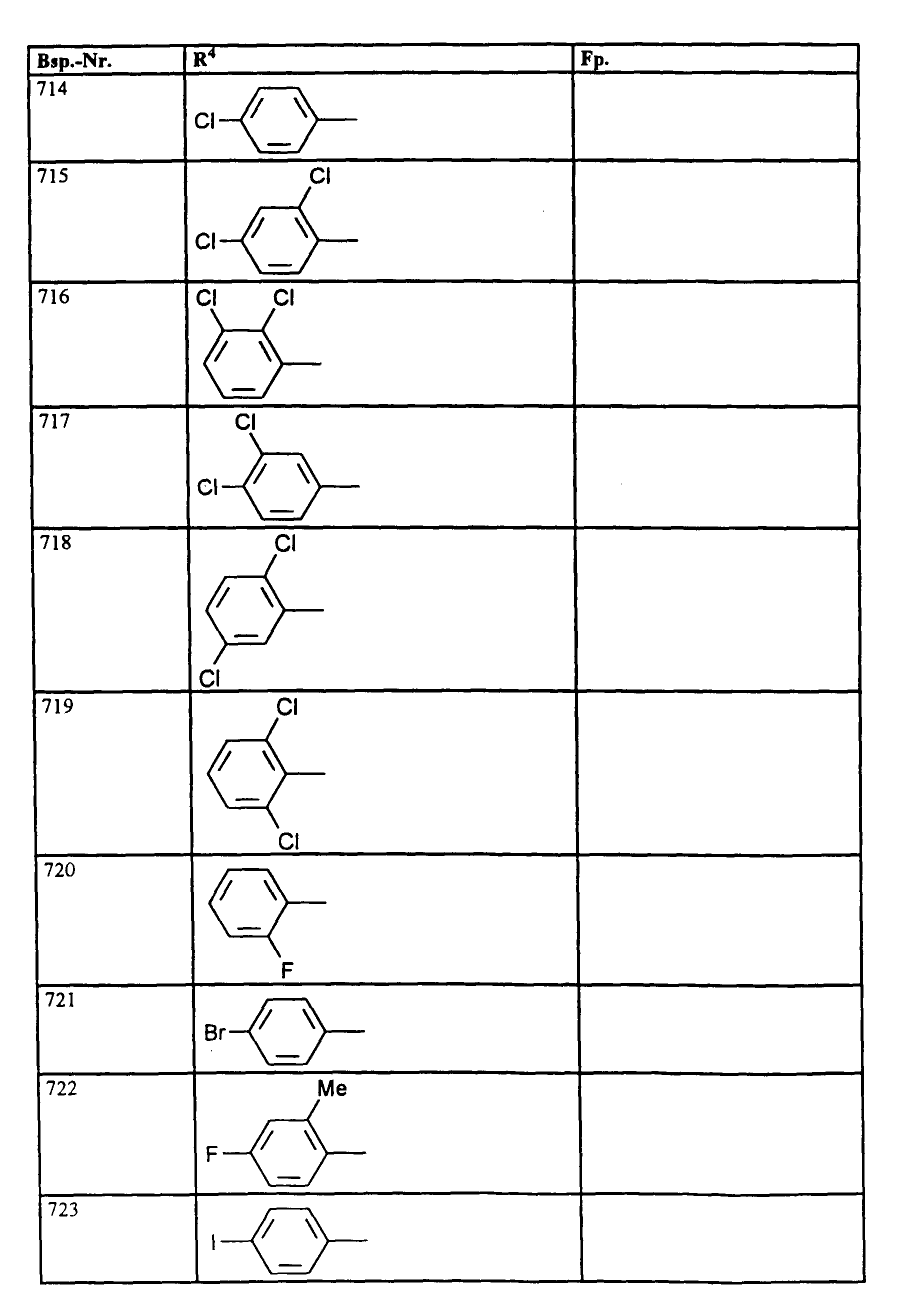

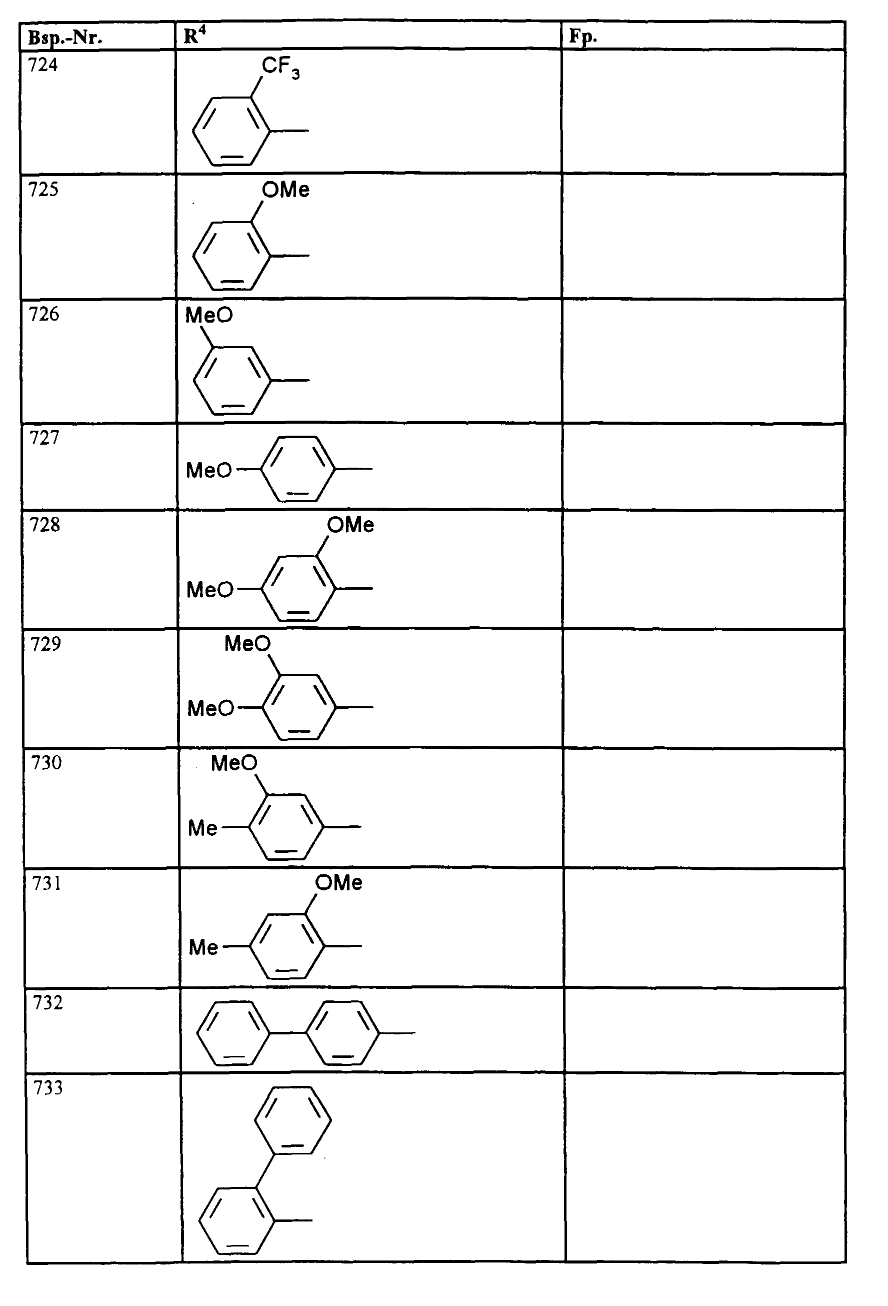

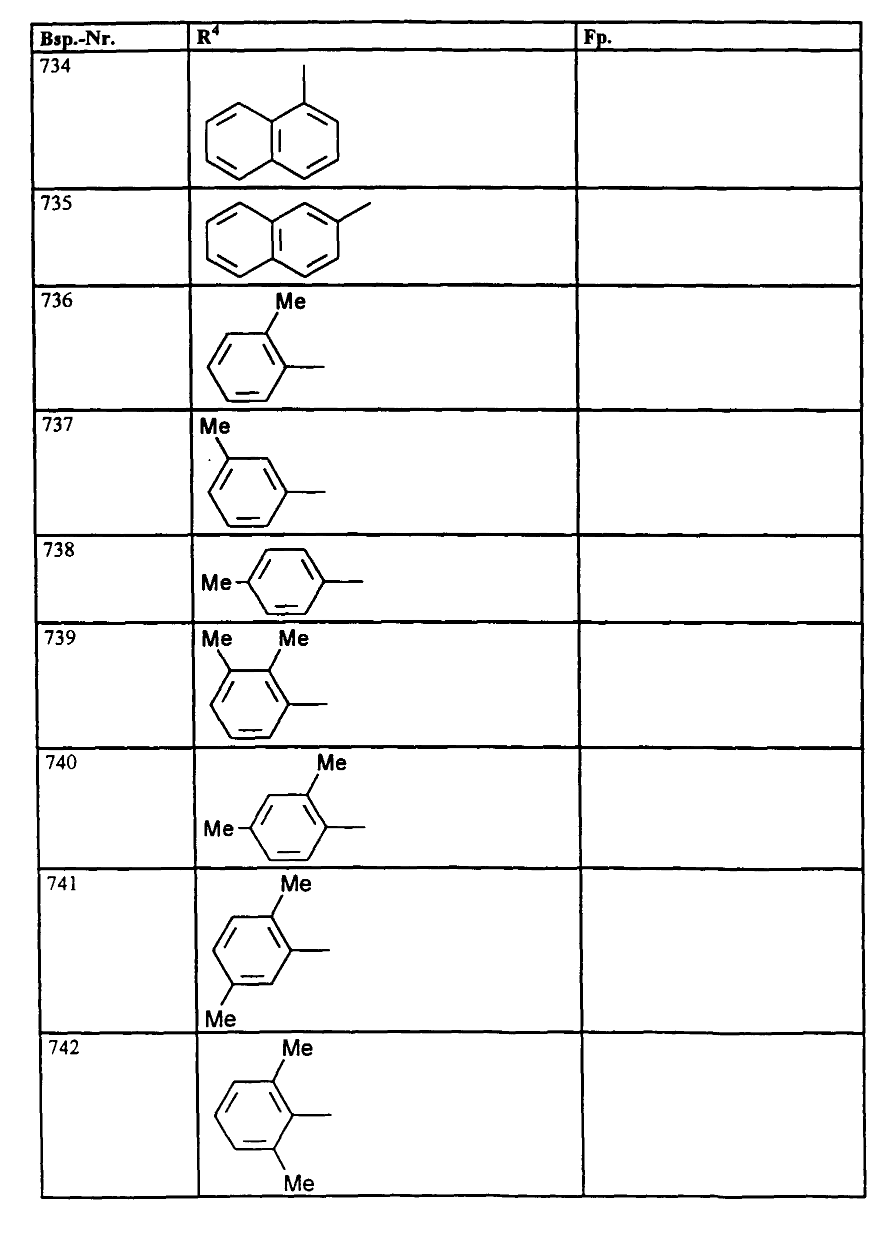

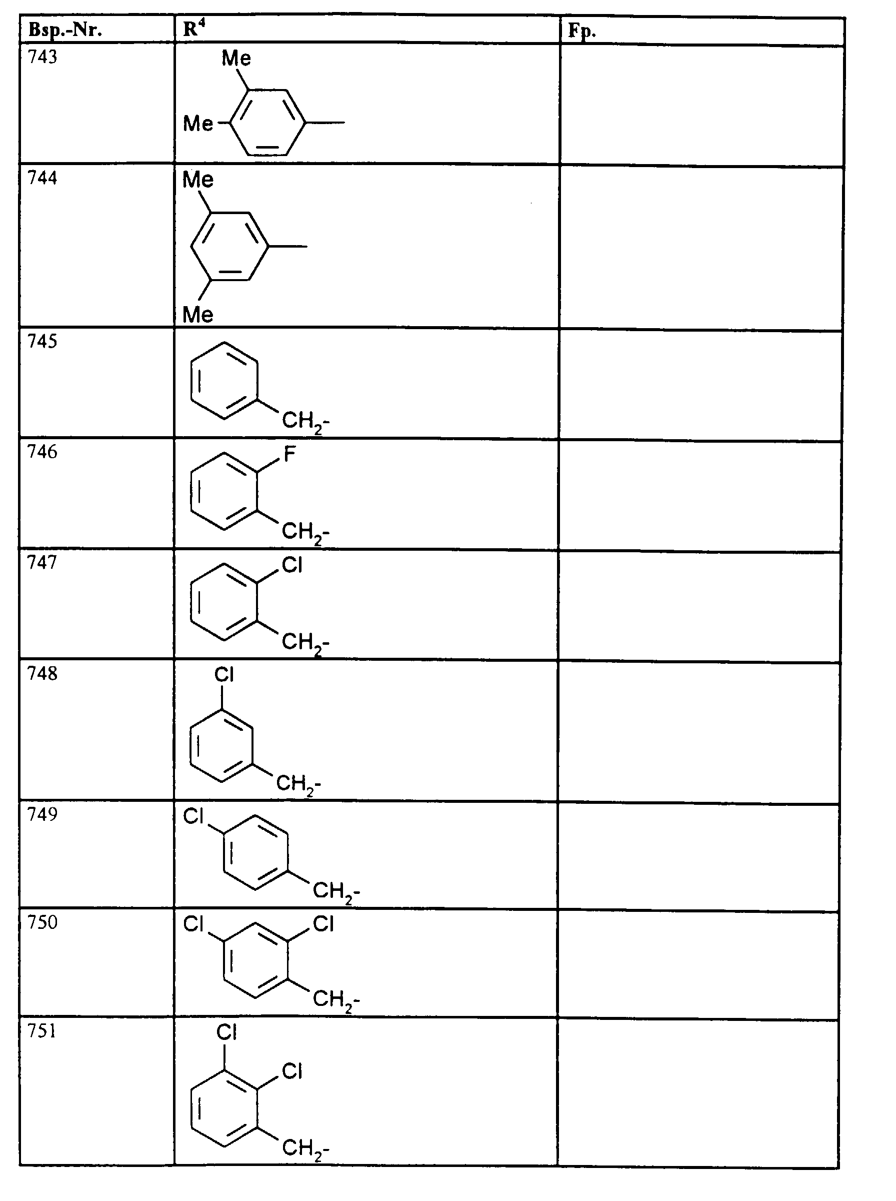

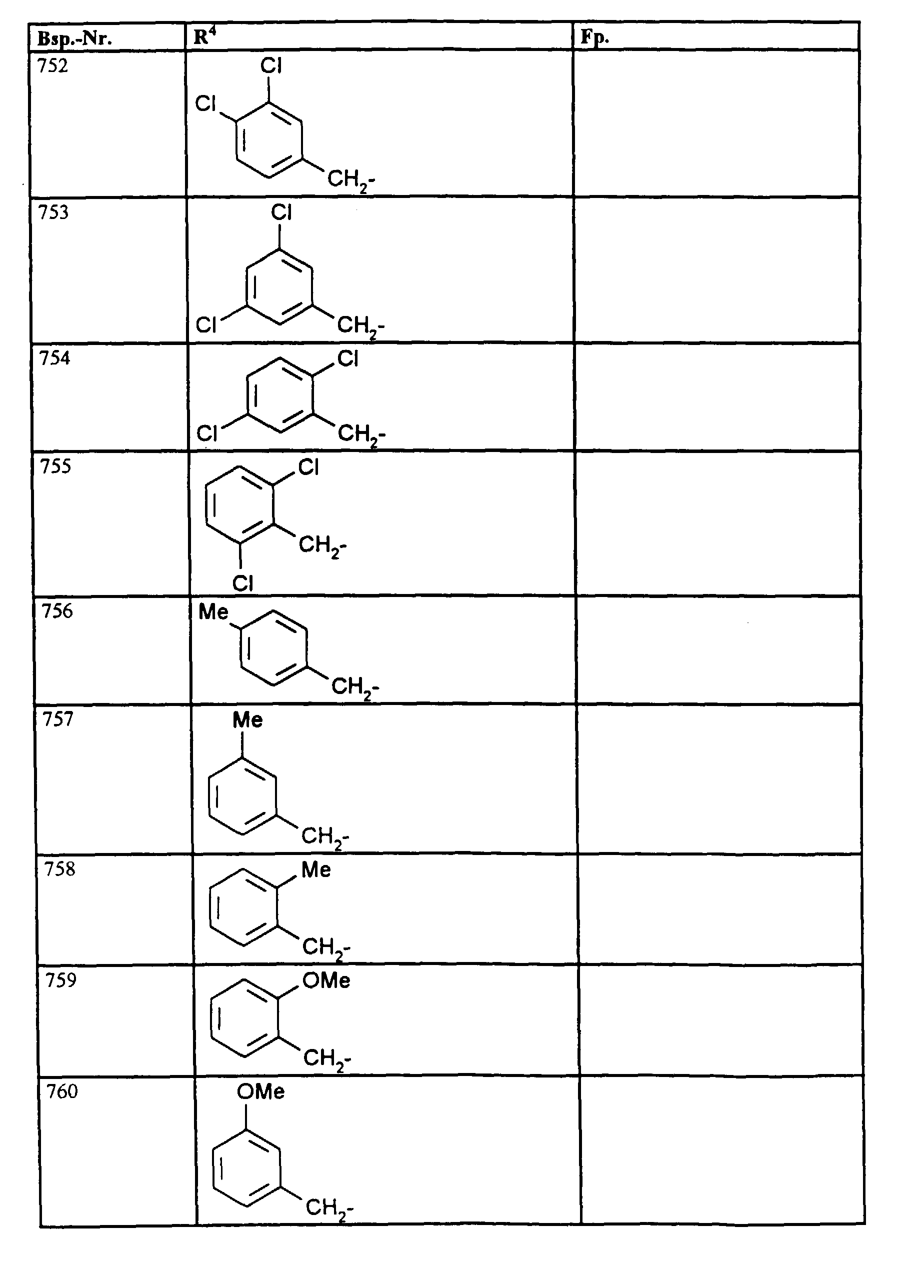

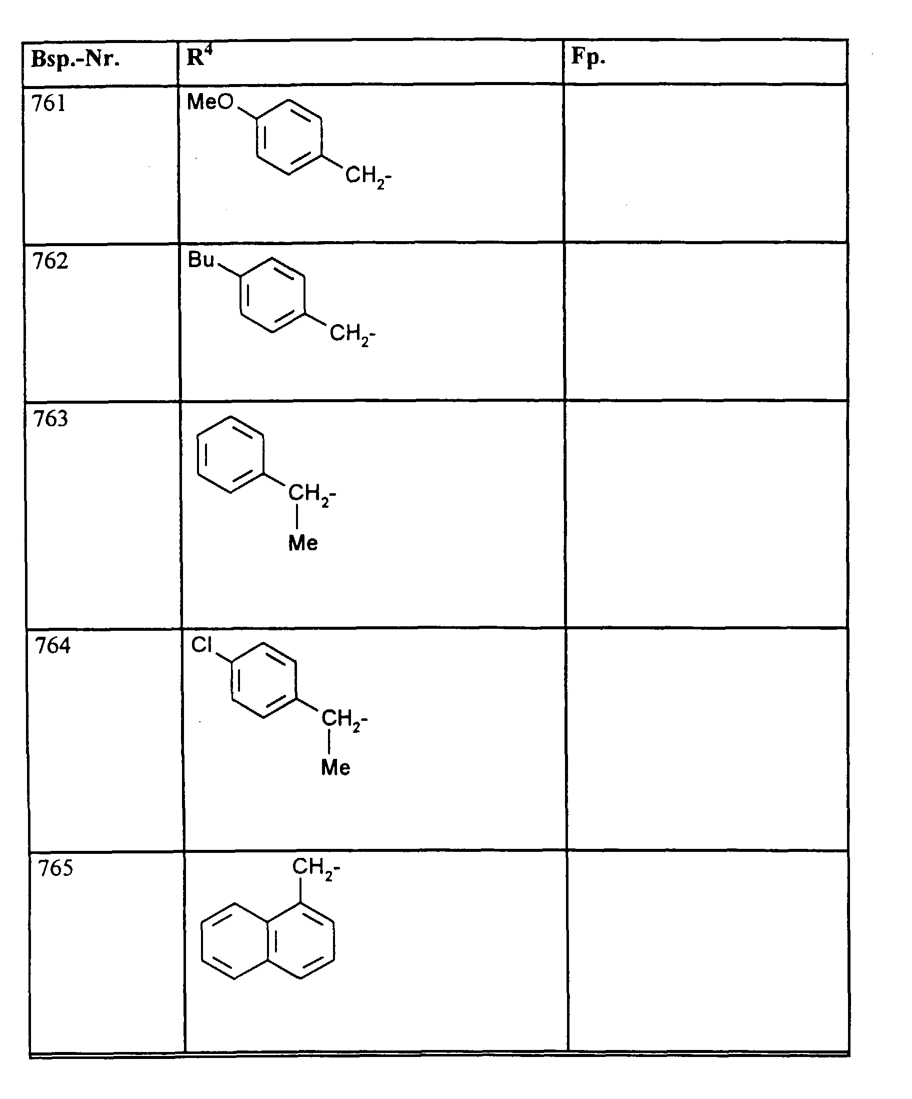

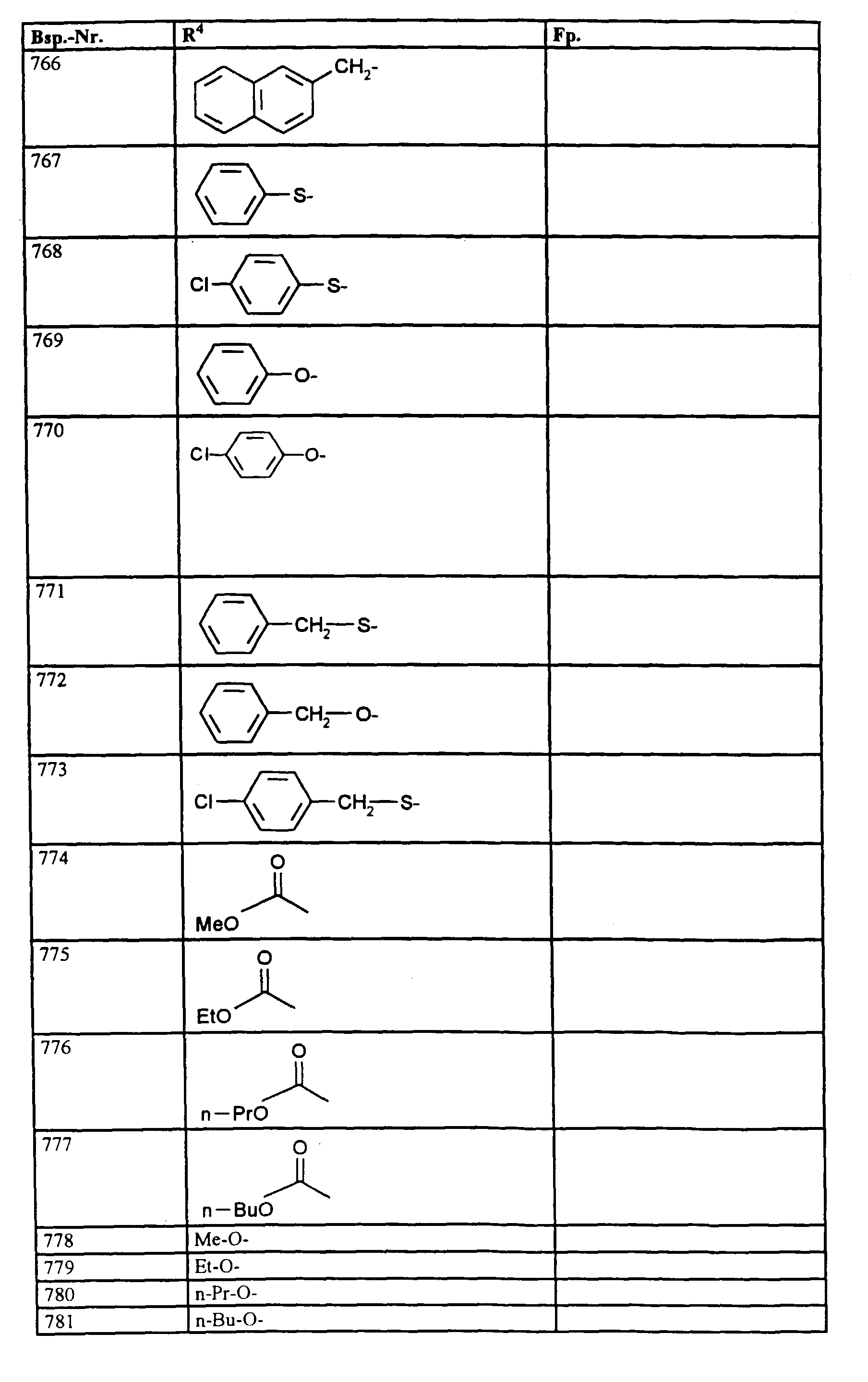

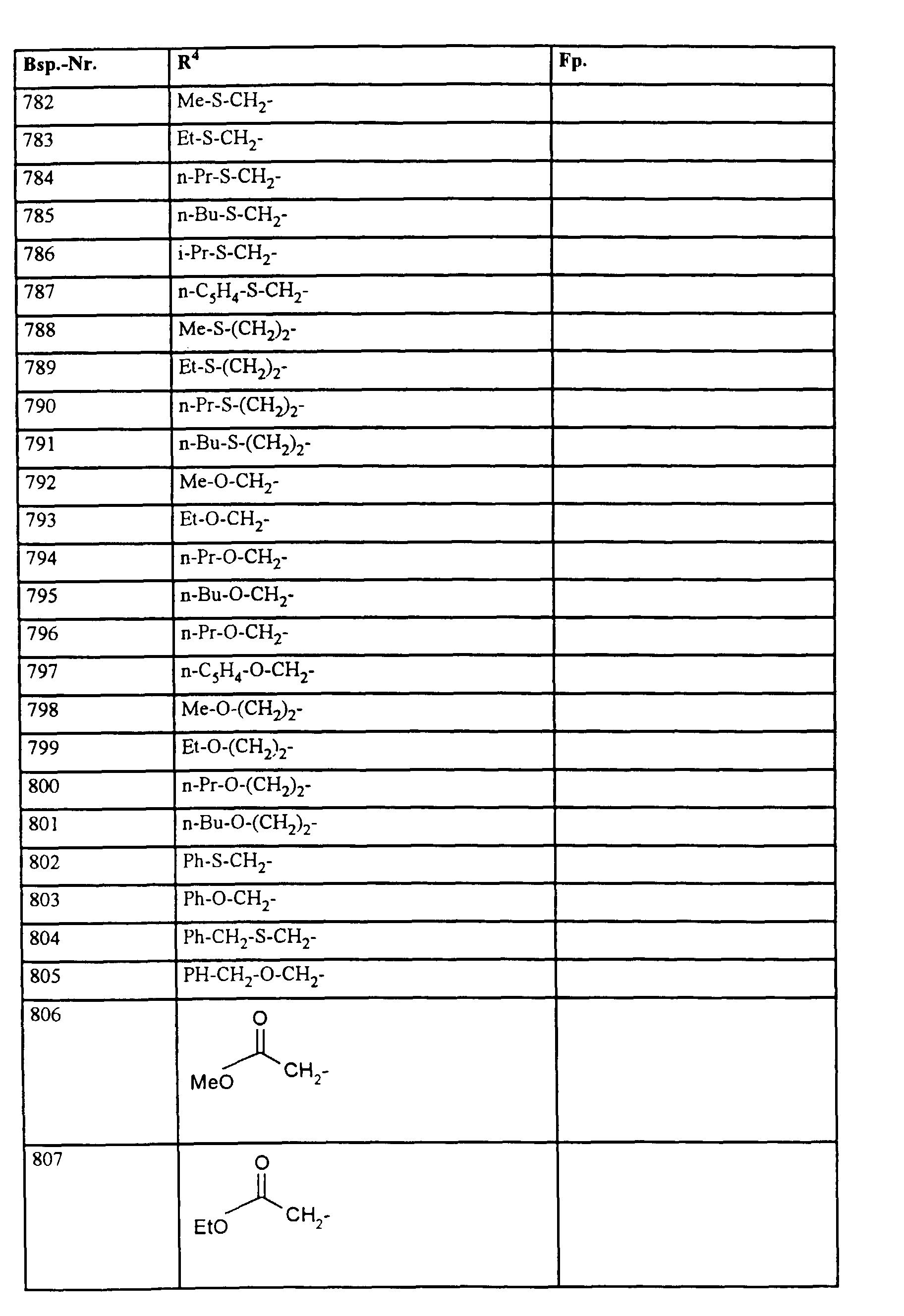

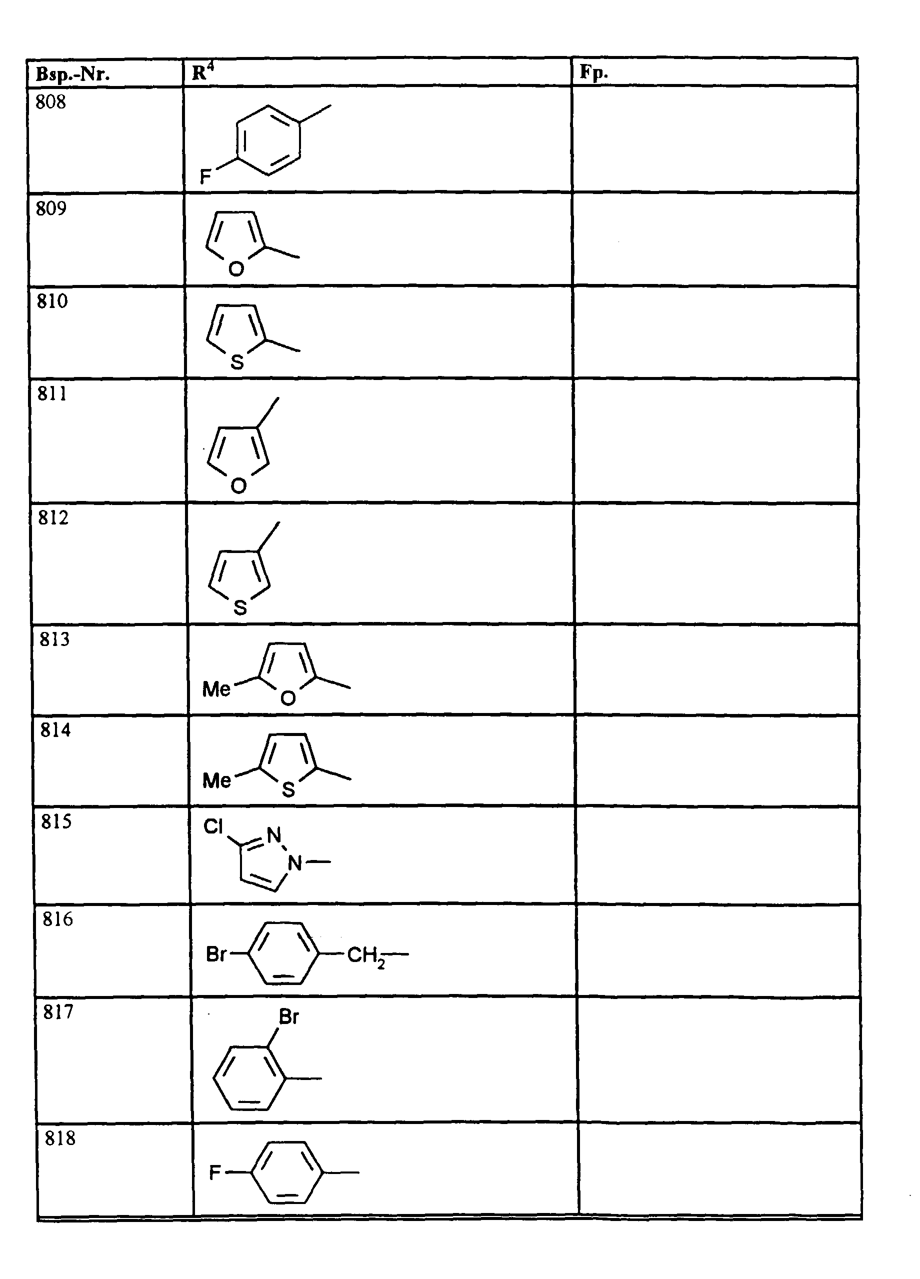

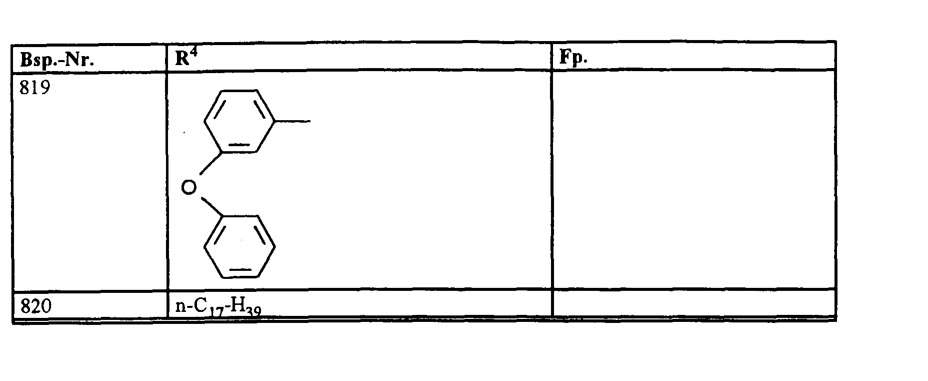

### B. Prüfung der Schimmelfestigkeit von Anstrichen

Die auf ihre fungizide Wirksamkeit zu prüfende Substanz wird in der gewünschten Konzentration in die (Dispersions)-Farbe mittels eines Dissolvers eingearbeitet. Anschließend wird die Farbe beidseitig auf eine geeignete Unterlage gestrichen.

Um praxisnahe Ergebnisse zu erhalten wird ein Teil der Prüflinge vor dem Test auf Schimmelfestigkeit mit fließendem Wasser (24 h; 20°C) ausgelaugt.

Dieso vorbereiteten Prüflinge werden auf einen Agar-Nährboden gelegt. Prüflinge und Nährboden werden mit Pilzsorten kontaminiert. Nach 1- bis 3-wöchiger Lagerung bei 29 ± 1°C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:
1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

Die folgende Tabelle V zeigt die Wirkstoffkonzentrationen, bei denen der Anstrichprüfling pilzfrei bleibt (Konzentrationen bezogen auf Feststoffgehalt der Dispersionsfarbe).

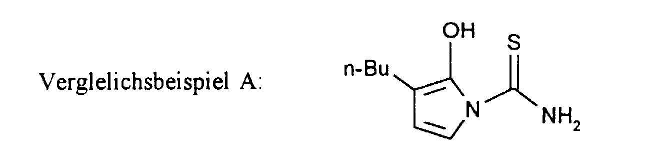

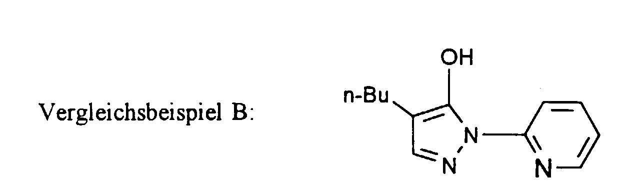

**Tabelle V**

| | ohne Belastung | nach Wässerung | Verfärbung |
|---|---|---|---|
| Bekannt Bsp. A | 0,3 % | > 3 % | keine |
| Bekannt Bsp. B | 0,2 % | 0,3 % | ab 0,3 % |
| erfindungsgemäß Bsp. 7 | 0,6 % | 2,0 % | keine |

## Patentansprüche

1. Verbindungen der Formel (I), worin 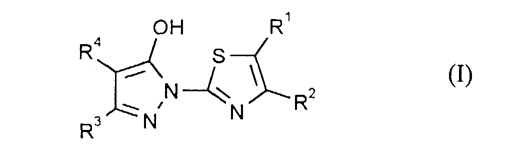
R¹, R², R³ Wasserstoff oder Methyl,
R⁴ unsubstituiertes Alkyl, Cycloalkyl, Alkenyl, Aralkyl oder Aryl auch durch Halogen substitueirtes Alkyl; durch 1 bis 3 C₁-C₄-Alkyl oder 1 bis 3 Halogen substituiertes Cycloalkyl; durch jeweils 1 bis 3 Halogen, Nitro, Cyano, C₁-C₄-Alkyl, C₁-C₄-Alkoxy, halogenisertes C₁-C₄-Alkyl, halogeniertes C₁-C₄-Alkoxy, C₁-C₄-Alkylmercapto oder halogenisertes C₁-C₄-Alkylmercapto substituiertes Aralkyl; oder durch jeweils 1 bis 3 Halogen, C₁-C₆-Alkyl, C₁-C₆-Alkoxy, C₁-C₆-Thioalkyl, Halogen-C₁-C₂-alkyl, Cyano, Nitro, C₁-C₆-Alkoxycarbonyl oder Amino substituiertes Aryl bedeuten.

2. Verbindungen der Formel (I) nach Anspruch 1, worin
R¹ Wasserstoff,
R², R³ Wasserstoff oder Methyl, bedeuten.

3. Verbindungen der Formel (I) nach Anspruch 1, worin
R¹, R² und R³ Wasserstoff,
R⁴ unsubstituiertes Alkyl oder Cycloalkyl oder durch die in Anspruch 1 angegebenen substituerten substituiertes Alkyl oder Cycloalkyl
bedeuten.

4. Verfahren zur Herstellung von neuen Thiazolylpyrazolinon-Derivaten der Formel (I) nach Anspruch 1, dadurch gekennzeichnet, daß man Thiocarbamoylverbindungen der Formel (II) 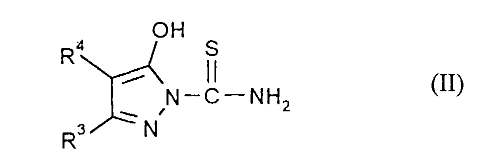 in denen R³ und R⁴ die oben angebenen Bedeutungen haben, mit Verbindungen der Formel (III) 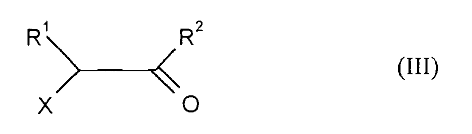 in denen R¹ und R² die oben angegebenen Bedeutungen haben und X für eine Abgangsgruppe steht, gegebenenfalls in Gegenwart eines Lösungsbzw. Verdünnungsmittels und gegebenenfalls in Gegenwart einer Base umsetzt.

5. Verfahren zum Schutz von technischen Materialien, dadurch gekennzeichnet, daß man Verbindungen der Formel (I) nach Anspruch auf die zu schützenden Materialien aufbringt oder mit diesen vermischt.

6. Mittel zum Schutz von technischen Materialien, enthaltend mindestens eine Verbindung der Formel (I) nach Anspruch 1.

7. Verwendung von Verbindungen der Formel (I) nach Anspruch 1 zum Schützen von Materialien gegen Befall und Zerstörung durch unerwünschte Mikroorganismen.

## Claims

1. Compounds of the formula (I), in which 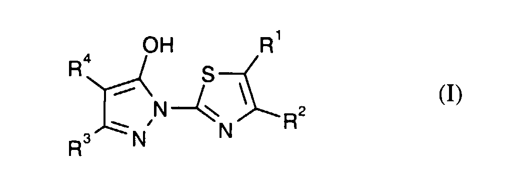
R¹, R², R³ denote hydrogen or methyl,
R⁴ denotes unsubstituted alkyl, cycloalkyl, alkenyl, aralkyl or aryl, also halogen-substituted alkyl; cycloalkyl substituted by 1 to 3 C₁-C₄-alkyl groups or 1 to 3 halogens; aralkyl substituted in each case by 1 to 3 halogens, nitro, cyano, C₁-C₄-alkyl, C₁-C₄-alkoxy, halogenated C₁-C₄-alkyl, halogenated C₁-C₄-alkoxy, C₁-C₄-alkylmercapto or halogenated C₁-C₄-alkylmercapto groups; or aryl substituted in each case by 1 to 3 halogens, C₁-C₆-alkyl, C₁-C₆-alkoxy, C₁-C₆-thioalkyl, halogeno-C₁-C₂-alkyl, cyano, nitro, C₁-C₆-alkoxycarbonyl or amino groups.

2. Compounds of the formula (I) according to Claim 1, in which
R¹ denotes hydrogen,
R², R³ denote hydrogen or methyl.

3. Compounds of the formula (I) according to Claim 1, in which
R¹, R² and R³ denote hydrogen,
R⁴ denotes unsubstituted alkyl or cycloalkyl, or alkyl or cycloalkyl substituted by the substituents given in Claim 1.

4. Process for the preparation of novel thiazolylpyrazolinone derivatives of the formula (I) according to Claim 1, characterized in that thiocarbamoyl compounds of the formula (II) 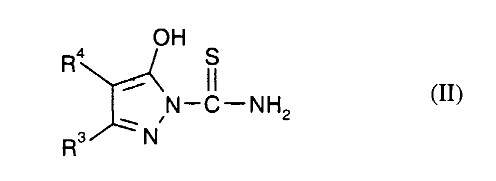 in which R³ and R⁴ have the meanings given above are reacted, in the presence or absence of a solvent or diluent and in the presence or absence of a base, with compounds of the formula (III) 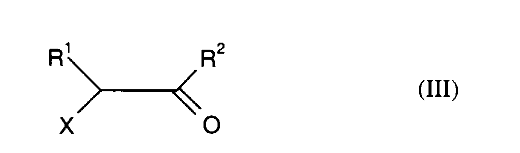 in which R¹ and R² have the meanings given above and X represents a leaving group.

5. Process for the protection of technical materials, characterized in that compounds of the formula (I) according to Claim are applied to the materials to be protected or are mixed with these.

6. Compositions for the protection of technical materials containing at least one compound of the formula (I) according to Claim 1.

7. Use of compounds of the formula (I) according to Claim 1 for protecting materials against attack and destruction by undesirable microorganisms.

## Revendications

1. Composés de formule (I) 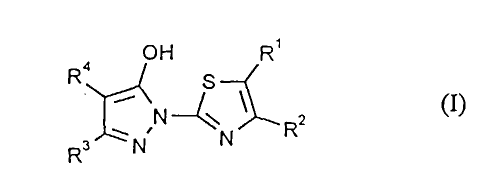 dans laquelle
R¹, R², R³ représentent l'hydrogène ou le groupe méthyle,
R⁴ est un groupe alkyle, cycloalkyle, alcényle, aralkyle ou aryle non substitué, ainsi qu'un groupe alkyle substitué par un halogène ; un groupe cycloalkyle substitué par 1 à 3 radicaux alkyle en C₁ à C₄ ou 1 à 3 atomes d'halogènes ; un groupe aralkyle portant 1 à 3 substituants halogéno, nitro, cyano, alkyle en C₁ à C₄, alkoxy en C₁ à C₄, halogénalkyle en C₁ à C₄, halogénalkoxy en C₁ à C₄, alkylmercapto en C₁ à C₄ ou halogénalkylmercapto en C₁ à C₄ ; ou un groupe aryle substitué par 1 à 3 radicaux halogéno, alkyle en C₁ à C₆, alkoxy en C₁ à C₆, thioalkyle en C₁ à C₆, halogénalkyle en C₁ ou C₂, cyano, nitro, (alkoxy en C₁ à C₆)carbonyle ou amino.

2. Composés de formule (I) suivant la revendication 1, dans lesquels
R¹ représente l'hydrogène,
R², R³ représentent l'hydrogène ou le groupe méthyle.

3. Composés de formule (I) suivant la revendication 1, dans lesquels
R¹, R² et R³ représentent l'hydrogène,
R⁴ est un groupe alkyle ou cycloalkyle non substitué ou un groupe alkyle ou cycloalkyle substitué par les substituants indiqués dans la revendication 1.

4. Procédé de production de nouveaux dérivés de thiazolylpyrazolinone de formule (I) suivant la revendication 1, caractérisé en ce qu'on fait réagir des composés thiocarbamoyliques de formule (II) 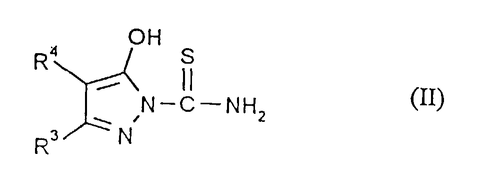 dans lesquels R³ et R⁴ ont les définitions indiquées ci-dessus, avec des composés de formule (III) 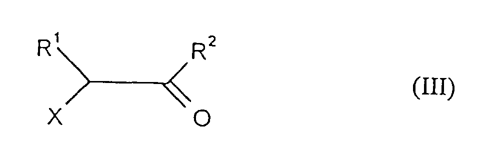 dans lesquels R¹ et R² ont les définitions indiquées ci-dessus et X représente un groupe partant, le cas échéant en présence d'un solvant ou d'un diluant et en la présence éventuelle d'une base.

5. Procédé de protection de matériaux à usage technique, caractérisé en ce qu'on applique des composés de formule (I) suivant la revendication 1 sur les matériaux à protéger ou on les mélange avec ces matériaux.

6. Compositions destinées à protéger des matériaux à usage technique, contenant au moins un composé de formule (I) suivant la revendication 1.

7. Utilisation de composés de formule (I) suivant la revendication 1, pour protéger des matériaux d'une attaque ou d'une destruction par des micro-organismes indésirables.
